(19) Europäisches Patentamt
European Patent Office
Office européen des brevets

(11) EP 1 272 468 B1

(12) FASCICULE DE BREVET EUROPEEN

(45) Date de publication et mention de la délivrance du brevet: **17.11.2004 Bulletin 2004/47**

(21) Numéro de dépôt: **01919610.4**

(22) Date de dépôt: **02.04.2001**

(51) Int Cl.[7]: **C07D 209/34**, A61K 31/404, A61P 5/10, C07D 209/38, C07D 405/10, C07D 401/04, C07D 401/12, C07D 403/12, C07D 403/10, C07D 401/10, C07D 405/12

(86) Numéro de dépôt international: **PCT/FR2001/000980**

(87) Numéro de publication internationale: **WO 2001/074775 (11.10.2001 Gazette 2001/41)**

# (54) DERIVES D'INDOLIN-2-ONE ET LEUR UTILISATION EN TANT QUE LIGANDS DES RECEPTEURS DE L'OCYTOCINE

INDOLIN-2-ON DERIVATE UND DEREN VERWENDUNG ALS LIGANDEN DER OCYTOCIN REZEPTOREN

INDOLIN-2-ONE DERIVATIVES, PREPARATION AND THEIR USE AS OCYTOCIN RECEPTOR LIGANDS

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **03.04.2000 FR 0004193**

(43) Date de publication de la demande: **08.01.2003 Bulletin 2003/02**

(73) Titulaire: **Sanofi-Aventis**
**75013 Paris (FR)**

(72) Inventeurs:
- **FOULON, Loic**
  **F-31120 Portet sur Garonne (FR)**
- **GARCIA, Georges**
  **F-34110 Frontignan (FR)**
- **SERRADEIL-LE GAL, Claudine**
  **F-31750 Escalquens (FR)**
- **VALETTE, Gérard**
  **F-31120 Lacroix-Falgarde (FR)**

(74) Mandataire: **Thouret-Lemaitre, Elisabeth et al**
**Sanofi-Aventis**
**174 avenue de France**
**75013 Paris (FR)**

(56) Documents cités:
**EP-A- 0 636 608** **WO-A-95/18105**

EP 1 272 468 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet de nouveaux dérivés d'indolin-2-one, un procédé pour leur préparation et les compositions pharmaceutiques les comprenant. Ces nouveaux dérivés sont des ligands puissants et sélectifs des récepteurs de l'ocytocine et peuvent donc être utilisés en tant que principe actif dans des compositions pharmaceutiques, notamment dans le domaine obstétrique ou gynécologique.
L'ocytocine (OT) est une hormone neurohypophysaire, de structure nonapeptidique cyclique proche de celle de la vasopressine arginine (AVP). Les récepteurs de l'ocytocine se trouvent essentiellement sur le muscle lisse de l'utérus et sur les cellules myoépithéliales des glandes mammaires. Ainsi, l'ocytocine joue un rôle important dans la parturition puisqu'elle intervient dans la contraction du muscle utérin et dans la lactation. Par ailleurs, les récepteurs de l'ocytocine sont également localisés dans d'autres tissus périphériques et dans le système nerveux central ; l'ocytocine peut donc avoir des effets dans les domaines cardiovasculaire, rénal, endocrinien ou comportemental.
Des dérivés d'indolin-2-one ont été décrits dans certaines demandes de brevet comme des ligands des récepteurs de la vasopressine et éventuellement des récepteurs de l'ocytocine ; on pourra citer les demandes de brevet WO 93/15051, EP 636608, EP 636609, WO 95/18105, WO 97/15556 et WO 98/25901. Jusqu'à présent, aucun dérivé d'indolin-2-one n'a été décrit en tant que ligand puissant et sélectif des récepteurs de l'ocytocine.
Il a été maintenant trouvé que certains dérivés d'indolin-2-one sont des ligands puissants et sélectifs des récepteurs de l'ocytocine.
Ainsi, selon l'un de ses aspects, la présente invention concerne de nouveaux dérivés d'indolin-2-one sous forme d'énantiomère pur ou de mélange d'énantiomères de formule :

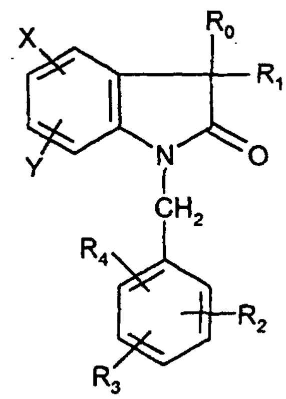

(I)

dans laquelle:

- $R_0$ représente un groupe choisi parmi :
  (i) :

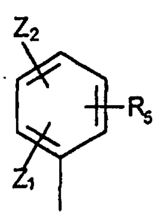

dans lequel :

- $Z_1$ représente un atome de chlore, brome, iode ou fluor, un groupe $(C_1-C_4)$alkyle, $(C_1-C_4)$alcoxy ou trifluorométhyle ;
- $Z_2$ représente un atome d'hydrogène, de chlore, brome, iode ou fluor, un groupe $(C_1-C_4)$alkyle, un groupe $(C_3-C_5)$cycloalkyle, $(C_1-C_4)$alcoxy, un groupe $(C_3-C_5)$cycloalcoxy, ou $(C_1-C_4)$polyfluoroalkyle ;
- $R_5$ représente $T_1W$ dans lequel $T_1$ représente $-(CH_2)_m$, m pouvant être égal à 0 ou 1, W réprésente un atome

d'hydrogène, un groupe hydroxycarbonyle (ou carboxyle), $(C_{1-}C_4)$alcoxycarbonyle, 1,3-dioxolan-2-yle, 1,3-dioxan-2-yle,

ou bien W représente un groupe $-NR_6R_7$ dans lequel $R_6$ et $R_7$ représentent indépendamment l'un de l'autre un atome d'hydrogène, un groupe $(C_1-C_4)$alkyle, un groupe $(C_1-C_4)$alkylsulfonyle ou phénylsulfonyle dans lequel le groupe phényle peut être mono, di ou trisubstitué par $Z_5$ ; ou bien $R_6$ et $R_7$ forment avec l'atome d'azote auquel ils sont liés un groupe morpholinyle éventuellement substitué par un groupe $(C_1-C_4)$alkyle ou un oxo, ou bien $R_6$ et $R_7$ forment avec l'atome d'azote auquel ils sont liés un groupe pipérazinyle éventuellement substitué en position 4 par un substituant $Z_3$ ; ou bien $R_6$ et $R_7$ forment avec l'atome d'azote auquel ils sont liés un groupe pyrrolidinyle ou pipéridinyle, lesdits groupes pyrrolidinyle et pipéridinyle étant éventuellement substitués par $Z_4$ ;

ou bien W représente un groupe $-NR_8COR_9$ dans lequel $R_8$ représente un atome d'hydrogène ou un groupe $(C_1-C_4)$alkyle et $R_9$ représente un atome d'hydrogène, un groupe $(C_1-C_4)$alkyle, benzyle, pyridyle, phényle, ledit groupe phényle pouvant être mono, di ou trisubstitué par $Z_5$ ; ou bien $R_9$ représente un groupe $-NR_{10}R_{11}$ dans lequel $R_{10}$ et $R_{11}$ représentent indépendamment l'un de l'autre un atome d'hydrogène ou un $(C_{1-}C_4)$alkyle, ou bien $R_{10}$ et $R_{11}$ forment avec l'atome d'azote auquel ils sont liés un groupe pyrrolidinyle, pipéridinyle, ou morpholinyle éventuellement substitué par un groupe $(C_1-C_4)$alkyle, ou bien $R_9$ représente un groupe pyrrolidin-2-yle ou 3-yle, pipéridin-2-yle, 3-yle ou 4-yle, lesdits groupes pyrrolidinyle et pipéridinyle étant éventuellement substitués par $Z_7$ ; ou bien $R_9$ représente un groupe $-T_2-R_{12}$ ou - $T_2-COR_{12}$ dans lequel $T_2$ représente $-(CH_2)_n-$, n pouvant être égal à 1, 2, 3 et 4, et $R_{12}$ représente un groupe $(C_{1-}C_4)$alcoxy ou $-NR_{10}R_{11}$, $R_{10}$ et $R_{11}$ étant tels que définis ci-dessus ;

ou bien W représente un groupe $-CONR_{13}R_{14}$ dans lequel $R_{13}$ représente un atome d'hydrogène, un groupe $(C_{1-}C_4)$alkyle, $(C_3-C_7)$cycloalkyle, un mono- ou polyfluoro$(C_1-C_4)$alkyle, et $R_{14}$ représente un atome d'hydrogène, un groupe $(C_1-C_4)$alkyle, phényle éventuellement substitué par $Z_5$, un groupe $-T_4-R_{15}$ dans lequel $T_4$ représente $-(CH_2)_q$ avec q égal à 1, 2, 3 ou 4 et $R_{15}$ représente un groupe hydroxyle, un groupe $(C_{1-}C_4)$alcoxy, $(C_{1-}C_4)$alcoxycarbonyle, $(C_1-C_4)$alcoxycarbonylamino, phényle éventuellement mono-, disubstitué par $Z_5$, un pyrid-2-yle, 3-yle ou 4-yle, un groupe $-NR_{16}R_{17}$ dans lequel $R_{16}$ et $R_{17}$ représentent indépendamment l'un de l'autre un atome d'hydrogène, ou un $(C_{1-}C_4)$alkyle, ou bien $R_{16}$ et $R_{17}$ forment avec l'atome d'azote auquel ils sont liés un groupe morpholinyle éventuellement mono ou disubstitué par un groupe $(C_1-C_4)$alkyle, ou bien $R_{16}$ et $R_{17}$ forment avec l'atome d'azote auquel ils sont liés un groupe pipérazinyle éventuellement substitué en position 4 par un substituant $Z_3$, ou bien $R_{16}$ et $R_{17}$ forment avec l'atome d'azote auquel ils sont liés un groupe pyrrolidinyle ou pipéridinyle, lesdits groupes pyrrolidinyle et pipéridinyle étant éventuellement substitués par $Z_5$ étant entendu que lorsque q = 1, $R_{15}$ est différent de hydroxyle, $(C_1-C_4)$alcoxy, $(C_1-C_4)$alcoxycarbonylamino, $-NR_{16}R_{17}$ ; ou bien $R_{13}$ et $R_{14}$ forment avec l'atome d'azote auquel ils sont liés un groupe morpholinyle éventuellement mono ou disubstitué par un groupe $(C_1-C_4)$alkyle, pipérazinyle éventuellement substitué en position 4 par un substituant $Z_3$; ou bien $R_{13}$ et $R_{14}$ forment avec l'atome d'azote auquel ils sont liés un groupe azétidinyle, pyrrolidinyle ou pipéridinyle, hexahydroazépinyle, lesdits groupes pyrrolidinyle, pipéridinyle et hexahydroazépinyle étant éventuellement mono ou disubstitués par $Z_8$ ;

ou bien W représente un groupe $OR_{18}$ dans lequel $R_{18}$ représente un atome d'hydrogène, un groupe $(C_1-C_4)$ alkyle, $(C_1-C_4)$alcoxy$(C_1-C_4)$alkyle ou $-T_3-R_{19}$ dans lequel $T_3$ représente $-(CH_2)_p-$, p pouvant être égal à 2, 3 et $R_{19}$ est choisi parmi les groupes hydroxyle, triphénylméthoxy, $-NR_{20}R_{21}$ dans lequel $R_{20}$ représente un atome d'hydrogène ou un groupe $(C_{1-}C_4)$alkyle et $R_{21}$ représente un atome d'hydrogène, un groupe $(C_{1-}C_4)$alkyle, tétrahydrofuranytméthyle ou tétrahydropyranylméthyle, ou bien $R_{20}$ et $R_{21}$ forment avec l'atome d'azote auquel ils sont liés un groupe morpholinyle éventuellement mono ou disubstitué par un groupe $(C_1-C_4)$alkyle, pipérazinyle éventuellement substitué en position 4 par un substituant $Z_3$ ou bien $R_{20}$ et $R_{21}$ forment avec l'atome d'azote auquel ils sont liés un groupe pyrrolidinyle ou pipéridinyle, lesdits groupes pyrrolidinyle et pipéridinyle étant éventuellement substitués par $Z_5$ ;

- $Z_3$ représente un groupe $(C_{1-}C_4)$alkyle, pyridyle ou phényle, un groupe $(C_1-C_4)$alkylcarbonyle, $(C_1-C_4)$ alcoxycarbonyle ;
- $Z_4$ représente un oxo, un atome de fluor, un hydroxyle, un $(C_1-C_4)$alkyle, un benzyle, un amino, un $(C_1-C_4)$alkylamino, un di$(C_1-C_4)$alkylamino, un $(C_1-C_4)$ alcoxy, un $(C_1-C_4)$alcoxycarbonyle, un $(C_1-C_4)$alcoxycarbonylamino ;
- $Z_5$ représente un atome de chlore, brome, iode ou fluor, un groupe hydroxyle, un groupe $(C_{1-}C_4)$alkyle, ou $(C_{1-}C_4)$ alcoxy ;
- $Z_7$ représente un atome de fluor, un groupe hydroxyle, un groupe hydroxy$(C_1-C_4)$alkyle, un $(C_1-C_4)$alkyle, un $(C_1-C_4)$alcoxy, un $(C_1-C_4)$alkylcarbonyle ;
- $Z_8$ représente un atome de fluor, un groupe hydroxyle, $(C_{1-}C_4)$alkyle, $(C_3-C_6)$cycloalkyle, benzyle, amino, $(C_{1-}C_4)$ alkylamino, di$(C_{1-}C_4)$alkylamino, $(C_{1-}C_4)$alcoxycarbonyle, $(C_1-C_4)$alcoxycarbonylamino, $(C_3-C_6)$cycloalcoxy, hydroxycarbonyle, hydroxy$(C_{1-}C_4)$alkyle ou $(C_{1-}C_4)$alcoxy$(C_{1-}C_4)$alkyle, $(C_1-C_4)$alcoxy,
- $CONR_{23}R_{24}$ dans lequel $R_{23}$ et $R_{24}$ représentent indépendamment l'un de l'autre, un atome d'hydrogène, un $(C_1-C_4)$alkyle, un mono ou polyfluoro$(C_1-C_4)$alkyle ou bien $R_{23}$ et $R_{24}$ forment avec l'atome d'azote auquel ils sont liés un groupe pyrrolidinyle ou pipéridinyle, lesdits groupes pyrrolidinyle ou pipéridinyle étant éventuellement subs-

tités par $Z_3$, un difluorométhylidène ;
(ii) :

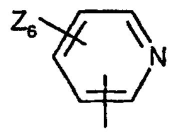

- $Z_6$ représente un atome de chlore ou un groupe ($C_1$-$C_4$)alkyle ou ($C_1$-$C_4$)alcoxy ;
- $R_1$ représente un groupe ($C_1$-$C_4$)alkyle comportant éventuellement une double ou une triple liaison ; ($C_1$-$C_4$) alcoxycarbonyle ; phényloxycarbonyle ou un groupe $T_1$-$R_{22}$ dans lequel $T_1$ est tel que défini ci-dessus et $R_{22}$ représente un groupe hydroxyle ou ($C_1$-$C_4$)alcoxy ;
- $R_2$ et $R_4$ représentent indépendamment l'un de l'autre, un atome d'hydrogène, de chlore ou de fluor, un groupe ($C_1$-$C_4$)alkyle ou ($C_1$-$C_4$)alcoxy ;
- $R_3$ représente un atome de chlore ou de fluor, un groupe ($C_1$-$C_4$)alkyle ; ($C_1$-$C_4$)alcoxy ; hydroxyle ; un groupe ($C_1$-$C_4$)carbamoyle ; un ($C_1$-$C_4$)alkylcarbonylamino ; nitro ; cyano; trifluorométhyle ; amino ; ($C_3$-$C_6$) cycloalkylamino ; ($C_1$-$C_4$)alkylamino ; di($C_1$-$C_4$)alkylamino ; tri($C_1$-$C_4$)alkylammonium, $A^-$, $A^-$ étant un anion ; pyrrolidin-1-yle ; pipéridin-1-yle ; pipérazin-1-yle ; morpholin-4-yle ou hexahydroazépin-1-yle ;
- X et Y représentent indépendamment l'un de l'autre un atome d'hydrogène, de chlore, brome, iode ou fluor ou un groupe ($C_1$-$C_4$)alcoxy ou trifluorométhoxy ;

ainsi que leurs sels pharmaceutiquement acceptables, solvats et hydrates.

Par alkyle, on entend un radical monovalent hydrocarboné saturé, linéaire ou ramifié.

Par ($C_1$-$C_4$)alkyle, on entend un radical alkyle comprenant de 1 à 4 atomes de carbone tel que méthyle, éthyle, n-propyle, isopropyle, n-butyle, isobutyle, sec-butyle et *tert*-butyle.

Par alkylène, on entend un radical bivalent hydrocarboné saturé, linéaire ou ramifié.

Par alcoxy, on entend un radical O-alkyle.

Par anion A', on entend par exemple un $Cl^-$, Br, $I^-$ ou $CH_3SO_4^-$.

Par di($C_1$-$C_4$)alkylamino, on entend un radical amino substitué par deux radicaux alkyle, lesquels pouvant être identiques ou différents. De la même manière, pour les tri($C_1$-$C_4$)ammonium, les radicaux alkyle peuvent être identiques ou différents.

Les sels des composés selon l'invention sont préparés selon des techniques bien connues de l'homme de l'art. Les sels des composés de formule (I) selon la présente invention comprennent ceux avec des acides minéraux ou organiques qui permettent une séparation ou une cristallisation convenable des composés de formule (I), ainsi que des sels pharmaceutiquement acceptables. En tant qu'acide approprié, on peut citer : l'acide picrique, l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le paratoluènesulfonate, le chlorhydrate étant préféré.

Lorsqu'un composé selon l'invention présente un ou plusieurs carbones asymétriques, les isomères optiques de ce composé font partie intégrante de l'invention. Lorsqu'un composé selon l'invention présente une stéréoisomérie par exemple de type axial-équatorial ou Z-E, l'invention comprend tous les stéréoisomères de ce composé.

La présente invention comprend les composés de formule (I) sous forme d'isomères purs mais également sous forme de mélange d'isomères en proportion quelconque.

Les composés (I) sont isolés sous forme d'isomères purs par les techniques classiques de séparation : on pourra utiliser, par exemple des recristallisations fractionnées d'un sel du racémique avec un acide ou une base optiquement actif dont le principe est bien connu ou les techniques classiques de chromatographies sur phase chirale ou non chirale.

Les composés de formule (I) ci-dessus comprennent également ceux dans lesquels un ou plusieurs atomes d'hydrogène, de carbone ou d'halogène, notamment d'iode, de chlore ou de fluor ont été remplacés par leur isotope radioactif par exemple le tritium ou le carbone-14. De tels composés marqués sont utiles dans des travaux de recherche, de métabolisme ou de pharmacocinétique, dans des essais biochimiques en tant que ligand de récepteurs.

Les groupes fonctionnels éventuellement présents dans la molécule des composés de formule (I) et dans les intermédiaires réactionnels peuvent être protégés, soit sous forme permanente soit sous forme temporaire, par des groupes protecteurs qui assurent une synthèse univoque des composés attendus. Les réactions de protection et déprotection sont effectuées selon des techniques bien connues de l'homme de l'art. Par groupe protecteur temporaire des amines ou alcools, on entend les groupes protecteurs tels que ceux décrits dans Protective Groups in Organic

Synthesis, Greene T.W. et Wuts P.G.M., Ed. Wiley Intersciences 1999 et dans Protecting Groups, Kocienski P.J., 1994, Georg Thieme Verlag.

On peut citer par exemple des groupements protecteurs temporaires des amines : benzyles, carbamates (tels que *tert*-butyloxycarbonyle clivables en milieu acide, benzyloxycarbonyle clivables par hydrogénolyse) ; des acides carboxyliques : esters d'alkyle (tels que méthyle ou éthyle, *tert*-butyle hydrolysables en milieu basiques ou acides) et benzyliques hydrogénolysables ; des alcools ou des phénols tels que des éthers de tétrahydropyranyle, méthyloxyméthyle ou méthyléthoxyméthyle, *tert*-butyle et benzyle ; des dérivés carbonylés tels que les acétals linéaires ou cycliques comme par exemple 1,3-dioxane-2-yle ou 1,3-dioxolan-2-yle ; et se référer aux méthodes générales bien connues décrites dans Protective Groups, cité ci-dessus.

L'homme de l'art sera à même de choisir les groupes protecteurs appropriés. Les composés de formule (I) peuvent comporter des groupes précurseurs d'autres fonctions qui sont générées ultérieurement en une ou plusieurs autres étapes.

Une famille de composés selon l'invention est constituée par les dérivés d'indolin-2-one sous forme d'énantiomère pur ou de mélange d'énantiomères de formule :

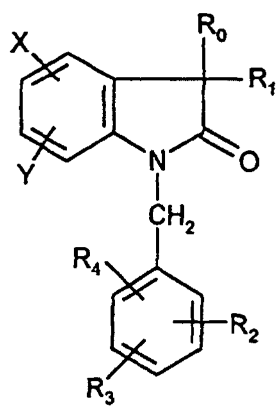

(I)

dans laquelle:

$R_0$ représente

(i) :

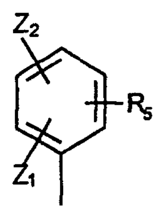

$Z_1$, $Z_2$, $R_1$, $R_2$, $R_3$, $R_4$, $R_5$. Y, X sont tels que définis pour (I) et leurs sels pharmaceutiquement acceptables, solvats et hydrates.

Selon un autre de ses aspects, l'invention concerne les composés de formule :

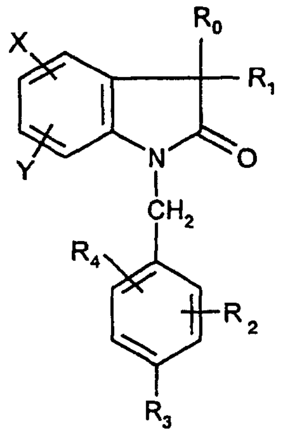

(Ia)

dans laquelle $R_1$ représente un groupe méthyle ou hydroxyle et $R_0$, $R_2$, $R_3$, $R_4$, X et Y sont tels que définis pour (I) ; sous forme d'énantiomère pur ou de mélange d'énantiomères, ainsi que leurs sels pharmaceutiquement acceptables solvats et hydrates.

Une sous famille des composés selon l'invention est constituée des composés de formule :

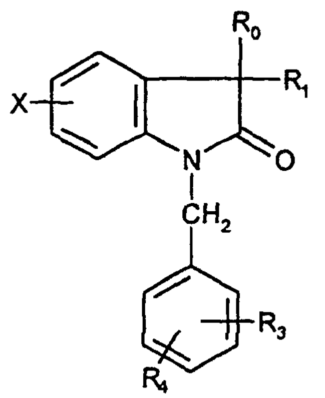

(Ib)

dans laquelle $R_1$ représente un groupe méthyle ou hydroxyle et $R_0$, $R_3$, $R_4$ et X sont tels que définis pour (I) ; sous forme d'énantiomère pur ou de mélange d'énantiomères, ainsi que leurs sels pharmaceutiquement acceptables solvats et hydrates.

Une autre sous famille des composés selon l'invention est constituée des composés de formule :

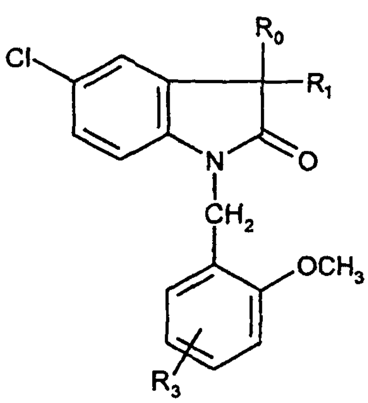

(Ic)

dans laquelle $R_1$ représente un groupe méthyle ou hydroxyle et $R_0$ et $R_3$ sont tels que définis pour (I) ; sous forme d'énantiomère pur ou de mélange d'énantiomères, ainsi que leurs sels pharmaceutiquement acceptables solvats et hydrates.

Une autre sous famille des composés selon l'invention est constituée des composés de formule :

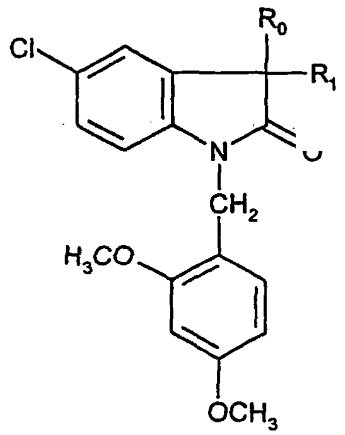

(Id)

dans laquelle $R_1$ représente un groupe méthyle ou hydroxyle et $R_0$ est tel que défini pour (I) ; sous forme d'énantiomère pur ou de mélange d'énantiomères, ainsi que leurs sels pharmaceutiquement acceptables solvats et hydrates.

Parmi ces composés de formule (I), (Ia), (Ib), (Ic) et (Id), ceux dans lesquels $R_0$ représente le groupe :

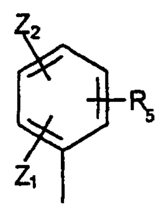

en particulier le groupe :

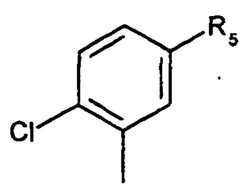

dans lequel $R_5$ est tel que défini pour (I) constituent un autre aspect de l'invention.

Parmi ces derniers composés, ceux dans lesquels $R_1$ représente un groupe méthyle constituent un autre aspect de l'invention.

Selon un autre de ses aspects, l'invention concerne les composés choisis parmi :

5-Chloro-3-(2-chlorophényl)-1-(2,4-diméthoxybenzyl)-3-méthylindolin-2-one (EXEMPLE 1) ;
5-Chloro-3-(2-chlorophényl)-1-[4-(isopropylamino)-2-méthoxybenzyl)-3-méthylindolin-2-one (EXEMPLE 56) ;
*N*-{4-Chloro-3-[5-chloro-1-(2,4-diméthoxybenzyl)-3-méthyl-2-oxoindolin-3-yl]phényl} acétamide (EXEMPLE 70) ;
*N*-{4-Chloro-3-[5-chloro-1-(2,4-diméthoxybenzyl)-3-méthyl-2-oxoindolin-3-yl]phényl}-3-méthylbutanamide (EXEMPLE 73) ;
*N*-{4-Chloro-3-[5-chloro-1-(2,4-diméthoxybenzyl)-3-méthyl-2-oxoindolin-3-yl]phényl} benzamide (EXEMPLE 74) ;
*N*-{4-Chloro-3-[5-chloro-1-(2,4-diméthoxybenzyl)-3-méthyl-2-oxoindolin-3-yl]phényl} nicotinamide (EXEMPLE 76) ;
*N*-{4-Chloro-3-[5-chloro-1-(2,4-diméthoxybenzyl)-3-méthyl-2-oxoindolin-3-yl]phényl}-2-méthoxyacétamide (EXEMPLE 77) ;
3-{4-Chloro-3-[5-chloro-1-(2,4-diméthoxybenzyl)-3-méthyl-2-oxoindolin-3-yl]anilino}-3-oxopropanoate de méthyle (EXEMPLE 78) ;
*N*-{4-Chloro-3-[5-chloro-1-(2,4-diméthoxybenzyl)-3-méthyl-2-oxoindolin-3-yl]phényl}-3-méthoxypropanamide

(EXEMPLE 81) ;
*N*-{4-Chloro-3-[5-chloro-1-(2,4-diméthoxybenzyl)-3-méthyl-2-oxoindolin-3-yl]phényl}-*N*-méthylacétamide (EXEMPLE 87) ;
*N*-{4-Chloro-3-[5-chloro-1-(2,4-diméthoxybenzyl)-3-méthyl-2-oxoindolin-3-yl]phényl}-*N*-méthylméthanesulfonamide (EXEMPLE 97) ;
4-Chloro-3-[5-chloro-1-(2,4-diméthoxybenzyl)-3-méthyl-2-oxoindolin-3-yl]-*N*,*N*-diéthyl benzamide (EXEMPLE 102) ;
4-Chloro-3-[5-chloro-1-(2,4-diméthoxybenzyl)-3-méthyl-2-oxoindolin-3-yl]-*N*,*N*-diméthyl benzamide (EXEMPLE 109) ;
5-Chloro-3-[2-chloro-5-(1-pipéridinylcarbonyl)phényl]-1-(2,4-diméthoxybenzyl)-3-méthyl indolin-2-one (EXEMPLE 112) ;
4-Chloro-3-[5-chloro-1-(2,4-diméthoxybenzyl)-3-méthyl-2-oxoindolin-3-yl]-*N*-éthyl benzamide (EXEMPLE 114) ;
5-Chloro-3-(2-chloro-5-{[2-(méthoxyméthyl)-1-pyrrolidinyl]carbonyl}phényl)-1-(2,4-diméthoxybenzyl)-3-méthylindolin-2-one (EXEMPLE 119) ;
5-Chloro-3-{2-chloro-5-[(2-méthyl-1-pipéridinyl)carbonyl]phényl}-1-(2,4-diméthoxybenzyl)-3-méthylindolin-2-one (EXEMPLE 122) ;
4-Chloro-3-[5-chloro-1-(2,4-diméthoxybenzyl)-3-méthyl-2-oxoindolin-3-yl]-*N*-éthyl-*N*-méthylbenzamide (EXEMPLE 124) ;
1-{4-Chloro-3-[5-chloro-1-(2,4-diméthoxybenzyl)-3-méthyl-2-oxoindolin-3-yl]benzoyl}-2-pipéridinecarboxylate de méthyle (EXEMPLE 131) ;
5-Chloro-3-{2-chloro-5-[(4-hydroxy-1-pipéridinyl)carbonyl]phényl}-1-(2,4-diméthoxybenzyl) -3-méthylindolin-2-one (EXEMPLE 134) ;
5-Chloro-3-{2-chioro-5-[(2-méthoxyéthoxy)méthyl]phényl}-1-(2,4-diméthoxybenzyl)-3-méthylindolin-2-one (EXEMPLE 142) ;
5-Chloro-3-[2-chloro-5-(4-morpholinylméthyl)phényl]-1-(2,4-diméthoxybenzyl)-3-méthyl indolin-2-one (EXEMPLE 148) ;
5-Chloro-3-(2-chloro-5-{[2-(4-morpholinyl)éthoxy]méthyl}phényl)-1-(2,4-diméthoxybenzyl)-3-méthylindolin-2-one (EXEMPLE 152) ;
1-[4-Chloro-3-[5-chloro-1-(2,4-diméthoxybenzyl)-3-méthyl-2-oxoindolin-3-yl]benzoyl]-3-hydroxypipéridine (EXEMPLE 194) ;
1-[4-Chloro-3-[5-chloro-1-(2,4-diméthoxybenzyl)-3-méthyl-2-oxoindolin-3-yl]benzoyl]-(R)-3-hydroxypipéridine (EXEMPLE 195) ;
1-[4-Chloro-3-[5-chloro-1-(2,4-diméthoxybenzyl)-3-méthyl-2-oxoindolin-3-yl]benzoyl]-4-méthoxypipéridine (EXEMPLE 166) ;
1-[4-Chloro-3-[5-chloro-1-(2,4-diméthoxybenzyl)-3-méthyl-2-oxoindolin-3-yl]benzoyl]-4-éthoxypipéridine (EXEMPLE 167) ;
1-[4-Chloro-3-[5-chloro-1-(2,4-diméthoxybenzyl)-3-méthyl-2-oxoindolin-3-yl]benzoyl]-(R,S)-2,6-diméthylpipéridine (EXEMPLE 189) ;
1-[4-Chloro-3-[5-chloro-1-(2,4-diméthoxybenzyl)-3-méthyl-2-oxoindolin-3-yl]benzoyl]-(R)-2-éthoxycarbonylpipéridine (EXEMPLE 175) ;
1-[4-Chloro-3-[5-chloro-1-(2,4-diméthoxybenzyl)-3-méthyl-2-oxoindolin-3-yl]benzoyl]-(R)-2-*N*,*N*-diméthylaminocarbonylpipéridine (EXEMPLE 169);
1-[4-Chloro-3-[5-chloro-1-(2,4-diméthoxybenzyl)-3-méthyl-2-oxoindolin-3-yl]benzoyl]-(R)-2-(*N*-méthyl-*N*-2,2,2-trifluoroéthylaminocarbonyl)pipéridine (EXEMPLE 170) ;
1-[4-Chloro-3-[5-chloro-1-(2,4-diméthoxybenzyl)-3-méthyl-2-oxoindolin-3-yl]benzoyl]-(R)-2-pyrrolidinocarbonylpipéridine (EXEMPLE 168) ;
1-[4-Chloro-3-[5-chloro-1-(2,4-diméthoxybenzyl)-3-méthyl-2-oxoindolin-3-yl]benzoyl]-(S)-2-méthylpipéridine (EXEMPLE 174) ;
4-Chloro-3-[5-chloro-1-(2,4-diméthoxybenzyl)-3-méthyl-2-oxoindolin-3-yl]-*N*-éthyl-*N*-(2-phényléthyl)benzamide (EXEMPLE 185) ;
4-Chloro-3-[5-chloro-1-(2,4-diméthoxybenzyl)-3-méthyl-2-oxoindolin-3-yl]-*N*-éthyl-*N*-(4-pyridyiméthyl)benzamide, chlorhydrate (EXEMPLE 188) ;
4-Chloro-3-[5-chloro-1-(2,4-diméthoxybenzyl)-3-méthyl-2-oxoindolin-3-yl]-*N*-éthyl-*N*-(3-pyridylméthyl)benzamide (EXEMPLE 201) ;
4-Chloro-3-[5-chloro-1-(2,4-diméthoxybenzyl)-3-méthyl-2-oxoindolin-3-yl]-*N*-éthyl-*N*-(2-pyridylméthyl)benzamide (EXEMPLE 200) ;
4-Chloro-3-[5-chloro-1-(2,4-diméthoxybenzyl)-3-méthyl-2-oxoindolin-3-yl]-*N*-éthyl-*N*-(2-méthoxyéthyl)benzamide (EXEMPLE 184) ;

4-Chloro-3-[5-chloro-1-(2,4-diméthoxybenzyl)-3-méthyl-2-oxoindolin-3-yl]-*N*-éthyl-*N*-(2-diméthylaminoéthyl)benzamide, chlorhydrate (EXEMPLE 177) ;
4-Chloro-3-[5-chloro-1-(2,4-diméthoxybenzyl)-3-méthyl-2-oxoindolin-3-yl]-*N*-éthyl-*N*-(2-morpholinoéthyl)benzamide (EXEMPLE 178) ;
4-Chloro-3-[5-chloro-1-(2,4-diméthoxybenzyl)-3-méthyl-2-oxoindolin-3-yl]-*N*-éthyl-*N*-(2-pyrrolidinoéthyl)benzamide, chlorhydrate (EXEMPLE 182) ;
4-Chloro-3-[5-chloro-1-(2,4-diméthoxybenzyl)-3-méthyl-2-oxoindolin-3-yl]-*N*-éthyl-*N*-(2-pipéridinoéthyl)benzamide, chlorhydrate (EXEMPLE 183) ;
4-Chloro-3-[5-chloro-1-(2,4-diméthoxybenzyl)-3-méthyl-2-oxoindolin-3-yl]-*N*-éthyl-*N*-(2-hydroxyéthyl)benzamide (EXEMPLE 198) ;
4-Chloro-3-[5-chloro-1-(2,4-diméthoxybenzyl)-3-méthyl-2-oxoindolin-3-yl]-*N*-éthyl-*N*-[2-(pyridin-4-yl)éthyl]benzamide, chlorhydrate (EXEMPLE 179) ;
4-Chloro-3-[5-chloro-1-(2,4-diméthoxybenzyl)-3-méthyl-2-oxoindolin-3-yl)-*N*-éthyl-*N*-(2,2,2-trifluoroéthyl)benzamide (EXEMPLE 180) ;
4-Chloro-3-[5-chloro-1-(2,4-diméthoxybenzyl)-3-méthyl-2-oxoindolin-3-yl]-*N*-méthyl-*N*-(2,2,2-trifluoroéthyl)benzamide (EXEMPLE 171) ;
4-Chloro-3-[5-chloro-1-(2,4-diméthoxybenzyl)-3-méthyl-2-oxoindolin-3-yl]-*N*-éthyl-*N*-isopropylbenzamide (EXEMPLE 187) ;
4-Chloro-3-[5-chloro-1-(2,4-diméthoxybenzyl)-3-méthyl-2-oxoindolin-3-yl]-*N*-(2-diméthylaminoéthyl)-*N*-(2,2,2-trifluoroéthyl)benzamide, chlorhydrate (EXEMPLE 202) ;
4-Chloro-3-[5-chloro-1-(2,4-diméthoxybenzyl)-3-méthyl-2-oxoindolin-3-yl]-*N*-cyclohexylbenzamide (EXEMPLE 192) ;
4-Chloro-3-[5-chloro-1-(2,4-diméthoxybenzyl)-3-méthyl-2-oxoindolin-3-yl]-*N*-éthyl-*N*-[3-(pyridin-4-yl)propyl]benzamide (EXEMPLE 204) ;

sous forme d'énantiomère pur ou de mélange d'énantiomères, ainsi que leurs sels pharmaceutiquement acceptables, solvats et hydrates.

Les composé de formule (I) peuvent être préparés selon le SCHEMA 1 suivant :

**SCHEMA 1**

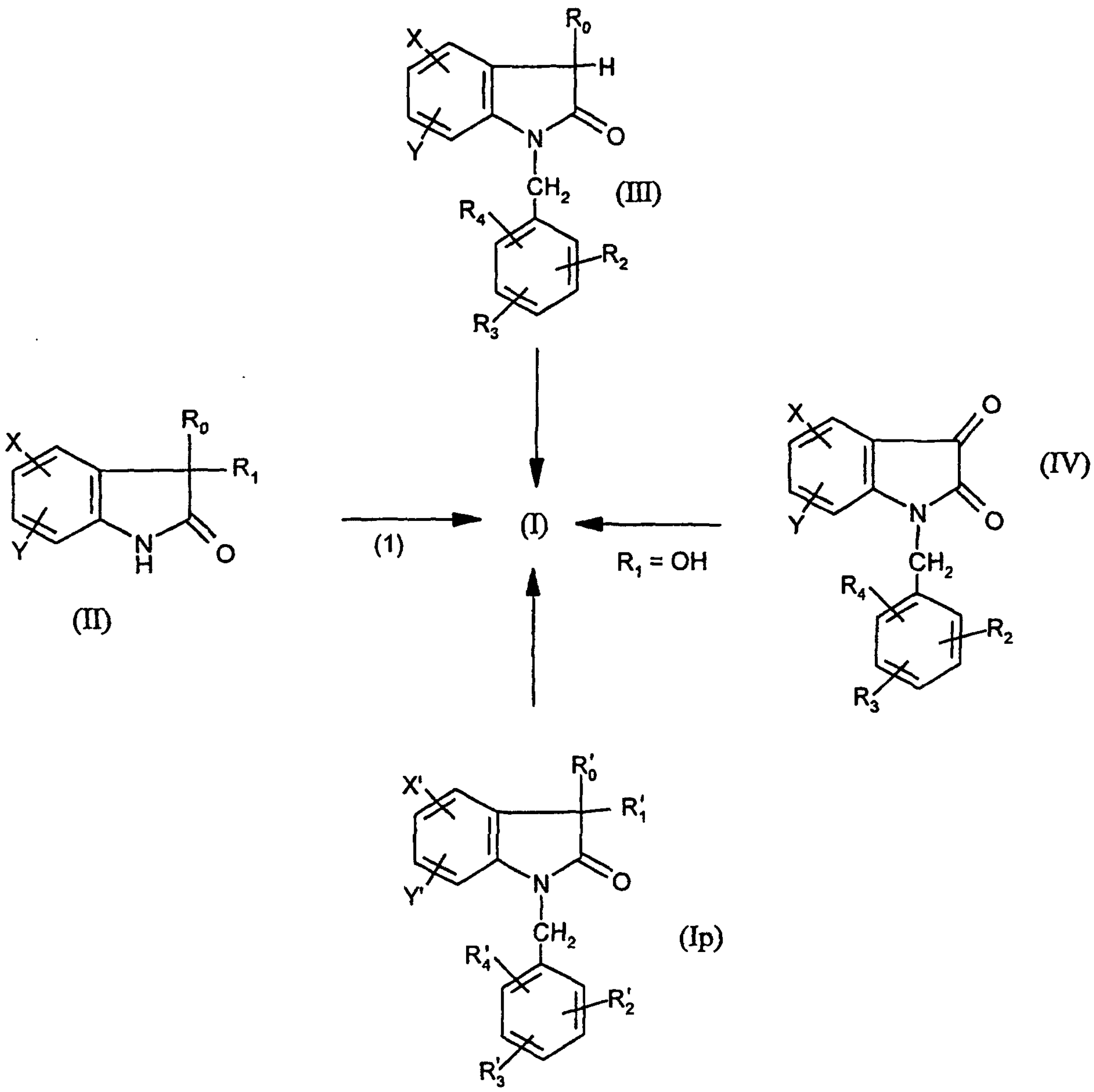


Dans ce schéma, $R_0$, $R_1$, $R_2$, $R_3$, $R_4$, X et Y sont tels que définis pour (I), et pour (Ip), $R'_0$, $R'_1$, $R'_2$, $R'_3$, $R'_4$, X' et Y' représentent respectivement soit $R_0$, $R_1$, $R_2$, $R_3$, $R_4$, X et Y tels que définis pour (I), soit un groupe précurseur de $R_0$, $R_1$, $R_2$, $R_3$, $R_4$, X et Y, étant entendu que $R'_1$ est différent de l'hydrogène.

La présente invention a également pour objet un procédé de préparation pour les composés de formule (I) caractérisé en ce que :

a) on fait réagir en présence d'une base un composé de formule :

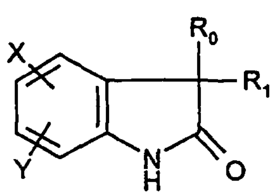
(II)

dans laquelle X, Y, $R_0$ et $R_1$ sont tels que définis pour (I), avec un halogènure de formule:

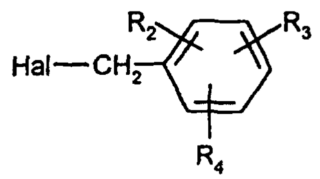

(1)

dans laquelle Hal représente un atome d'halogène et $R_2$, $R_3$, $R_4$ sont tels que définis pour (I) ;

b) ou bien, lorsque $R_1$ représente un groupe électrophile, on transforme le composé de formule:

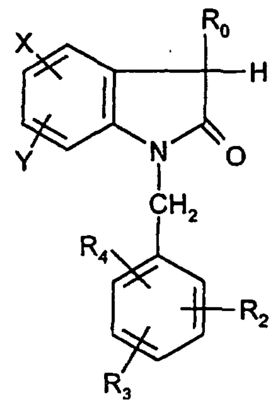

(III)

dans laquelle $R_0$, $R_2$, $R_3$, $R_4$, X et Y sont tels que définis pour (I), par l'action d'un dérivé $R_1$-Z, dans lequel Z représente un groupe partant, en présence d'une base ;

c) ou bien lorsque $R_1$ = OH, on met en réaction un dérivé de l'isatine de formule :

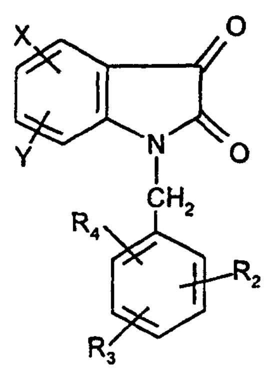

(IV)

dans laquelle $R_2$, $R_3$, $R_4$, X et Y sont tels que définis pour (I), avec un dérivé organométallique $R_0$-M ou $R_0$MgHal, $R_0$ étant tel que défini pour (I), M étant un atome de métal et Hal un atome de brome ou d'iode ;

d) ou bien on soumet le composé de formule :

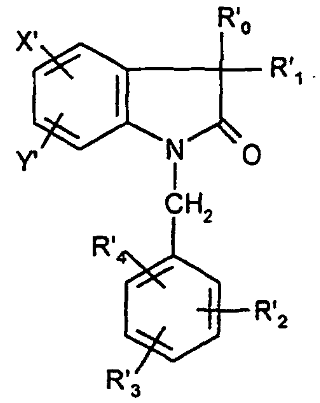

(Ip)

dans laquelle $R'_0$, $R'_1$, $R'_2$, $R'_3$, $R'_4$, X' et Y' représentent respectivement soit $R_0$, $R_1$, $R_2$, $R_3$, $R_4$, X et Y tels que définis pour (I), soit un groupe précurseur de $R_0$, $R_1$, $R_2$, $R_3$, $R_4$, X et Y, à un traitement ultérieur pour transformer l'un quelconque des groupes $R'_0$, $R'_1$, $R'_2$, $R'_3$, $R'_4$, X' ou Y' en respectivement, $R_0$, $R_1$, $R_2$, $R_3$, $R_4$, X ou Y tels que définis pour (I), selon des réactions bien connues de l'homme de l'art.

La réaction décrite en a) est de préférence effectuée avec un composé (I) dans lequel Hal = Cl ou Br, en utilisant comme base un hydrure métallique tel que l'hydrure de sodium ou un alcoolate alcalin comme le *tert*-butylate de potassium dans un solvant anhydre tel que le diméthylformamide ou le tétrahydrofurane.

Dans la réaction décrite en b) par groupe partant on entend par exemple un atome d'halogène tel que chlore, brome ou iode ou bien un groupe estersulfonique comme le *para*-toluènesulfonate. On fait de préférence réagir sur le composé (III) un halogènure $R_1$-Hal, $R_1$ étant tel que défini pour (I) et Hal étant un atome d'halogène de préférence d'iode en présence d'une base ; on opérera par exemple en présence d'une base comme un alcoolate alcalin tel que le *tert*-butylate de potassium, dans un solvant éthéré comme le tétrahydrofurane ou bien en présence d'un carbonate comme le carbonate de sodium, potassium ou césium dans un solvant tel que le diméthylformamide ou l'acétonitrile.

Avantageusement, dans la réaction décrite en c) on fait réagir sur le composé de formule (IV) un magnésien $R_0$Mg-Hal, $R_0$ étant tel que défini pour (I) ou (Ip) et Hal étant un atome de brome ou de préférence d'iode, ou bien, on fait réagir sur le composé (IV) un dérivé $R_0$M dans lequel M est de préférence un atome de lithium. Ce dérivé $R_0$Li est obtenu soit par lithiation directe par exemple par action du butyllithium ou du diisopropylamidure de lithium selon Heterocycles 1993, 35(1), 151-169, soit par une réaction d'échange halogène lithium selon Organolithium Methods, Pergamon Press, New York, 1988 ou J. Am. Chem. Soc. 1956, 2217. Ces réactions sont de préférence effectuées dans un solvant anhydre comme l'éther diéthylique ou le tétrahydrofurane.

La transformation du composé (Ip), précurseur du composé (I) décrite en d) s'effectue selon des techniques classiques.

Par ailleurs les composés (I) pourront être obtenus à partir d'un autre composé (I) par transformation d'un des substituants $R_0$, $R_1$, $R_2$, $R_3$, $R_4$, X ou Y en particulier $R_0$, $R_1$ ou $R_3$. Par exemple :

- les composés (I) dans lesquels $R_3$ = $-NH_2$ pourront être obtenus par réduction des composés (I) correspondants dans lesquels $R_3$ = $-NO_2$, par exemple par action d'acide chlorhydrique en présence d'étain dans un alcool comme l'éthanol ;
- les composés (I) dans lesquels $R_3$ représente un groupe ($C_1$-$C_4$)alkylamino ou di($C_1$-$C_4$)alkylamino pourront être obtenus à partir des composés (I) correspondants dans lesquels $R_3$ = $-NH_2$ par une réaction d'amination réductrice. On pourra se référer à J. Org. Chem., 1996, *61*, 3849-3862 et opérer par action d'un aldéhyde en ($C_1$-$C_4$)alkyle en présence de triacétoxyborohydrure de sodium ou encore à J. Am. Chem. Soc., 1974, *96(25)*, 7812 et opérer par action d'un acide en ($C_1$-$C_4$)alkyle en présence de borohydrure de sodium. On pourra également utiliser les réactions classiques de N-alkylation par exemple en faisant agir sur le groupe amino un halogènure de ($C_1$-$C_4$) alkyle en présence de diméthylformamide et de carbonate de potassium ;
- les composés (I) dans lesquels $R_3$ représente un ($C_1$-$C_4$)alcoxy pourront être obtenus à partir des composés (Ip) correspondants dans lesquels $R'_3$ = OH par une réaction classique de O-alkylation, par exemple par action d'un halogènure de ($C_1$-$C_4$)alkyle en présence de diméthylformamide et de carbonate de césium ou de potassium ;
- les composés (I) dans lesquels $R_3$ représente un groupe ($C_1$-$C_4$)alkylcarbonylamino pourront être obtenus à partir des composés (I) correspondants dans lesquels $R_3$= $-NH_2$ par une acylation classique telle que l'action d'un chlorure d'acide en ($C_1$-$C_4$)alkyle, en présence d'une base comme la triéthylamine, dans un solvant tel que le dichlorométhane ;

- les composés (I) dans lesquels $R_3$ représente une amine cyclique ou une morpholin-4-yle pourront être obtenus à partir des composés (I) correspondants dans lesquels $R_3$= $-NH_2$ selon la méthode décrite dans Tetrahedron, 1989, 45(3), 629-636.
- les composés de formule (I), dans lesquels $R_0$ représente un groupe :

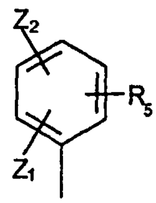

pourront être obtenus à partir des composés de formule (I) correspondants par transformation du groupe $R_5$ selon des réactions classiques par exemple d'alkylation, acylation, d'oxydation, réduction, amination bien connues de l'homme de l'art. Les composés (III) sont préparés par déshalogénation des composés de formule :

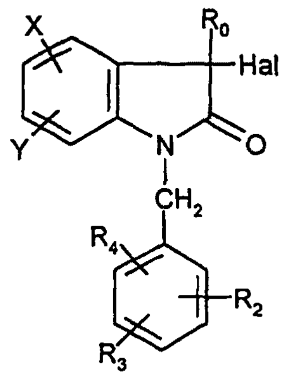

(I')

dans laquelle $R_0$, $R_2$, $R_3$, $R_4$, X et Y sont tels que définis pour (I) et Hal représente un atome de chlore, brome ou iode, par exemple, par action d'un dialkylamidure de lithium encombré tel que le diisopropylamidure de lithium (LDA) par analogie avec la méthode décrite par N. Newcom et *al*. dans J. Am. Chem. Soc. 1990, 5186-5193.

Le composé (I') est par exemple obtenu par transformation du composé (I) correspondant dans lequel $R_1$ = OH par action d'un dérivé halogéné par exemple de type halogénure d'acide. On pourra citer comme dérivé chloré $SOCl_2$.

Le composé (IV) est en général obtenu par action du composé (1) sur le dérivé de l'isatine de formule :

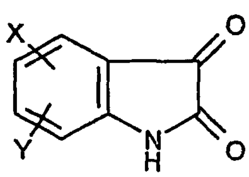

(2)

dans laquelle X et Y sont tels que définis pour (I) dans les mêmes conditions que celles décrites précédemment pour la préparation du composé (I) à partir du composé (II). Les dérivés de l'isatine (2) sont des composés commerciaux ou sont préparés selon les méthodes décrites dans Tetrahedron Letters 1998, *39*, 7679-7682 ; Tetrahedron Letters 1994, 35, 7303-7306 ; J. Org. Chem 1977, *42*, 1344-1348 et Advances in Heterocyclic Chemistry, A.R. Katritzky and A. J. Boulton, Academic Press, New York, 1975, *18*, 2-58.

Les composés (II) peuvent être synthétisés selon différentes méthodes décrites notamment dans les demandes de brevet EP 526348 et WO 95/18105.

Le SCHEMA 2 illustre certaines voies d'obtention des composés (II) :

## SCHEMA 2

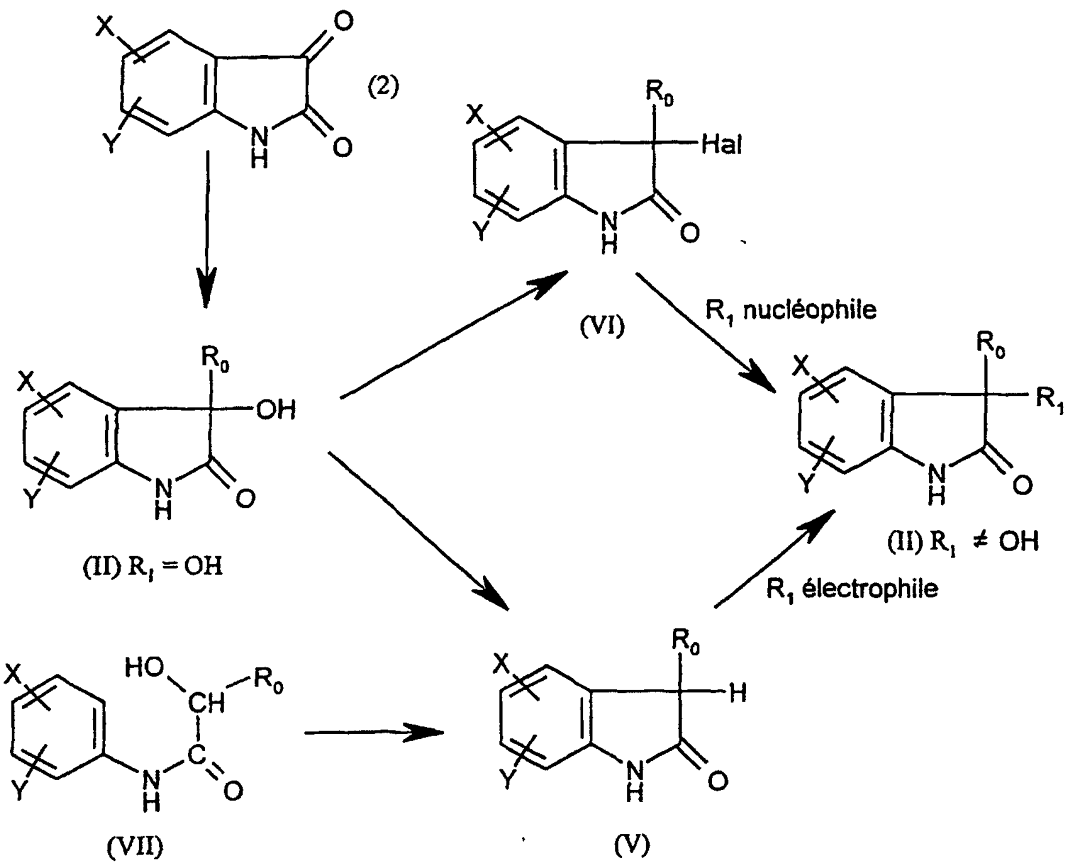


Par $R_1$ nucléophile , on entend un groupe $(C_1\text{-}C_4)$alcoxy.

Les composés (II) dans lesquels $R_1$ représente un groupe électrophile, par exemple un groupe $(C_1\text{-}C_4)$alkyle peuvent être préparés à partir des composés de formule :

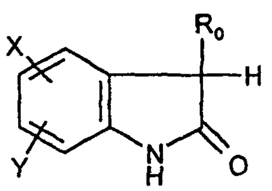

(V)

dans laquelle $R_0$, X et Y sont tels que définis pour (I), par action d'un dérivé $R_1$-Z dans lequel Z représente un groupe partant, dans les mêmes conditions que celles décrites précédemment pour le passage du composé (III) au composé (I).

Le composé (V) est en général synthétisé :

- soit par déshydroxylation du composé (II) correspondant dans lequel $R_1$ = OH, par action de chlorure d'étain en milieu acide selon la méthode décrite dans Tetrahedron 1996, 52(20), 7003-7012 ou par action du triéthylsilane selon Bioorganic and Medicinal Letters, 1997, 7(10), 1255-1260 ;
- soit par une réaction de cyclisation en milieu acide fort comme par exemple l'acide sulfurique, du composé de formule :

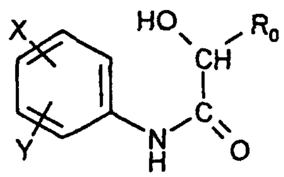

(VII)

dans laquelle $R_0$, X et Y sont tels que définis pour (I) ; ce composé (VII) étant lui-même obtenu par réaction de condensation entre un dérivé d'acide α-hydroxyacétique de formule :

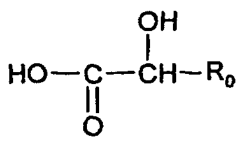

(VIII)

$R_0$ étant tel que défini pour (I) avec un amino benzène de formule :

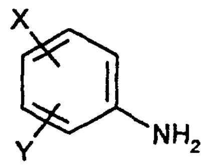

(3)

dans laquelle X et Y sont tels que définis pour (I).

Les composés (3) sont commerciaux ou synthétisés de manière classique.

Les composés de formule (VIII) sont commerciaux ou synthétisés selon des méthodes bien connues de l'homme de l'art. On pourra se référer notamment à J. Med. Chem., 1987, *30*(8), 1447.

D'autres réactions peuvent également conduire aux composés (V). On peut citer :

- la réaction de Brunner décrite dans Tetrahedron 1986, *42*(15), 4267-4272 :

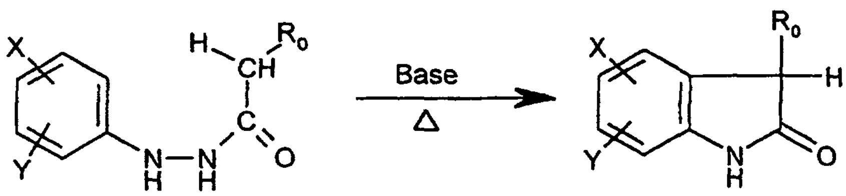


(V)

- la réaction de cyclisation en présence d'acide formique décrite dans J. Chem. Soc. Perkin Trans., 1986, *1*, 349-360 :

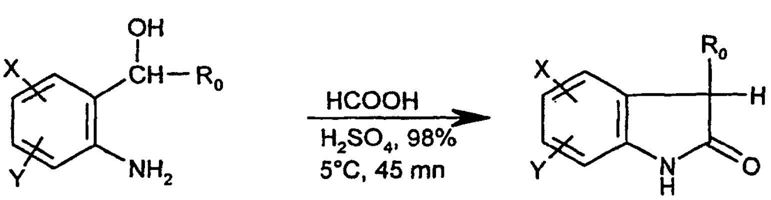


(V)

- les réactions de cyclisation suivantes :

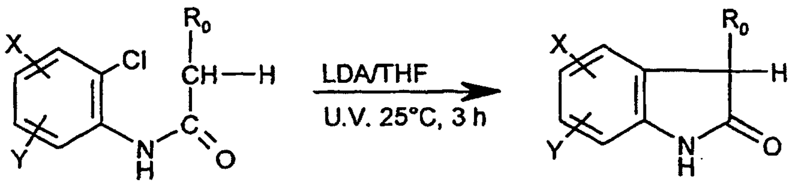


(V)

selon J. Am. Chem. Soc., 1985, *107(2)*, 435-443 :

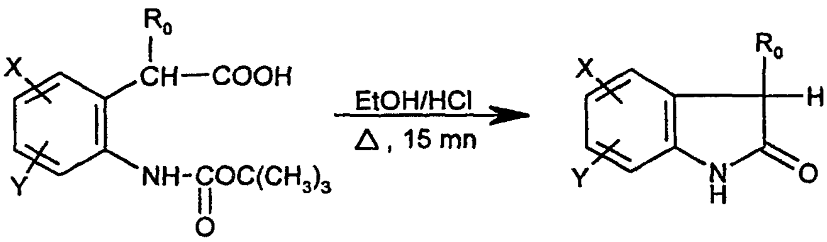


(V)

selon Tetrahedron 1996, *52*(20), 7003-7012.

Les composés (II) dans lesquels $R_1$ représente un groupe $(C_1-C_4)$alcoxy sont obtenus à partir des composés de formule :

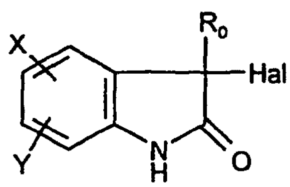

(VI)

dans laquelle $R_0$, X et Y sont tels que définis pour (I) et Hal représente un atome d'halogène par exemple de chlore par action de l'alcool $R_1H$ correspondant.

Le composé (VI) est préparé à partir du composé (II) correspondant dans lequel $R_1$ = OH, par action du chlorure de thionyle en présence de pyridine dans le dichlorométhane.

Les composés (II) dans lesquels $R_1$ = OH sont en général préparés à partir de l'isatine corresoondante de formule :

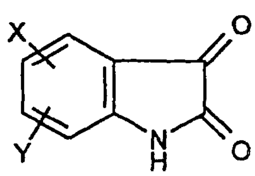

(2)

dans laquelle X et Y sont tels que définis pour (I), selon la méthode décrite précédemment pour la préparation des composés (I) dans lesquels $R_1$ = OH à partir des composés (IV).

Lorsque $R_1$ ne représente pas un groupe hydroxy, les composés (II) peuvent également être préparés selon le SCHEMA 3 ci-après :

**SCHEMA 3 ($R_1 \neq OH$)**

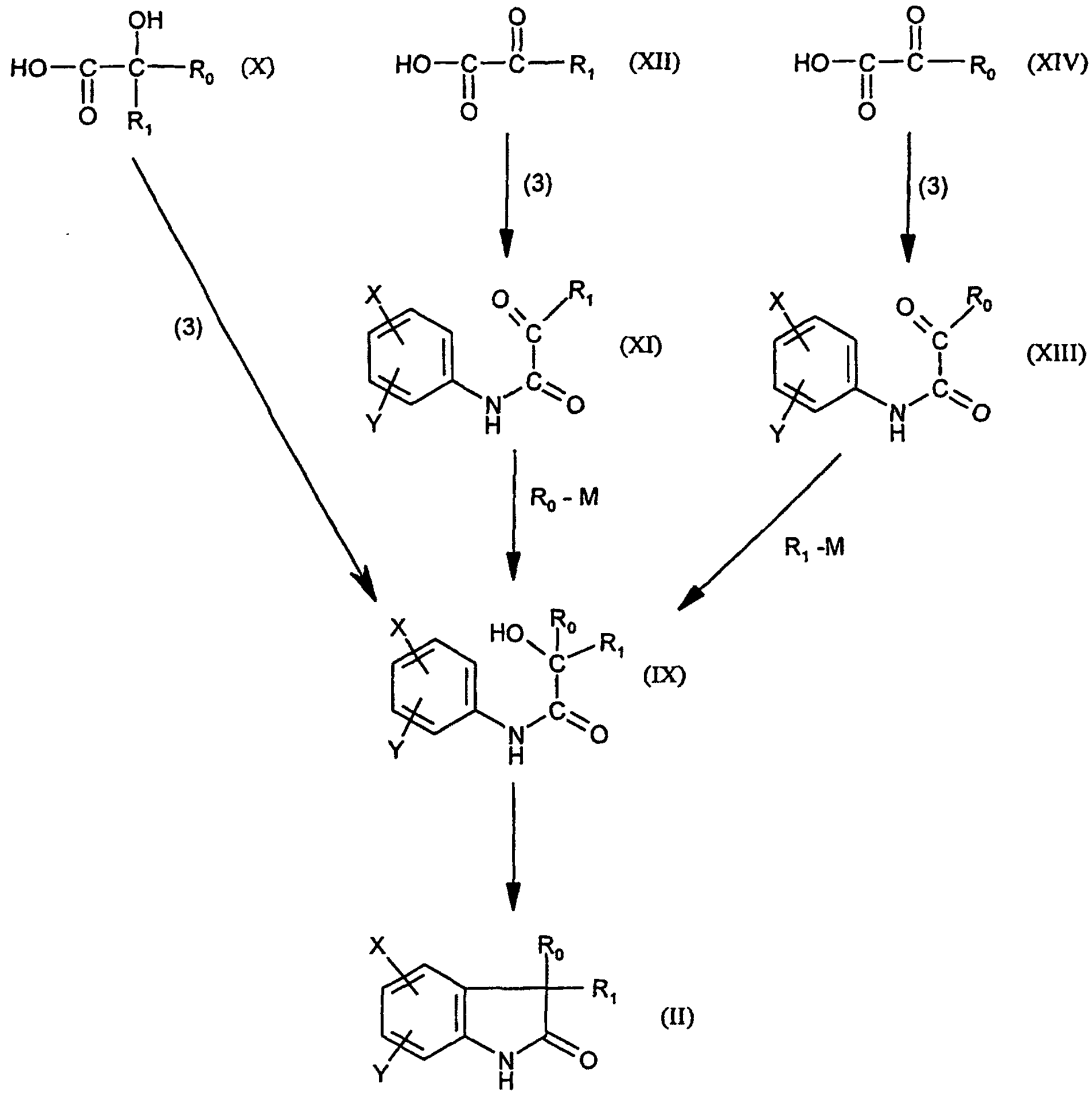


Dans ce SCHEMA 3, $R_0$, $R_1$, X et Y sont tels que définis pour (I) et $R_1$ ne représente pas un groupe hydroxy et M représente par exemple un atome de lithium ou MgHal, Hal étant un atome halogène.

Le passage du composé (X) au composé (IX) pour donner le composé (II) est notamment effectué selon la méthode décrite dans J. Chem. Soc. 1957, 1928.

Les halogénures de benzyle (1) sont connus ou préparés selon des méthodes connues. On peut citer par exemple J.V. Rajanbabu, J. Org. Chem., 1986, 51, 1704-1712 et les publications citées dans EP 636609.

D'une manière générale, les dérivés halogénométhylbenzène (1) peuvent être préparés par action des *N*-halogénosuccinimides sur les dérivés de méthylbenzène correspondants et selon EP 229566. La réaction est effectuée dans un solvant comme le tétrachlorure de carbone en présence de peroxyde de dibenzoyle. On peut également préparer un dérivé d'halogénométhylbenzène à partir d'un dérivé d'hydroxyméthylbenzène correspondant par action du tribromure de phosphore dans l'éther diéthylique ou par action du chlorure de thionyle.

A tous les stades du procédé, on peut former intermédiairement un composé intermédiaire de type (IIp), (IIIp), (IVp). dans lequel au moins un des substituants est remplacé par un de ses groupes précurseurs. Ces composés (IIp), (IIIp), (IVp) seront transformés par des réactions classiques en respectivement (II), (III), (IV). L'homme de l'art sera à même d'adapter les réactions ci-dessus mentionnées aux composés (IIp), (IIIp), (IVp).

Les composés selon l'invention ont fait l'objet d'études biochimiques et pharmacologiques. L'affinité des composés selon l'invention pour les récepteurs de l'ocytocine a été déterminée dans un test de liaison *in vitro* en utilisant la méthode décrite par J. Elands et *al.* dans Eur. J. Pharmacol. 1987, *147*, 197-207. Cette méthode consiste à étudier

*in vitro* le déplacement d'un analogue radioiodé de l'ocytocine aux récepteurs de l'ocytocine dans une préparation membranaire de récepteurs ocytociques humains utérins. Les $CI_{50}$ (concentration inhibitrice de 50 % de la liaison de l'analogue radioiodé de l'ocytocine à ses récepteurs) sont faibles et varient de $10^{-10}$ à $10^{-6}$ M dans ce dernier test.

L'affinité des composés selon l'invention pour les récepteurs humains $V_{1a}$ (méthode décrite par M. Thibonnier *et al*. dans J. Biol. Chem. 1994, 269, 3304-3310) $V_{1b}$ (méthode décrite par T. Sugimoto *el al*. dans J. Biol. Chem. 1994, 269, 27088-27092) et $V_2$ (méthode décrite par M. Bimbaumer *et al*. dans Nature (Lond.). 1992, 357, 333-335) de la vasopressine a également été étudiée. Les composés étudiés sont peu ou pas affins pour les récepteurs $V_{1a}$ $V_{1b}$ et $V_2$. A titre indicatif, le composé de l'EXEMPLE 1 présente une $CI_{50}$ inférieure à 50 nM, les $CI_{50}$ sur les récepteurs $V_{1a}$, $V_{1b}$ et $V_2$ étant supérieures à 1 μM.

Le caractère agoniste ou antagoniste des composés est déterminé *in vitro* dans un test de mesure de calcium intracellulaire sur cellules exprimant les récepteurs ocytociques humains selon la technique générale décrite dans (Am. J. Physiol. 268 (Heart Circ. Physiol. 37), 1995, H404-H410.

Lorsque les composés selon l'invention se comportent comme des antagonistes, leur $CI_{50}$ est avantageusement comprise entre 0,5 μM et 0,5 nM. A titre d'exemple, l'énantiomère dextrogyre de l'EXEMPLE 1 est antagoniste avec une $CI_{50}$ de 3,2 ± 1,9 nM.

Les composés selon l'invention, ligands puissants et sélectifs des récepteurs de l'ocytocine sont particulièrement intéressants et ce, dans la prévention et/ou le traitement des désordres ocytocine-dépendants. Les composés selon la présente invention peuvent soit mimer, soit inhiber les effets de l'ocytocine.

Ils seront particulièrement intéressants dans la cicatrisation, dans l'analgésie et l'anxiolyse (prévention de la douleur et de l'anxiété), la dépression, la schizophrénie, l'autisme, le syndrome obsessionnel compulsif, dans le comportement maternel (facilitation de la reconnaissance et de l'acceptation mère-enfant) et social, la mémoire, la régulation de la prise de nourriture et de boisson, la dépendance aux drogues, le sevrage et la motivation sexuelle. Ils pourront être utilisés avantageusement dans les désordres de la sphère urogènitale, notamment dans les domaines obstétrique et gynécologique, notamment comme agent tocolytique ou relaxant utérin ou pour contrôler les contractions de l'utérus avant que la grossesse soit arrivée à terme, pour contrôler le travail prénatal ou encore contrôler le travail préparatoire en vue d'un accouchement par césarienne, pour résoudre les problèmes de stérilité, fertilité, contrôler les naissances (usage vétérinaire en particulier), contrôler l'oestrus, l'arrêt de l'allaitement, le sevrage, le transfert et l'implantation d'embryons ; traiter l'endométriose, les dysménorrhées ainsi que l'incontinence urinaire à l'effort ou d'urgence, l'hypertrophie bénigne de la prostate et les dysfonctions érectiles, l'hypertension, l'hyponatrémie, l'insuffisance cardiaque, l'arthérosclérose, l'angiogénèse et réguler le stockage des graisses par l'adipocyte.

Par ailleurs, étant donné le rôle de l'ocytocine sur le contrôle de l'hormone lutéinisante (J.J. Evans, J. Endocrin. 1996, *151*, 169-174) les composés de l'invention pourront être utilisés pour induire la contraception.

De plus, les composés selon l'invention pourront être utilisés pour leurs effets anti-tumoraux, dans les tumeurs ocytocine sécrétantes, en particulier les cancers mammaires et prostatiques.

L'utilisation des composés selon l'invention pour la prévention et/ou le traitement des maladies ci-dessus mentionnées, ainsi que pour la préparation de médicaments destinés à traiter ces maladies fait partie intégrante de l'invention.

La présente invention a donc également pour objet des compositions pharmaceutiques contenant un composé selon l'invention ou d'un sel, d'un solvat ou d'un hydrate pharmaceutiquement acceptable de celui-ci, et des excipients convenables.

Lesdits excipients sont choisis selon la forme pharmaceutique et le mode d'administration souhaité : orale, sublinguale, sous-cutanée, intramusculaire, intra-veineuse, topique, intratrachéale, intranasale, transclermique, rectale ou intraoculaire. Les compositions pharmaceutiques sont préparées selon les techniques connues de l'homme de l'art.

Afin d'obtenir l'effet prophylactique ou thérapeutique désiré, chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Les composés selon l'invention pourront également être utilisés, pour la préparation de compositions à usage vétérinaire destinées à réguler les naissances.

Les composés selon l'invention pourront aussi être utilisés pour la préparation de compositions cosmétiques. Ces formulations pourront se présenter sous forme de crème pour utilisation topique et seront destinées à contrôler la lipolyse.

Les compositions de la présente invention peuvent contenir, à côté des produits de formule (I) ci-dessus ou de leurs sels, solvats et hydrates pharmaceutiquement acceptables et par exemple des principes actifs qui peuvent être utiles dans le traitement des troubles ou maladies indiquées ci-dessus. Ainsi, la présente invention a également pour objet des compositions pharmaceutiques contenant plusieurs principes actifs en association dont l'un est un composé selon l'invention. En particulier, la présente invention concerne des compositions pharmaceutiques contenant

un composé selon l'invention, antagoniste des récepteurs de l'ocytocine avec un composé $V_{1a}$ antagoniste. Ce type de composition sera particulièrement utile dans le traitement des dysménorrhées, de l'endométriose, le contrôle du travail prématuré et pour contrôler le travail préparatoire en vue d'un accouchement par césarienne.

L'invention a, en outre, pour objet des produits contenant un antagoniste des récepteurs de l'ocytocine tel que défini précédemment et un antagoniste des récepteurs $V_{1a}$ de la vasopressine pour une utilisation simultanée, séparée ou étalée dans le temps dans le traitement des dysménorrhées, de l'endométriose, le contrôle du travail prématuré et pour contrôler le travail préparatoire en vue d'un accouchement par césarienne.

Les PREPARATIONS et EXEMPLES suivants illustrent l'invention sans toutefois la limiter.

Les spectres de résonance magnétique nucléaire ont été effectués dans le chloroforme deutérié sauf mention contraire, à 200 MHz et les déplacements chimiques sont exprimés en p.p.m. Les abréviations utilisées ci-après sont les suivantes : s = singulet ; m = multiplet ; d = doublet ; t = triplet ; q = quintuplet.

Tous les composés selon l'invention ont fait l'objet d'analyse élémentaire organique effectuée par combustion à 1000°C en présence d'oxygène, en utilisant une balance de type Supermicro S4 Sartorius et un analyseur élémentaire de type EA 1108. Les analyses centésimales des éléments carbone, hydrogène, azote et soufre obtenues sont en accord avec les résultats théoriques attendus.

**PREPARATIONS**

**PREPARATION 1**

***N*-(4-Chlorophényl)-2-oxopropionamide, composé XI.1.**

A 22 g de chlorure de 2-oxopropionyle (préparé selon Synthesis 1975, 163-164 à partir de l'acide 2-oxopropionique et du 1,1-dichlorodiméthyléther) dans 350 ml de dichlorométhane, on additionne à -60°C 26,3 g de 4-chlorophénylamine dans 150 ml de dichlorométhane et 35 ml de triéthylamine. On agite le mélange réactionnel à -60°C pendant 2 heures puis on additionne à -30°C 200 ml d'une solution aqueuse d'acide chlorhydrique à 0,15 N et 500 ml de dichlorométhane. La phase organique est extraite, lavée avec une solution aqueuse d'acide chlorhydrique 0,25 N et séchée sur sulfate de sodium. On évapore les solvants sous pression réduite, le résidu obtenu est cristallisé dans le dichlorométhane ; F = 151°C.

**PREPARATION 2**

**2-(2-Chloro-4-fluorophényl)-*N*-(4-chlorophényl)-2-hydroxypropionamide, composé IX.1.**

On agite à reflux 0,18 g de magnésium et 2,57 g de 2-chloro-4-fluoro-1-iodobenzène dans 30 ml d'éther diéthylique. Le mélange ainsi obtenu est additionné à -60°C à 0,99 g du composé XI.1 dans 9 ml de tétrahydrofurane. On agite le mélange réactionnel à 20°C pendant 2 heures puis on additionne une solution aqueuse saturée en $NH_4Cl$. On extrait à l'acétate d'éthyle, sèche la phase organique sur $Na_2SO_4$ et évapore les solvants sous pression réduite. Le résidu obtenu est purifié par chromatographie sur une colonne de gel de silice en éluant avec un mélange cyclohexane/dichlorométhane 1/1 (v/v) puis dichlorométhane/méthanol 99/1 (v/v). Le solide ainsi isolé est cristallisé dans l'éther diisopropylique ; F = 167°C.

De la même manière, on prépare les composés suivants :

***N*-(4-Chlorophényl)-2-(2,5-diméthoxyphényl)-2-hydroxypropionamide, composé IX.2** ; F = 145°C.
***N*-(4-Chlorophényl)-2-(2-chloro-4-méthylphényl)-2-hydroxypropionamide, composé IX.3** ; F = 116°C.
***N*-(4-Chlorophényl)-2-(2-chloro-5-méthylphényl)-2-hydroxypropionamide, composé IX.4** ; F = 147°C.
***N*-(4-Chlorophényl)-2-(2-chloro-5-fluorophényl)-2-hydroxypropionamide, composé IX.5** ; F = 171°C.

**PREPARATION 3**

**(2-Chlorophényl)-*N*-(4-chlorophényl)-hydroxyacétamide, composé VII.1.**

Un mélange de 60 g d'acide (2-chlorophényl)hydroxyacétique et de 41 g de 4-chlorophénylamine dans 300 ml de 1,2-dichlorobenzène est chauffé à 200°C. Le montage comprend un Dean-Stark, ainsi l'eau formée est éliminée au cours de la réaction. On distille environ 150 ml de solvant et cristallise le composé attendu à 20°C.

Le solide obtenu est rincé à l'éther diisopropylique ; F = 120°C.

De la même façon, on prépare le **(2-chlorophényl)-*N*-(4-méthoxyphényl)-hydroxyacétamide** , composé VII.2 à partir de la 4-méthoxyphénylamine ; F = 130°C.

De la même façon, on prépare le **(2-chloro-4-fluorophényl)-*N*-(4-chlorophényl)-hydroxyacétamide,** composé VII.3 à partir de l'acide (2-chloro-4-fluorophényl) hydroxyacétique (synthétisé selon J. Med. Chem., 1987, *30*(8), 1447 à partir du 2-chloro-4-fluorobenzaldéhyde et du bromoforme) ; F = 136°C.

**PREPARATION 4**

**5-Chloro-3-(2-chlorophényl)indolin-2-one, composé V.1.**

(V.1) :

: $R_0$ = 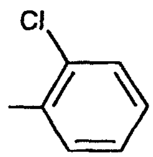 ;

X = 5-Cl ; Y = H

On prépare à 10°C une solution de 263 ml d'acide sulfurique à 95 % et 100 ml d'oléum à 20 %. On agite cette solution avec 74 g du *composé VII*. **1** pendant 2 heures à 40°C. Le mélange réactionnel est ensuite refroidi puis versé sur de l'eau glacée. Le précipité obtenu est filtré puis lavé avec 1000 ml d'eau. Le solide est dissous dans du dichlorométhane et la solution ainsi obtenue est lavée successivement avec une solution aqueuse saturée en hydrogénocarbonate de sodium et avec de l'eau puis séchée sur $Na_2SO_4$. On évapore les solvants sous pression réduite puis on lave le solide obtenu à l'éther diéthylique ; F = 201 °C.

De la même manière, on prépare le *composé V.2* ci-après :

**5-Chloro-3-(2-chloro-4-fluorophényl)indolin-2-one, composé V.2.**

(V.2) :

$R_0$ = 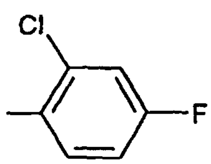 ;

X = 5-Cl ; Y = H
F = 221°C

**PREPARATION 5**

**5-Méthoxy-3-(2-chlorophényl)indolin-2-one, composé V.3**

Dans un mélange d'acide polyphosphorique obtenu à partir de 65 ml d'acide phosphorique à 85% et de 130 g d'anhydride phosphorique, à une température de 50°C, on ajoute 20,1 g de 2-(2-chlorophényl)-*N*-(4-méthoxyphényl)-2-hydroxyacétamide, *composé VII*.2 puis on maintient le mélange réactionnel à cette température pendant 6 heures. Après refroidissement, on traite avec une solution aqueuse d'hydrogénocarbonate de sodium jusqu'à obtenir un pH de 5. On extrait à l'acétate d'éthyle. La phase organique est lavée à l'eau, puis séchée sur sulfate de sodium anhydre. On évapore partiellement le solvant sous pression réduite et filtre le produit attendu ; F = 179°C.

**PREPARATION 6**

**5-Chloro-3-(2-chlorophényl)-3-méthylindolin-2-one, composé II.1.**

(II. 1) :

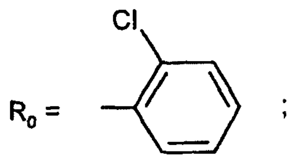

$R_1$ = $-CH_3$ ; X = 5-Cl ; Y = H

A une solution de 15 g du *composé V.1* dans 240 ml de tétrahydrofurane, on ajoute à - 40°C 18,2 g de *tert*-butylate de potassium. On agite le mélange réactionnel pendant 5 minutes à 0°C puis on additionne à -60°C une solution de 3,7 ml d'iodure de méthyle dans 80 ml de tétrahydrofurane. Une fois que la température du mélange réactionnel est revenue à 0°C, on additionne 100 ml d'une solution aqueuse saturée en chlorure d'ammonium et extrait à l'acétate d'éthyle. La phase organique est lavée à l'eau puis séchée sur sulfate de sodium anhydre. On évapore les solvants sous pression réduite. Le solide obtenu est purifié par chromatographie sur une colonne de gel de silice en éluant avec un mélange acétate d'éthyle/cyclohexane 1/9 (v/v). Le solide obtenu est cristallisé dans le *n*-pentane ; F = 185°C.

De la même manière, on prépare les *composés II.2* à *II.*6 ci-après.

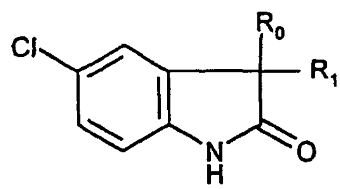

(II)

**TABLEAU 1**

| Composé | $R_0$ | $R_1$ | F ; °C |
|---|---|---|---|
| II.2 | 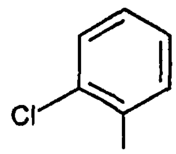 | $-CH_2-C\equiv CH$ | 219 |
| II.3 | 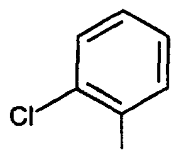 | $-CH_2-CH=CH_2$ | 218 |
| II.4 | 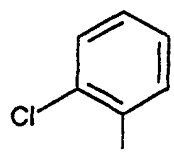 | $-CH_2CH_3$ | 198 |
| II.5 | 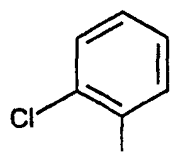 | $-CH_2CH_2CH_3$ | 218 |
| II.6 | 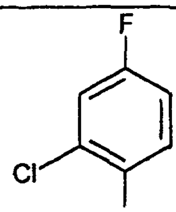 | $-CH_3$ | 189 |

Selon le même mode opératoire on prépare le **5-méthoxy-3-(2-chlorophényl)-3-méthyl indolin-2-one,** composé II.7 à partir du 5-méthoxy-3-(2-chlorophényl)indolin-2-one ; F = 176°C.

**PREPARATION 7**

**5-Chloro-3-(2-chloro-4-fluorophényl)-3-méthylindolin-2-one, composé II.6.**

(II.6) :

$R_0$ = 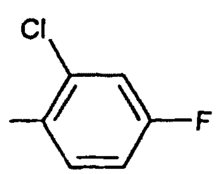 ;

$R_1$ = $-CH_3$ ; X = 5-Cl ; Y = H

Le *composé II.6* décrit précédemment peut également être préparé comme suit : On chauffe à 150°C pendant 5 heures 0,3 g du *composé IX.1* et une solution préparée préalablement de 5,3 g d'anhydride phosphorique dans 3 ml d'une solution aqueuse d'acide phosphorique à 85 %. On verse le mélange réactionnel sur de la glace, ajoute une

solution aqueuse saturée en carbonate de potassium et extrait à l'acétate d'éthyle. La phase organique ainsi obtenue est séchée sur sulfate de sodium anhydre. On évapore les solvants sous pression réduite. Le solide obtenu est cristallisé dans le *n*-pentane ; F = 189°C.

De la même manière, on prépare le composé **5-Chloro-3-(2,5-diméthoxyphényl)-3-méthylindolin-2-one, composé II.8 ;**

(II.8) :

$R_0$ = 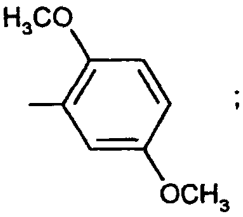 ;

$R_1$ = $-CH_3$ ; X = 5-Cl ; Y = H
F = 163°C

**PREPARATION 8**

**5-Chloro-3-[2-chloro-5-(4-méthyl-1-pipérazinyl)phényl]-3-méthylindol-2-one, Composé II.9**

(II.9) :

$R_0$ = 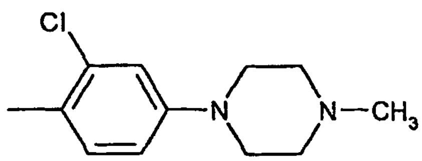 ;

; $R_1$ = $-CH_3$ ; X = 5-Cl ; Y = H

On chauffe à 120°C pendant 24 heures le mélange composé de 0,5 g du composé II.6, 2,5 ml de diméthylsulfoxyde, 3,6 ml de *N*-méthylpipérazine, 1 g de carbonate de sodium et 0,1 g d'iodure cuivreux. Après retour à température ambiante, on filtre les sels sur du talc, rince le précipité avec du diméthylsulfoxyde puis avec 60 ml d'acétate d'éthyle. Le filtrat est lavé avec 40 ml d'eau et la phase organique est séchée sur sulfate de sodium anhydre. On évapore les solvants sous pression réduite et purifie le résidu par chromatographie sur une colonne de gel de silice en éluant avec du dichlorométhane. On isole le produit attendu après reprise du résidu solide avec de l'éther diisopropylique puis filtration ; F = 155°C.

**PREPARATION 9**

**5-Chloro-3-(2-chloro-4-fluorophényl)-3-hydroxyindolin-2-one, composé II.10.**

(II. 10) :

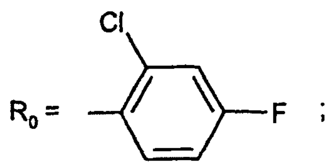

$R_1$ = -OH ; X = 5-Cl ; Y = H

A une suspension refroidie de 2 g de 5-chloroindolin-2,3-dione dans 60 ml de tétrahydrofurane on ajoute à -40°C 0,44 g d'une dispersion d'hydrure de sodium à 60 % dans l'huile et agite le mélange réactionnel pendant 15 minutes à 0°C. On agite au reflux pendant 3 heures 0,45 g de magnésium et 4,23 g de 2-chloro-4-fluoro-1-iodobenzène dans 18 ml d'éther diéthylique. La solution ainsi obtenue est lentement additionnée à -60°C au mélange réactionnel. On agite le mélange réactionnel pendant 30 minutes à 20°C et ajoute une solution aqueuse saturée en chlorure d'ammonium On extrait à l'acétate d'éthyle, sèche la phase organique sur sulfate de sodium anhydre et évapore les solvants sous pression réduite. Le résidu obtenu est purifié par chromatographie sur une colonne de gel de silice en éluant avec du dichlorométhane puis avec un mélange dichlorométhane/métanol 95/5 (v/v). Le solide obtenu est cristallisé dans le n-pentane ; F = 239°C.

De la même manière, on prépare à partir du 1-bromo-2,5-diméthoxybenzène le **5-Chloro-3-(2,5-diméthoxyphényl)-3-hydroxyindolin-2-one, composé II.11**

(II.11) :

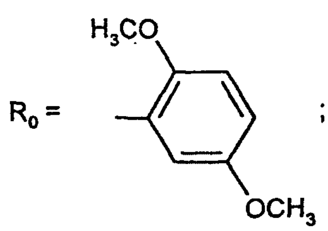

$R_1$ = -OH ; X = 5-Cl ; Y = H
F = 221°C

**PREPARATION 10**

**3,5-Dichloro-3-(2,5-diméthoxyphényl)indolin-2-one, composé VI.1.**

(VI.1):

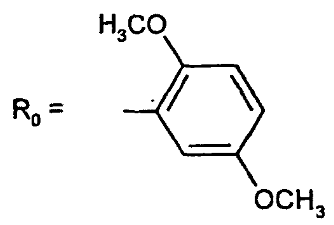

X = 5-Cl ; Y=H

A une température inférieure à 20°C, on additionne 0,8 ml de chlorure de thionyle à 3 g du *composé II.11* en présence de 1,2 ml de pyridine dans 50 ml de dichlorométhane puis agite le mélange réactionnel pendant une heure. Le mélange réactionnel est lavé à l'eau, séché sur sulfate de sodium anhydre. Les solvants sont évaporés sous pression réduite puis le résidu est chromatographié sur une colonne de gel de silice en éluant au dichlorométhane. F =157°C

De la même manière, on prépare les composés suivants :

**3,5-Dichloro-3-(2-chlorophényl)indolin-2-one, composé VI.2.**

(VI.2):

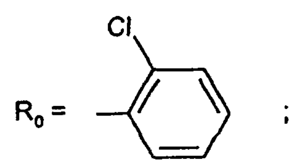

X = 5-Cl ; Y = H
F = 190°C

**3,5-Dichloro-3-(2-chloro-4-fluorophényl)indolin-2-one, composé VI.3.**

(VI.3):

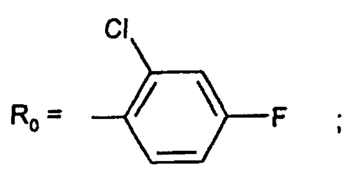

X = 5-Cl ; Y=H
F = 87°C

**PREPARATION 11**

**5-Chloro-3-(2,5-diméthoxyphényl)-3-méthoxyindolin-2-one, composé II.11.**

(II. 11) :

$R_0$ = 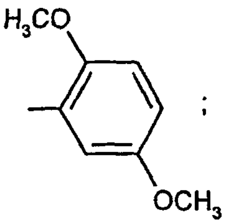 ;

$R_1$ = $-OCH_3$ ; X = 5-Cl ; Y = H

On maintient au reflux pendant 3 heures, 0,4 g du *composé VI.1* en présence de 25 ml de méthanol dans 50 ml de tétrahydrofurane. Les solvants sont évaporés sous pression réduite. F = 180°C.

De la même manière, on prépare les composés suivants :

**5-Chloro-3-(2-chlorophényl)-3-méthoxylndolin-2-one, composé II.12.**

(II.12) :

$R_0$ = 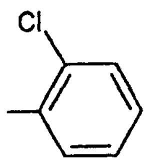 ;

$R_1$ = $-OCH_3$ ; X = 5-Cl ; Y = H
F = 179°C

**5-Chloro-3-(2-chloro-4-fluorophényl)-3-méthoxyindolin-2-one, composé II.13.**

(II. 13) :

$R_0$ = 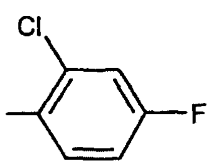 ;

$R_1$ = $-OCH_3$ ; X = 5-Cl ; Y = H
F = 82°C

**PREPARATION 12**

**5-Chloro-1-(2,4-diméthoxybenzyl)indolin-2,3-dione, composé IV.1.**

(IV.1) : $R_2$ = H ; $R_3$ = 4-$OCH_3$ ; $R_4$ = 2-$OCH_3$ ; X = 5-Cl ; Y=H

a) A une suspension de 1,45 g de 2,4-diméthoxyphénylméthanol dans 25 ml d'éther diéthylique, on ajoute à -50°C 0,25 ml de tribromure de phosphore. On laisse revenir la solution ainsi obtenue à une température de 0°C.
b) A une suspension de 1,3 g de 5-chloroindolin-2,3-dione dans 50 ml de tétrahydrofurane, on additionne à -60°C 2 g de *tert*-butylate de potassium. On agite le mélange réactionnel pendant 5 minutes à 0°C puis on additionne à -60°C la solution préparée en a). On agite le mélange réactionnel à température ambiante pendant 16 heures puis on évapore les solvants sous pression réduite. Le résidu obtenu est purifié par chromatographie sur une colonne de gel de silice en éluant avec un mélange cyclohexane/dichlorométhane variant de 8/2 à 2/8 (v/v). Le solide obtenu est cristallisé dans le toluène ; F = 175°C.

De la même manière on prépare les composés suivants :

**5-Chloro-1-(4-chloro-2-méthoxybenzyl)indolin-2,3-dione, Composé IV.2.**
F = 136°C
**5,7-Dichloro-1-(2,4-diméthoxybenzyl)indolin-2,3-dione, Composé IV.3.**
F = 171°C
**5-Fluoro-1-(2,4-diméthoxybenzyl)indolin-2,3-dione, Composé IV.4.**
F = 163°C
**1-(2,4-Diméthoxybenzyl)indolin-2,3-dione, Composé IV.5.**
F = 142°C

**EXEMPLE 1**

**5-Chloro-3-(2-chlorophényl)-1-(2,4-diméthoxybenzyl)-3-méthylindolin-2-one.**

(I):

$R_0$ = 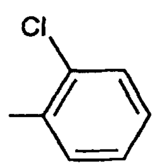 ;

$R_1$ = -$CH_3$ ; $R_2$ = H ;
$R_3$ = 4 -$OCH_3$ ; $R_4$ = 2 -$OCH_3$ ; X = 5-Cl ; Y = H

a) A une suspension de 2,6 g de 2,4-diméthoxyphénylméthanol dans 45 ml d'éther diéthylique, on ajoute à -50°C 0,48 ml de tribromure de phosphore. On laisse revenir la solution ainsi obtenue à une température de 0°C.
b) A 3 g du *composé II.1* en solution dans 90 ml de tétrahydrofurane, on ajoute à -40°C 1,2 g de *tert*-butylate de potassium puis agite le mélange réactionnel jusqu'à ce que la température soit revenue à 0°C. On refroidit ensuite le mélange réactionnel à - 60°C et on additionne la solution préparée en a). On agite le mélange réactionnel à 20°C pendant 2 heures, additionne 50 ml d'eau et extrait à l'acétate d'éthyle. On sèche les phases organiques sur sulfate de sodium et évapore les solvants sous pression réduite. Le solide obtenu est cristallisé dans l'éther diisopropylique ; F = 179°C.

Ce composé sous forme racémique est alors séparé par chromatographie sur une colonne Chiralpak® AD de Daïcel en éluant avec un mélange 2-méthylpentane/éthanol 98/2 (v/v).
On isole ainsi l'énantiomère dextrogyre : F = 92°C ; et son antipode $[\alpha]_D^{23,5}$ = + 39° (c = 1, $CH_3OH$)

**EXEMPLE 2**

**5-Méthoxy-3-(2-chlorophényl)-1-(2,4-diméthoxybenzyl)-3-méthylindolin-2-one.**

(I) :

$R_0$ = 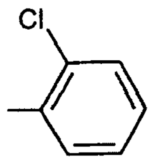 ;

$R_1$ = $-CH_3$ , $R_2$ = H ;
$R_3$ = 4 $-OCH_3$ ; $R_4$ = 2 $-OCH_3$ ; X = 5-$OCH_3$ ; Y = H

Selon le même mode opératoire, on prépare le composé de l'EXEMPLE 2 à partir du 5-méthoxy-3-(2-chlorophényl)indolin-2-one, *composé II.7*; F = 133°C.

**EXEMPLE 3**

**5-Chloro-3-(2-chlorophényl)-1-[4-(1,1-diméthyléthoxy)-2-méthoxybenzyl]-3-méthylindolin-2-one.**

(I):

$R_0$ = 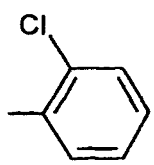 ;

$R_1$ = $-CH_3$ ; $R_2$ = H ;
$R_3$ = 4 $-OC(CH_3)_3$ ; $R_4$ = 2 $-OCH_3$ ; X = 5-Cl ; Y = H

a) Préparation du [4-(1,1-diméthyléthoxy)-2-méthoxy]phénylméthanol

- Préparation du [4-(1,1-diméthyléthoxy)-2-méthoxy]benzoate de méthyle selon J. Org. Chem. 1986, *51*, 111-113.
  A 6,2 g de 4-hydroxy-2-méthoxybenzoate de méthyle (selon J. Med. Chem. 1985, 28, 717-727 à partir du 2,4-dihydroxybenzoate de méthyle commercial) dans 60 ml de dichlorométhane, on additionne à -70°C 0,25 ml d'acide trifluorométhane sulfonique puis au moyen d'un tube plongeant on ajoute 25 ml de 2-méthylpropène préalablement condensé à -20°C et dégazé par réchauffement naturel. Après 24 heures d'agitation à une température comprise entre -30 et -70°C, on additionne au mélange réactionnel 0,5 ml de triéthylamine. On évapore les solvants sous pression réduite , reprend le résidu à l'acétate d'éthyle et lave avec une solution diluée de bicarbonate de sodium. La phase organique séparée est séchée sur sulfate de sodium anhydre et les solvants sont évaporés sous pression réduite. On isole le produit attendu, en purifiant par chromatographie sur une colonne de gel de silice en éluant avec du cyclohexane.
  $^1$HRMN : 7,75(d,1H) ; 6,62-653(m,2H) ; 3,85(s,3H) ; 3,84(s,3H) ; 1,40(s,9H)
- Selon J. Chem. Soc. Perkin Trans 1991, 3291-3294.
  A 2,5 g du composé précédent obtenu en a) dans 25 ml de toluène, on ajoute 15,90 ml d'une solution 2M de $LiBH_4$ dans du tétrahydrofurane. On chauffe le mélange réactionnel à 100°C pendant 45 minutes. Vers 20°C on verse le mélange réactionnel sur un mélange eau/glace et on extrait la phase aqueuse à l'acétate d'éthyle. Après décantation on extrait la phase aqueuse à l'acétate d'éthyle. Les phases organiques sont rassemblées

et séchées sur sulfate de sodium anhydre puis on évapore les solvants sous pression réduite.
$^{1}$HRMN : 7,10(d,1H) ; 6,59-6,50(m,2H) ; 4,61(d,2H) ; 3.81(s,3H) ; 2,20(t,1H) ; 1,34(s, 9H).

b) Le composé de l'EXEMPLE 3 est préparé selon le même mode opératoire que pour l'EXEMPLE 1 ; F = 131°C

**EXEMPLE 4**

**5-Chloro-3-(2-chlorophényl)-1-[4-(1-méthyléthoxy)-2-méthoxybenzyl]-3-méthylindolin-2-one.**

(I):

$R_0$ = 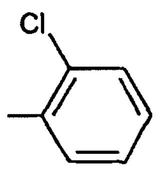

$R_1$ = $-CH_3$ ; $R_2$ = H ;
$R_3$ = 4 $-OCH(CH_3)_2$ ; $R_4$ = 2-$OCH_3$ ; X = 5-Cl ; Y = H

a) Préparation du [4-(1-méthyléthoxy)-2-méthoxy]phénylméthanol.

- Préparation du [4-(1-méthyléthoxy)-2-méthoxy]benzoate de méthyle selon Synthesis 1988,712.
  A 0,8 g de 4-hydroxy-2-méthoxybenzoate de méthyle dans 20 ml de diméthylformamide, on additionne à 0°C, 2,86 g de carbonate de césium puis 1,28 ml de 2-iodopropane. On agite le mélange réactionnel à 20°C pendant 2 heures puis on ajoute 50 ml d'eau et extrait à l'acétate d'éthyle. La phase organique est lavée à l'eau puis séchée sur sulfate de sodium anhydre. Les solvants sont évaporés sous pression réduite.
  $^{1}$HRMN : 7,81(d,1H) ; 6,47-6,42(m,2H) ; 4,69-4,51(m,1H) ; 3,85(s,3H) ; 3,83(s,3H) ; 1,33(d,6H).
- Le [4-(1-méthyléthoxy)-2-méthoxyphényl]méthanol est préparé selon la méthode décrite précédemment à l'EXEMPLE 3 pour le passage du [4-(1,1-diméthyléthoxy)-2-méthoxy]benzoate de méthyle au [4-(1,1-diméthyléthoxy)-2-méthoxy]phénylméthanol.

b) Le composé de l'EXEMPLE 4 est préparé selon le mode opératoire décrit pour l'EXEMPLE 1 ; F = 158°C.

Les EXEMPLES 5 à 17, ci-après sont préparés selon le mode opératoire décrit pour l'EXEMPLE 1.

**EXEMPLE 5**

**5-Chloro-3-(2-chlorophényl)-1-(2-méthoxy-4-nitrobenzyl)-3-méthylindolin-2-one.**

(I) :

$R_0$ = 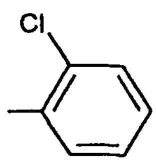 ;

$R_1$ = $-CH_3$ ; $R_2$ = H ;
$R_3$ = 4 $-NO_2$ ; $R_4$ = 2-$OCH_3$ ; X = 5-Cl ; Y = H
F = 179°C.

**EXEMPLE 6**

**5-Chloro-1-(2,4-diméthoxybenzyl)-3-(2,5-diméthoxyphényl)-3-méthylindolin-2-one**

(I) :

$R_0$ = 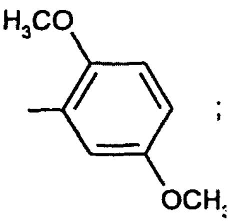 ;

$R_1$ = $-CH_3$ ; $R_2$ = H ;
$R_3$ = 4 $-OCH_3$ ; $R_4$ = 2-$OCH_3$; X = 5-Cl ; Y = H
F = 140°C (0,3 $H_2O$)

## TABLEAU 2

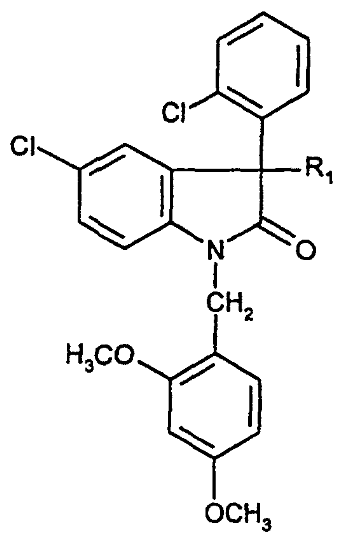

(I)

| EXEMPLE | $R_1$ | F ; °C ; sel, hydrate |
|---|---|---|
| 7 | $-CH_2-C\equiv CH$ | 135 |
| 8 | $-CH_2-CH=CH_2$ | 117 |
| 9 | $-CH_2CH_3$ | 102 |
| 10 | $-CH_2CH_2CH_3$ | 119 |

**TABLEAU 3**

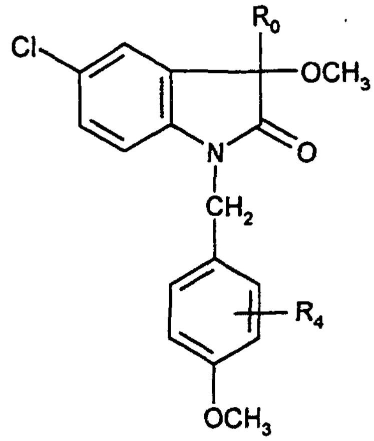

(I)

| EXEMPLE | $R_0$ | $R_4$ | F ; °C |
|---|---|---|---|
| 11 | 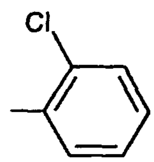 | 2-$OCH_3$ | 155 |
| 12 | 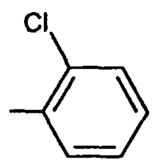 | 3-$OCH_3$ | 150 |
| 13 | 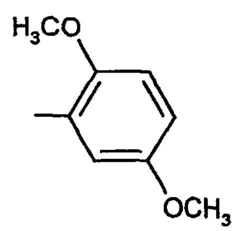 | 2-$OCH_3$ | 121 |
| 14 | 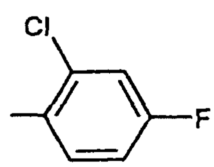 | 2-$OCH_3$ | cire |

**TABLEAU 4**

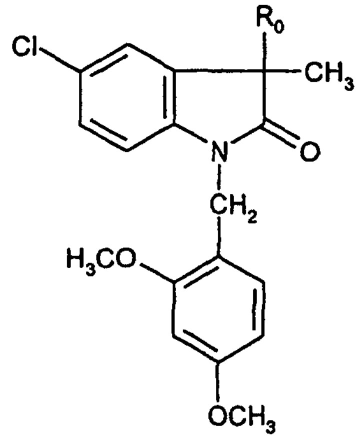

(I)

| EXEMPLE | $R_0$ | F ; °C |
|---|---|---|
| 15 | 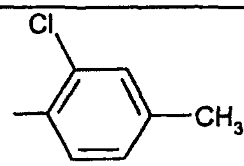 | 112 |
| 16 | 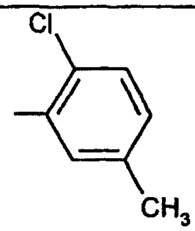 | 168 |
| 17 | 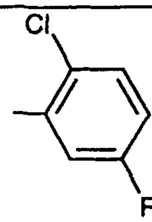 | 113 |

**EXEMPLE 18**

**5-Chloro-3-(2-chloro-4-fluorophényl)-1-(2,4-diméthoxybenzyl)-3-hydroxyindolin-2-one.**

(I) :

$R_0$ = 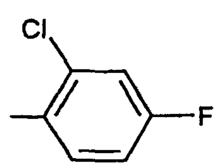 ;

$R_1$ = -OH ; $R_2$ = H ;

$R_3$ = 4 -$OCH_3$ ; $R_4$ = 2 -$OCH_3$ ; X = 5 -Cl ; Y = H

Ce composé peut être préparé à partir du *composé II.10* selon le même mode opératoire que pour l'EXEMPLE 1 ou bien selon la méthode ci-après :

On agite à reflux pendant 1 heure 0,87 ml de 2-chloro-4-fluoro-1-iodobenzène et 0,09 g de magnésium dans 15 ml d'éther diéthylique. A -40°C, on additionne 0,75 g du *composé IV.1* en solution partielle dans 15 ml de tétrahydrofurane. On agite le mélange réactionnel pendant 2 heures à 20°C puis on additionne une solution aqueuse saturée en chlorure d'ammonium. On extrait à l'acétate d'éthyle, sèche la phase organique sur sulfate de sodium anhydre puis évapore les solvants sous pression réduite. Le résidu obtenu est purifié par chromatographie sur une colonne de gel de silice en éluant avec un mélange cyclohexane/dichlorométhane 1/1 (v/v). Le solide obtenu est cristallisé dans le cyclohexane ; F = 177°C.

Ce composé sous forme racémique est séparé par chromatographie sur une colonne Chiralpak® AD de Daïcel en éluant avec un mélange 2-méthylpentane/éthanol 9/1 (v/v).

On isole ainsi l'enantiomère dextrogyre : et son antipode $[\alpha]_D^{20,5}$ = + 63° (c = 1, $CH_3OH$)

## EXEMPLE 19

**5-Chloro-3-(2-chloro-5-méthoxyméthoxyméthylphényl)-1-(2,4-diméthoxybenzyl)-3-hydroxyindolin-2-one.**

(I) :

$R_0$ = 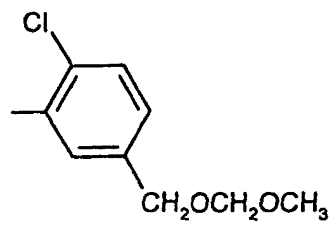 ;

$R_1$ = -OH ; $R_2$ = H ;
$R_3$ = 4 -$OCH_3$ ; $R_4$ = 2-$OCH_3$ ; X = 5-Cl ; Y = H

a) préparation du 2-chloro-1-iodo-5-hydroxyméthylbenzène selon J. Org. Chem. 1991, 56, 5964-5965, à partir de l'acide benzoique commercial correspondant.
A 25 g d'acide 4-chloro-3-iodobenzoique en solution dans 200 ml de tétrahydrofurane à 0°C, on ajoute par portions 5,02 g de borohydure de sodium puis 14,6 g d'iode en solution dans 50 ml de tétrahydrofurane très lentement. On agite le mélange réactionnel pendant 2 heures à température ambiante puis à 35°C pendant 30 minutes. On hydrolyse à 10°C avec une solution d'acide chlorhydrique 0,5N et extrait avec de l'acétate d'éthyle. La phase organique est séparée par décantation puis traitée par une solution aqueuse de bisulfite de sodium puis avec de l'eau. La phase organique est séchée sur sulfate de sodium anhydre et les solvants sont évaporés sous pression réduite. On obtient le composé attendu par distillation ;
$E_b$= 109°C sous 3 Pa.
b) Préparation du 2-chloro-1-iodo-5-méthoxyméthylèneoxyméthylbenzène selon Synthesis, 1985, 74.
A 24,45 g du composé précédent dans 100 ml de diméthoxyméthane, on ajoute 1,5 ml d'acide *para*-toluènesulfonique monohydrate et 1,4 g de bromure de lithium. On agite le mélange réactionnel à 35°C pendant 4 heures puis pendant 2 heures à reflux. A température ambiante on hydrolyse avec une solution aqueuse diluée de bicarbonate de sodium et extrait avec de l'éther diéthylique. On lave la phase organique à l'eau, la sèche sur sulfate de sodium anhydre, et évapore les solvants sous pression réduite. On obtient le produit attendu par distillation ;
$E_b$ = 108°C sous 1,9 Pa.
c) le composé de l'EXEMPLE 19 est préparé selon le mode opératoire décrit pour l'EXEMPLE 18 ; F = 142°C.

**EXEMPLE 20**

**4-Chloro-3-[5-chloro-1-(2,4-diméthoxybenzyl)-3-hydroxy-2-oxo-indolin-3-yl]benzoate de méthyle**

(I) :

$R_0$ = 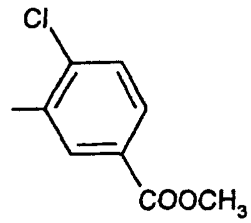 ;

$R_1$ = -OH ; $R_2$ = H
$R_3$ = 4-$OCH_3$ ; $R_4$ = 2-$OCH_3$ ; X = 5-Cl ; Y = H

A 10,72 g de 4-chloro-3-iodobenzoate de méthyle (préparé par estérification de l'acide commercial correspondant ; F = 56°C) dans 200 ml de tétrahydrofurane refroidi à - 100°C, on ajoute lentement 45,2 ml d'une solution de *n*-butyllithium à 1,6M dans l'hexane diluée dans 200 ml de tétrahydrofurane et refoidie à -90°C. On agite le mélange réactionnel pendant 20 minutes à -95°C puis ajoute la solution de 10 g du composé *IV.1* dans 600 mi de tétrahydrofurane refroidie à -70°C. Après retour à température ambiante, on hydrolyse avec 200 ml d'une solution saturée en chlorure d'ammonium, on évapore partiellement les solvants sous pression réduite, extrait à l'acétate d'éthyle, sèche la phase organique sur sulfate de sodium anhydre puis évapore les solvants sous pression réduite. Le résidu obtenu est lavé à l'éther diéthylique, filtré puis séché à 50°C sous pression réduite ; F = 236°C.

**EXEMPLE 21**

**3-(5-Amino-2-chlorophényl)-5-chloro-1-(2,4-diméthoxybenzyl)-3-hydroxyindolin-2-one**

(I) :

$R_0$ = 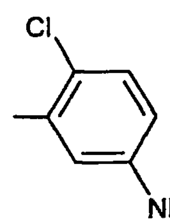 ;

$R_1$ = -OH ; $R_2$ = H ;
$R_3$ = 4-$OCH_3$ ; $R_4$ = 2-$OCH_3$ ; X = 5-Cl ; Y = H

a) Préparation de la 4-chloro-3-bromo-*N*,*N*-(tétraméthyléthylène)disilylaniline
On chauffe à 140°C pendant 5 heures sous courant d'argon, un mélange composé de 3,3 g de 3-bromo-4-chloroaniline, 3,72 g de bis(diméthylaminodiméthylsilyl)éthylène obtenue selon Tetrahedron letters 1984, 25(12), 1253-1254 et 0,03 g d'iodure de zinc. Le produit attendu est distillé ; $E_b$ = 105°C sous 37Pa.
b) le composé de l'EXEMPLE 21 est préparé selon le même mode opératoire décrit pour l'EXEMPLE 20 en purifiant par chromatographie sur une colonne de gel de silice en éluant avec un mélange dichlorométhane/méthanol : 99/1 (v/v) ; F = 133°C.

De la même manière que pour l'EXEMPLE 18, on prépare les composés des EXEMPLES 22 à 31 ci-après :

## TABLEAU 5

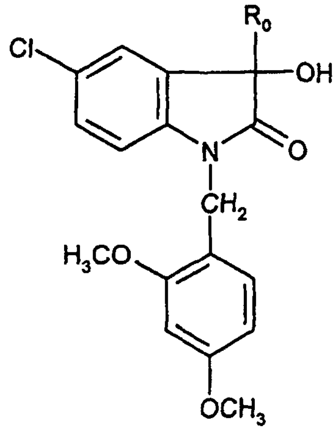

(I)

| EXEMPLE | $R_0$ | F ; °C ; sel, hydrate |
|---|---|---|
| 22 | 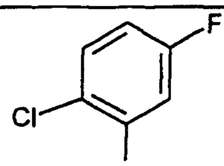 | 156 |
| 23 | 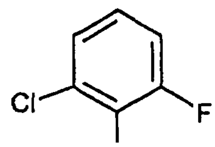 | 185 |
| 24 | 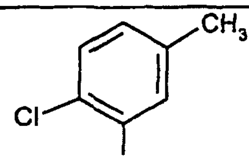 | 190<br>0,7 $H_2O$ |
| 25 | 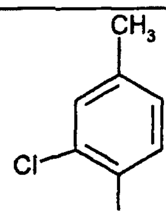 | 207 |
| 26 | 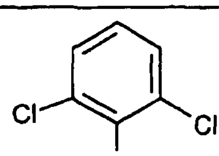 | 198 |
| 27 | 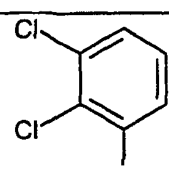 | 186 |

**TABLEAU 5 (suite)**

| EXEMPLE | $R_0$ | F ; °C ; sel, hydrate |
|---|---|---|
| 28 | 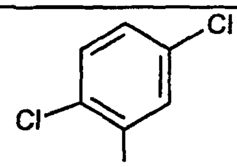 | 196 |
| 29 | 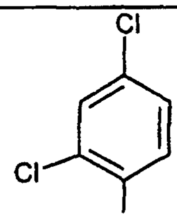 | 199 |
| 30 | 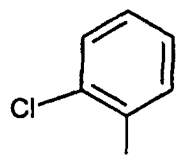 | 161 |
| 31 | 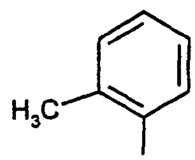 | 148 |

**EXEMPLE 32**

**5-Chloro-1-(2,4-diméthoxybenzyl)3-[5-(1,3-dioxolan-2-yl)-2-méthoxyphényl]-3-hydroxyindolin-2-one ,**

(I) :

$R_0$ = 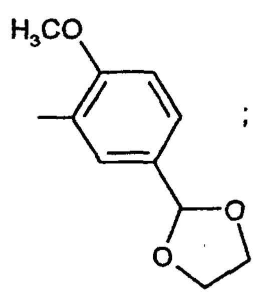 ;

$R_1$ = -OH ; $R_2$ = H ;
$R_3$ = 4-$OCH_3$ ; $R_4$ = 2-$OCH_3$ ; X = 5-Cl ; Y = H

a) Préparation du **2-(3-bromo-4-méthoxyphényl)-1,3-dioxolane** selon J. Med. Chem. 1990, 33(3), 972. Dans un réacteur muni d'un appareillage de Dean-Stark, on chauffe à reflux pendant 1 heure 30 minutes un mélange composé de 5 g de 3-bromo-*para*-anisaldéhyde, 5 ml d'éthylèneglycol, 0,088 g d'acide *para*-toluènesulfonique et 125 ml de toluène. A température ambiante, on verse le mélange réactionnel sur 50 ml d'eau, extrait à l'éther diéthylique et sèche la phase organique sur sulfate de sodium anhydre. Les solvants sont évaporés sous pression réduite. L'huile obtenue est purifiée par chromatographie sur une colonne de gel de silice en éluant avec un mélange cyclohexane/acétate d'éthyle 8/2 (v/v). On obtient le produit attendu après distillation sous pression réduite ; $E_b$ = 128°C sous 5 Pa.
b) A partir du composé précédent, on prépare le composé de l'EXEMPLE 32 selon le mode opératoire décrit pour l'EXEMPLE 18 ; F = 140°C.

**EXEMPLE 33**

**5-Chloro-1-(2,4-diméthoxybenzyl)-3-{5-[(diméthylamino)méthyl]-2-méthoxyphényl}-3-hydroxyindolin-2-one**

(I) :

$R_0$ = 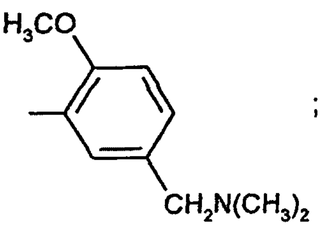 ;

$R_1$ = -OH ; $R_2$ = H
$R_3$ = 4-$OCH_3$ ; $R_4$ = 2-$OCH_3$ ; X = 5-Cl ; Y = H

a) 3-[5-Chloro-1-(2,4-diméthoxybenzyl)-3-hydroxy-2-oxoindolin-3-yl]-4-méthoxybenzaldé hyde obtenu par déprotection du composé de l'EXEMPLE 32 en milieu acide selon J. Chem. Soc. Chem. Commun 1987, 1351.
On porte à 30°C pendant 2 heures sous agitation, le mélange composé de 0,55 g du composé de l'EXEMPLE 32, 5 ml d'acétone, 2,5 ml d'eau et 0,22 ml d'acide chlrohydrique 1*N*. A température ambiante, on neutralise le mélange réactionnel avec une solution aqueuse de bicarbonate de sodium et extrait avec de l'acétate d'éthyle. On sèche la phase organique sur sulfate de sodium anhydre, évapore les solvants sous pression réduite et obtient le composé désiré par filtration du résidu d'évaporation repris dans de l'éther diéthylique ; F = 189°C.

b) Amination réductrice selon J. Org. Chem. 1996, *61*(11), 3849-3862.
A 0,113 g du composé précédent obtenu en a) en suspension dans 3 ml de 1,2-dichloroéthane, on ajoute 0,015 g de diméthylamine en solution dans 1 ml de 1,2-dichloroéthane puis 0,072 g de triacétoxyborohydrure de sodium. Après 15 heures d'agitation à température ambiante, on hydrolyse avec 10 ml d'eau et extrait à l'acétate d'éthyle. On sèche la phase organique sur sulfate de sodium, évapore les solvants sous pression réduite, purifie le résidu obtenu par chromatographie sur une colonne de gel de silice en éluant avec un mélange dichlorométhane/méthanol 95/5 (v/v). On obtient le produit attendu après cristallisation dans de l'éther isopropylique ; F = 162°C (0,4 $H_2O$).

**EXEMPLE 34**

**5-Chloro-3-(3-chloropyridin-4-yl)-1-(2,4-diméthoxybenzyl)-3-hydroxyindolin-2-one.**

$R_0$ = 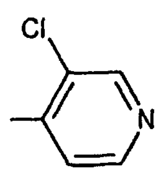 ;

$R_1$ = -OH ; $R_2$ = H ; $R_3$ = 4 -$OCH_3$ ; $R_4$ = 2 -$OCH_3$ ; X = 5-Cl ; Y=H

A une solution de 2,88 ml de diisopropylamidure de lithium 1,5M dans le cyclohexane, diluée dans 7 ml de tétrahydrofurane et refroidie à -75°C, on additionne goutte à goutte une solution de 0,414 ml de 3-chloropyridine dans 5 ml de tétrahydrofurane. Après l'addition, on agite le mélange réactionnel à -75°C pendant 20 minutes puis ajoute 1,2 g du *composé IV.1* dans 15 ml de tétrahydrofurane. On laisse remonter lentement la température du mélange réactionnel à 0°C puis hydrolyse avec 30 ml d'une solution aqueuse de chlorure d'ammonium. On extrait à l'acétate d'éthyle et sèche la phase organique sur sulfate de sodium. On évapore les solvants sous pression réduite et purifie le résidu obtenu par chromatographie sur une colonne de gel de silice en éluant avec un mélange cyclohexane/acétate d'éthyle 75/25 (v/v). Le solide obtenu est ensuite cristallisé dans l'acétate d'éthyle ; F = 215°C.

De la même manière on prépare les composés des EXEMPLES 35 et 36 ci-après :

**TABLEAU 6**

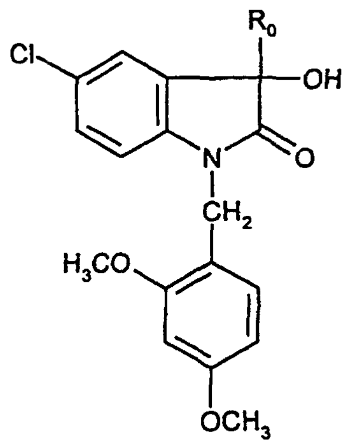

(I)

| EXEMPLE | $R_0$ | F ; °C ; sel, hydrate |
|---|---|---|
| 35 | 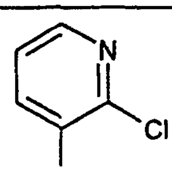 | 210 |
| 36 | 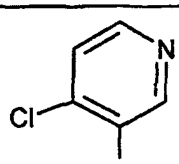 | 215 |

**PREPARATION 13**

**5-Chloro-3-(2-chlorophényl)-1-(2,4-diméthoxybenzyl)indolin-2-one,** ***composé III.1.***

(III.1):

$R_0$ = 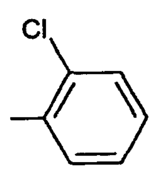 ;

$R_1$ = H ; $R_2$ = H ; $R_3$ = 4-$OCH_3$ ; $R_4$ = 2-$OCH_3$ ;
X = 5-Cl ; Y=H

a) **3,5-Dichloro-3-(2-chlorophényl)-1-(2,4-diméthoxybenzyl)indolin-2-one, *composé I'.1*.**
A une solution de 2 g du composé de l'EXEMPLE 30 et de 1,4 ml de pyridine dans 24 ml de dichlorométhane, on additionne à -20°C, 0,98 ml de chlorure de thionyle. On agite le mélange réactionnel pendant 1 heure 30 mn à température ambiante, le refroidit à 0°C puis ajoute 50 ml d'eau et 50 ml de dichlorométhane. On décante, lave la phase organique avec une solution aqueuse de $NaHCO_3$, sèche sur sulfate de sodium anhydre et évapore le solvant sous pression réduite. On sèche le résidu obtenu sous pression réduite pendant 2 heures et on isole le *composé I'.1* sous forme d'une résine qui est directement engagée dans l'étape suivante.
b) *composé III.1*
A la solution du *composé I'.1* obtenu précédemment dans 24 ml de tétrahydrofurane, on additionne à -68°C 6,53 ml d'une solution de diisopropylamidure de lithium à 1,5 M dans du cyclohexane redilué avec 15 ml de tétrahydrofurane. On agite le mélange réactionnel pendant 45 minutes à -68°C, puis on ajoute lentement 5 ml de méthanol. Vers 0°C, on additionne de l'eau et extrait à l'acétate d'éthyle. On lave la phase organique avec une solution aqueuse de chlorure de sodium, sèche sur sulfate de sodium, évapore le solvant sous pression réduite. On purifie le résidu obtenu par chromatographie sur une colonne de gel de silice en éluant avec un mélange cyclohexane/acétate d'éthyle 85/15 (v/v). On isole le produit attendu après cristallisation dans de l'éther isopropylique ; F = 151°C (0,2$H_2O$)

De la même manière, on prépare les *composés III.*2 *à III.8* ci-après :

## TABLEAU 7

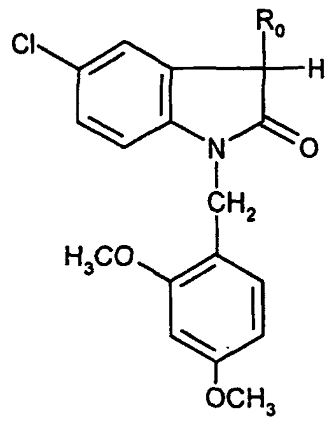

(I')

| Composé | $R_0$ | F ; °C ; (solvat) |
|---|---|---|
| III.2 | 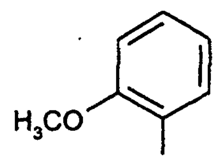 | 142 |
| III.3 | 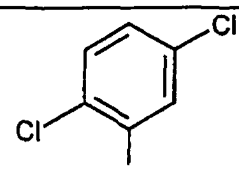 | 175<br>0,7 $H_2O$ |
| III.4 | 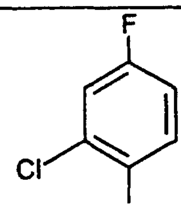 | 156 |
| III.5 | 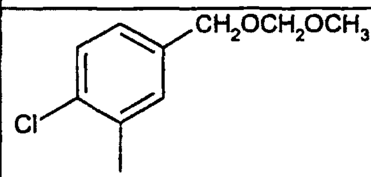 | 136 |
| III.6 | 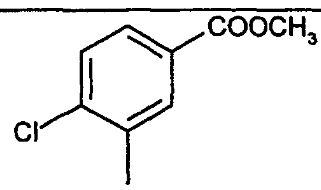 | 165 |
| III.7 | 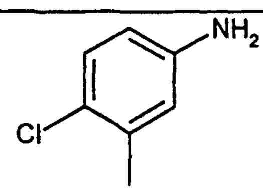 | 128 |

**TABLEAU 7 (suite 1)**

| Composé | $R_0$ | F ; °C ; (solvat) |
|---|---|---|
| III.8 | 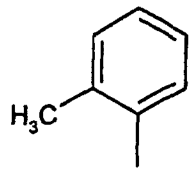 | 151 |

**EXEMPLE 37**

**5-Chloro-3-(2-chloro-4-fluorophényl)-1-(2,4-diméthoxybenzyl)-3-méthylindolin-2-one.**

(I) :

$R_0$ = 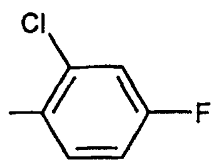 ;

$R_1$ = $-CH_3$ ; $R_2$ = H ;
$R_3$ = 4 $-OCH_3$ ; $R_4$ = 2 $-OCH_3$ ; X = 5 -Cl ; Y = H

A une solution de 1,14 g du *composé III.4* dans 20 ml de tétrahydrofurane, on additionne à -40°C 0,34 g de *tert*-butylate de potassium. On agite le mélange réactionnel à 0°C pendant 5 minutes puis on additionne à -40°C 0,32 ml d'iodure de méthyle. On agite le mélange réactionnel pendant 2 heures à température ambiante puis on additionne 10 ml d'une solution aqueuse saturée en chlorure d'ammonium et extrait à l'acétate d'éthyle. On sèche la phase organique sur sulfate de sodium anhydre puis évapore les solvants sous pression réduite. Le résidu obtenu est cristallisé dans l'éther diisopropylique ; F = 166°C.

De la même manière, en purifiant éventuellement par chromatographie sur silice, on prépare les composés des EXEMPLES 38 à 44 ci-après :

## TABLEAU 8

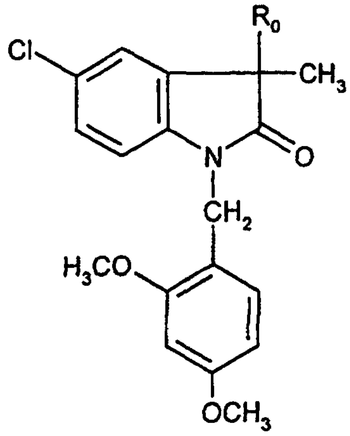

(I)

| EXEMPLE | $R_0$ | F ; °C<br>sel, solvat |
|---|---|---|
| 38 | 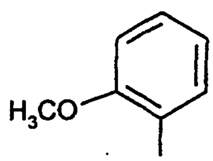 | 139<br>0,2 $H_2O$ |
| 39 | 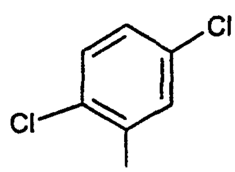 | 161<br>0,4 $H_2O$ |
| 40 | 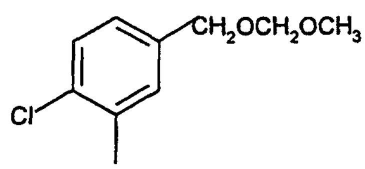 | 81 |
| 41 | 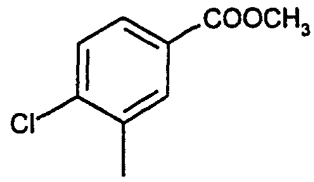 | 155 |
| 42 | 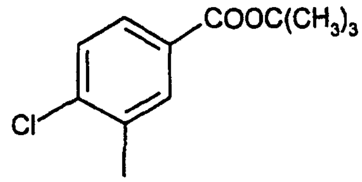 | 140 |

**TABLEAU 8 (suite 1)**

| EXEMPLE | $R_0$ | F ; °C sel, solvat |
|---|---|---|
| 43 | 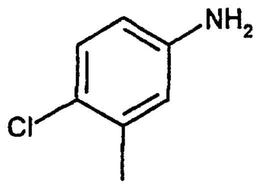 | 165 |
| 44 | 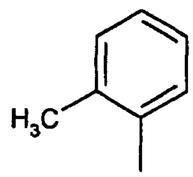 | 145<br>0,2 $H_2O$ |

Le composé racémique de l'EXEMPLE 41 est chromatographié sur une colonne chirale dans les conditions de l'EXEMPLE 1 en éluant avec un mélange 2-méthylpentane/2-propano1/90110. On obtient l'énantiomère dextrogyre : F = 120°C
$[\alpha]^{20}_D$ = +112°(c = 1, acétate d'éthyle) et son antipode.

**EXEMPLE 45**

**Ester éthylique de l'acide [5-chloro-3-(2-chlorophényl)-1-(2,4-diméthoxybenzyl)-2-oxo-indolin-3-yl]carboxylique.**

(I)

$R_0$ = 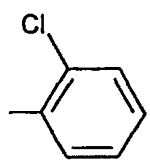 ;

$R_1$ = -$COOCH_2CH_3$ ; $R_2$ = H ;
$R_3$ = 4 -$OCH_3$ ; $R_4$ = 2 -$OCH_3$ ; X = 5 -Cl ; Y = H

A 0,26 g du *composé III.1* dans 7 ml de tétrahydrofurane refroidi à -40°C, on additionne 0,082 g de *tert*-butylate de potassium. Le mélange réactionnel est agité pendant 15 minutes à 0°C puis on additionne lentement à -65°C 0,086 ml de chloroformate d'éthyle. Après 30 minutes d'agitation à 20°C, on hydrolyse le mélange réactionnel avec 20 ml d'une solution à 5 % de chlorure d'ammonium et extrait à l'acétate d'éthyle. La phase organique est séchée sur sulfate de sodium puis on évapore les solvants sous pression réduite. On isole le produit attendu après cristallisation dans l'isopropanol ; F = 112°C (0,3$H_2O$).

**EXEMPLE 46**

**Ester phénylique de l'acide [5-chloro-3-(2-chlorophényl)-1-(2,4-diméthoxybenzyl)-2-oxo-indolin-3-yl]carboxylique.**

(I):

$R_0$ = 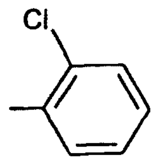 ; $R_1$ = —COO— 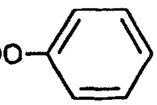 ;

$R_2$ = H ;

$R_3$ = 4 -$OCH_3$ ; $R_4$ = 2 -$OCH_3$ ; X = 5 -Cl ; Y = H

Le composé de l'EXEMPLE 46 est obtenu avec le chloroformate de phényle selon le même mode opératoire que l'EXEMPLE 45 ; F = 126°C.

**EXEMPLE 47**

**5-Chloro-3-(2-chlorophényl)-1-(2,4-diméthoxybenzyl)-3-hydroxyméthylindolin-2-one.**

(I) :

$R_0$ = 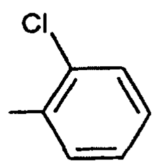 ;

$R_1$ = -$CH_2OH$ ; $R_2$ = H ;

$R_3$ = 4 -$OCH_3$ ; $R_4$ = 2 -$OCH_3$ ; X = 5 -Cl ; Y = H

A 0,3 g du *composé III.1* dans 5 ml de tétrahydrofurane, on additionne à -40°C 0,13 g de *tert*-butylate de potassium. A 0°C, on fait barbotter dans le mélange réactionnel 0,3 g de paraformaldéhyde que l'on dépolymérise lentement par chauffage. On agite le mélange réactionnel pendant 1 heure à température ambiante puis l'hydrolyse avec une solution aqueuse de $NH_4Cl$ à 5 %. On extrait à l'acétate d'éthyle et sèche la phase organique sur sulfate de sodium. On évapore les solvants sous pression réduite et purifie le résidu obtenu par chromatographie sur une colonne de gel de silice en éluant avec un mélange cyclohexane/acétate d'éthyle 90/10 (v/v). Le produit attendu est isolé après cristallisation dans un mélange n-pentane/acétate d'éthyle; F = 165°C.

**EXEMPLE 48**

**1-(4-Amino-2-méthoxybenzyl)-5-chloro-3-(2-chlorophényl)-3-méthylindolin-2-one,**

(I) :

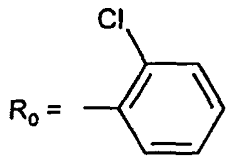

;

$R_1$ = -$CH_3$ ; $R_2$ = H ; $R_3$ = 4 -$NH_2$ ;

$R_4$ = 2 -$OCH_3$ ; X = 5 -Cl ; Y = H

A 5,19 g du composé de l'EXEMPLE 5 dans 64 ml d'éthanol, on ajoute 2,83 g d'étain en poudre puis 5,6 ml d'acide chlorhydrique concentré. On chauffe le mélange réactionnel à 50°C pendant 3 heures. A température ambiante, on filtre le mélange réactionnel sur célite, évapore partiellement le solvant sous pression réduite, reprend avec de l'acétate d'éthyle, puis traite avec une solution aqueuse de bicarbonate de sodium. On sèche la phase organique sur sulfate de sodium anhydre, puis évapore les solvants sous pression réduite. On reprend le résidu obtenu avec de l'éther diisopropylique, filtre et sèche sous pression réduite ; F = 232°C.

**EXEMPLE 49**

**5-Chloro-3-(2-chlorophényl)-1-(2-méthoxy-4-pyrrolidin-1-ylbenzyl)-3-méthylindolin-2-one.**

(I) :

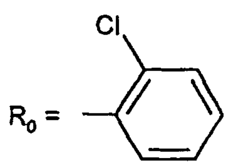

;

$R_1$ = -$CH_3$ ; $R_2$ = H

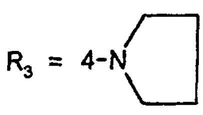

$R_4$ = 2 -$OCH_3$ ; X = 5 -Cl ; Y = H

A 0,5 g du composé de l'EXEMPLE 48 dans 50 ml d'hexaméthylphosphoramide on ajoute 0,4 g de bicarbonate de sodium en poudre et 0,14 ml de 1,4-dibromobutane. On chauffe le mélange réactionnel à 115°C pendant 10 heures. A température ambiante, on hydrolyse le mélange réactionnel et extrait à l'acétate d'éthyle. On lave plusieurs fois la phase organique à l'eau, la sèche sur sulfate de sodium anhydre, puis évapore les solvants sous pression réduite. On purifie le résidu obtenu par chromatographie sur une colonne de gel de silice en éluant avec un mélange cyclohexane/acétate d'éthyle 95/5(v/v). L'huile obtenue est traitée avec de l'acide chlorhydrique dans de l'éther diéthylique ; F = 198°C (HCl, 0,4$H_2O$)

De la même manière, on prépare les composés des EXEMPLES 50 à 52 ci-après :

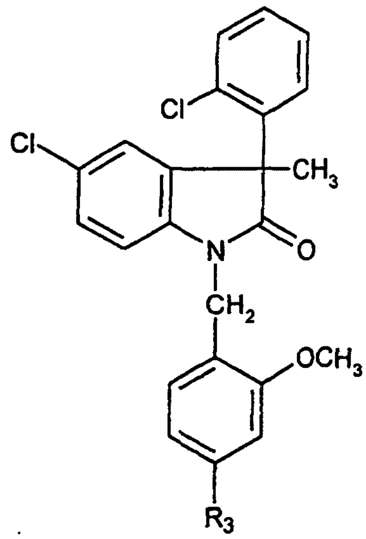

(I)

## TABLEAU 9

| EXEMPLES | $R_3$ | F ; °C sel |
|---|---|---|
| 50 | 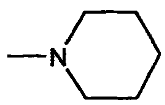 | 147 |
| 51 | 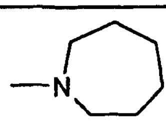 | 197 (1 HCl) |
| 52 | 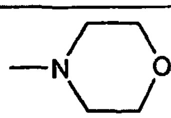 | 147 |

**EXEMPLE 53**

**5-Chloro-3-(2-chlorophényl)-1-[4-diméthylamino-2-méthoxybenzyl]-3-méthylindolin-2-one.**

(I) :

$R_0$ = 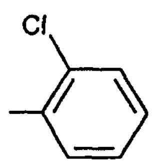 ;

$R_1$ = $-CH_3$ ; $R_2$ = H ; $R_3$ = 4 $-N(CH_3)_2$
$R_4$ = 2 $-OCH_3$ ; X = 5 -Cl ; Y = H

A 238 mg du composé de l'EXEMPLE 48 dans 4 ml de méthanol et 1 ml de diméthylformamide et 100 mg de carbonate de potassium, on ajoute 150 ml d'iodure de méthyle puis on chauffe le mélange réactionnel à 45°C pendant 24 heures. A température ambiante, on additionne 10 ml d'eau et on extrait à l'acétate d'éthyle. On lave la phase organique 2 fois à l'eau, la sèche sur sulfate de sodium anhydre et évapore les solvants sous pression réduite et purifie

le résidu par chromatographie sur une colonne de gel de silice en éluant avec un mélange cyclohexane/acétate d'éthyle 90/10 (v/v). Le résidu obtenu est cristallisé dans du n-pentane, filtré et séché sous pression réduite pendant 4 heures ; F = 135°C

**EXEMPLE 54**

**5-Chloro-3-(2-chlorophényl)-1-(2-méthoxy-4-méthylaminobenzyl)-3-méthylindolin-2-one.**

(I) :

$R_0$ = 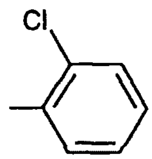 ;

$R_1$ = $-CH_3$ ; $R_2$ = H $R_3$ = 4-$NHCH_3$
$R_4$ = 2 -$OCH_3$ ; X = 5 -Cl ; Y = H

Le composé de l'EXEMPLE 54 est préparé selon le mode opératoire décrit pour l'EXEMPLE 53 ; F = 226°C ($H_2O$)

**EXEMPLE 55**

**5-Chloro-3-(2-chlorophényl)-1-(4-diisobutylamino-2-méthoxybenzyl)-3-méthyl indolin-2-one**

(I) :

$R_0$ = 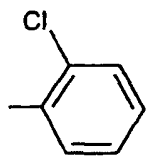 ;

$R_1$ = $-CH_3$ ; $R_2$ = H ; $R_3$ = 4 -$N[CH_2CH(CH_3)_2]_2$ ;
$R_4$ = 2 -$OCH_3$ ; X = 5 -Cl ; Y = H.

Obtenu par diamination réductrice selon J. Org. Chem. 1996, 69(11), 3849-3862. A 0,25 g du composé de l'EXEMPLE 48 dans 6 ml de 1,2 dichloroéthane, 167 µl d'acide acétique et 106 µl d'isobutyraldéhyde, on ajoute 347 mg de triacétoxyborohydrure de sodium à 20°C. Après 1 heure d'agitation à température ambiante, on hydrolyse le mélange réactionnel avec 20 ml d'eau et extrait à l'acétate d'éthyle. On sèche la phase organique sur sulfate de sodium anhydre puis évapore les solvants sous pression réduite. Le résidu est chromatographié sur une colonne de gel de silice en éluant avec un mélange cyclohexane/acétate d'éthyle 97/3 (v/v). On reprend l'huile obtenue avec une solution d'acide chlorhydrique dans de l'éther diéthylique, filtre et évapore les solvants sous pression réduite. Le solide obtenu est séché à 45 °C sous pression réduite pendant 5 heures. F = 153°C (HCl, 0,5$H_2O$)

**EXEMPLE 56**

**5-Chloro-3-(2-chlorophényl)-1-(4-isopropylamino-2-méthoxybenzyl)-3-méthyl indolin-2-one**

(I) :

$R_0$ = 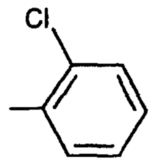 ;

$R_1$ = $-CH_3$ ; $R_2$ = H ; $R_3$ = 4 $-NH[CH(CH_3)_2]$ ;
$R_4$=2-$OCH_3$ ; X=5-Cl ; Y=H.

Obtenu par amination réductrice selon J. Org. Chem. 1996, *61*(11), 3849-3862. A 0,40 g du composé de l'EXEMPLE 48 dans 10 ml de 1,2-dichloroéthane à température ambiante, on ajoute 0,26 ml d'acide acétique, 0,14 ml d'acétone puis 0,56 g de triacétoxyborohydrure de sodium. Après 2 heures sous agitation à température ambiante, le mélange réactionnel est hydrolysé avec une solution aqueuse dhydrogénocarbonate de sodium et extrait avec de l'acétate d'éthyle. On lave la phase organique à l'eau, la sèche sur sulfate de sodium anhydre et évapore les solvants sous pression réduite. On obtient le produit attendu par filtration du résidu d'évaporation cristallisé repris dans du *n*-pentane ; F = 154°C.

Ce composé sous forme racémique est alors séparé par chromatographie sur une colonne chirale dans les mêmes conditions que dans l'EXEMPLE 1, on isole ainsi l'énantiomère dextrogyre : F = 137°C ; $[\alpha]^{20}_D$=+34,6° (c = 1, $CH_3OH$) et son antipode.

**EXEMPLE 57**

**5-Chloro-3-(2-chlorophényl)-1-[4-(isopropylméthylamino)-2-méthoxybenzyl]-3 -méthylindolin-2-one.**

(I) :

$R_0$ = 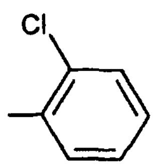 ;

$R_1$ = $-CH_3$ ; $R_2$ = H

$R_3$ = 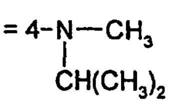

$R_4$ = 2 $-OCH_3$ ; X = 5 -Cl ; Y = H

Le composé de l'EXEMPLE 56 est traité avec du formol aqueux et du borohydrure de sodium. Le composé obtenu est salifié par une solution d'acide chlorhydrique dans de l'éther diéthylique. Le chlorhydrate est ainsi isolé après filtration et séchage à 45°C sous pression réduite ; F = 156°C (HCl ; 1,5 $H_2O$)

**EXEMPLE 58**

**Iodure de {4-[5-chloro-3-(2-chlorophényl)-3-méthyl-2-oxo-indolin-1-ylméthyl]-3 - méthoxyphényl}isopropyldiméthylammonium.**

(I) :

$R_0$ = 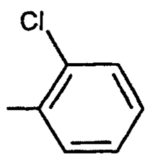 ;

$R_1$ = $-CH_3$ ; $R_2$ = H

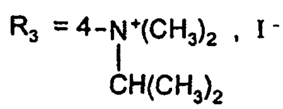

$R_4$ = 2 $-OCH_3$ ; X = 5 -Cl ; Y = H

A 0,4 g du composé de l'EXEMPLE 56 dans 10 ml de diméthylformamide on ajoute 0,56 g de carbonate de césium puis 0,27 ml d'iodure de méthyle. On chauffe le mélange réactionnel sous agitation à 40°C pendant 48 heures. A température ambiante, on traite le mélange réactionnel avec 40 ml d'eau, extrait 2 fois avec de l'éther diéthylique, puis 3 fois avec du dichlorométhane. Les phases organiques de solvant chloré sont séchées sur sulfate de sodium anhydre et évaporées sous pression réduite. Le résidu ainsi obtenu est repris avec de l'éther diéthylique, filtré et séché à 50°C sous pression réduite ; F = 146°C ($1H_2O$)

**EXEMPLE 59**

**5-Chloro-3-(2-chlorophényl)-1-(4-(cyclopropylamino)-2-méthoxybenzyl]-3-méthyl indolin-2-one**

(I) :

$R_0$ = 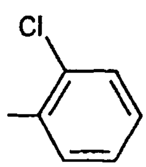 ;

$R_1$ = $-CH_3$ ; $R_2$ = H;

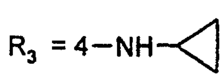

$R_4$ = 2 $-OCH_3$ ; X = 5 -Cl ; Y = H

Obtenu à partir du composé de l'EXEMPLE 48 selon T. L. 1995, 36(41) 7399-7402. A 0,4 g du composé de l'EXEMPLE 48 en solution dans 10 ml de méthanol, on ajoute 0,54 ml d'acide acétique, 0,4 g de tamis moléculaire

3Å, 0,207 ml de (1-éthoxy cyclopropyl)oxytriméthylsilane. Après 30 minutes sous agitation à température ambiante, on ajoute 0,265 g de cyanoborohydrure de sodium, puis on chauffe à reflux pendant 10 heures. Après refroidissement, on hydrolyse avec 20 ml de soude 2N, filtre sur célite et rince la célite avec de l'acétate d'éthyle. On lave la phase organique avec une solution aqueuse à 10% de chlorure de sodium, la sèche sur sulfate de sodium et évapore les solvants sous pression réduite. On purifie le résidu par chromatographie sur une colonne de gel de silice en éluant avec un mélange cyclohexane/dichlorométhane 50/50 (v/v), puis avec du dichlorométhane pur. On isole le produit attendu après cristallisation dans du *n*-pentane ; F = 185°C (0,5 $H_2O$).

**EXEMPLE 60**

**5-Chloro-3-(2-chlorophényl)-1-[4-diéthylamino-2-méthoxybenzyl]-3-méthylindolin - 2-one.**

(I) :

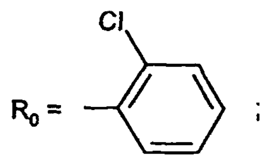

$R_1$ = $-CH_3$ ; $R_2$ = H $R_3$ = 4-$N(CH_2CH_3)_2$
$R_4$ = 2 -$OCH_3$ ; X = 5 -Cl ; Y = H

Selon Gordon W. Gribble et *al*., J. Am. Chem. Soc. 1974, 96(25), 7812.

A 0,5 g du composé de l'EXEMPLE 48 dans 7 ml d'acide acétique, on ajoute 0,45 g de borohydrure de sodium. On chauffe le mélange réactionnel à 60°C sous agitation pendant 4 heures, évapore partiellement les solvants, hydrolyse le mélange réactionnel avec une solution aqueuse de bicarbonate de sodium et extrait à l'acétate d'éthyle. La phase organique est séchée sur sulfate de sodium anhydre et concentrée sous pression réduite. L'huile obtenue est traitée avec une solution d'acide chlorhydrique dans l'éther diéthylique ; F = 198°C (HCl)

**EXEMPLE 61**

**5-Chloro-3-(2-chlorophényl)-1-[4-éthylamino-2-méthoxybenzyl]-3-méthylindolin-2 - one.**

(I) :

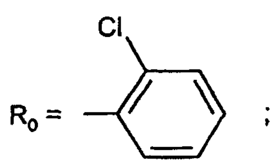

$R_1$ = $-CH_3$ ; $R_2$ = H $R_3$ = 4-$NH(CH_2CH_3)$
$R_4$ = 2 -$OCH_3$ ; X = 5 -Cl ; Y = H

Le composé de l'EXEMPLE 61 est préparé selon le même mode opératoire que pour l'EXEMPLE 60 ; F = 167°C

**EXEMPLE 62**

***N*-{4-[5-Chloro-3-(2-chlorophényl)-3-méthyl-2-oxoindolin-1-yl]-3-méthoxyphényl} acétamide.**

(I) :

$R_0$ = 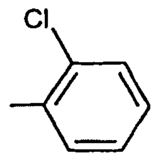 ;

$R_1$ = $-CH_3$ ; $R_2$ = H ; $R_3$ = 4 $-NH(COCH_3)$ ;
$R_4$ = 2 $-OCH_3$ ; X = 5 -Cl ; Y = H.

A 0,5 g du composé de l'EXEMPLE 48 dans 10 ml de dichlorométhane et 0,5 ml de triéthylamine, on ajoute lentement à 0°C, 0,10 ml de chlorure d'acétyle. On hydrolyse le mélange réactionnel à température ambiante, ajoute 20 ml de dichlorométhane sèche la phase organique sur $Na_2SO_4$ et évapore les solvants sous pression réduite. Le résidu obtenu est purifié par chromatographie sur une colonne de gel de silice en éluant avec un mélange cyclohexane/acétate d'éthyle, 50/50 (v/v) ; F = 83°C.

**EXEMPLE 63**

**5-Chloro-3-(2-chlorophényl)-1-(4-éthoxy-2-méthoxybenzyl)-3-méthylindolin-2-one.**

(I) :

$R_0$ = 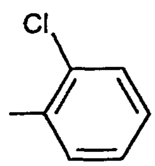 ;

$R_1$ = $-CH_3$ ; $R_2$ = H $R_3$ = 4-$OCH_2CH_3$
$R_4$ = 2 $-OCH_3$ ; X = 5 -Cl ; Y = H

a) 5-Chloro-3-(2-chlorophényl)-1-(4-hydroxy-2-méthoxybenzyl)-3-méthylindolin-2-one. A 1,14 g du composé de l'EXEMPLE 3 dans 20 ml de dichlorométhane et 1 ml de méthylphénylsulfure, on ajoute, à 0°C, 3 ml d'acide trifluoroacétique. Après 2 heures d'agitation à température ambiante, on hydrolyse le mélange réactionnel et extrait à l'acétate d'éthyle. On lave la phase organique avec une solution aqueuse de bicarbonate de sodium, la sèche sur sulfate de sodium anhydre puis évapore les solvants sous pression réduite. Le résidu obtenu est purifié par chromatographie sur une colonne de gel de silice en éluant avec un mélange cyclohexane/acétate d'éthyle 80/20 (c/v) ; F = 200°C.

b) A 0,2 g du composé obtenu en a) dans 5 ml de diméthylformamide, on ajoute 0,23 g de carbonate de césium, puis à 0°C 0,112 ml d'iodoéthane. Après 1 heure d'agitation à 28°C, on hydrolyse le mélange réactionnel et extrait à l'acétate d'éthyle. On sèche la phase organique sur sulfate de sodium anhydre et évapore les solvants sous pression réduite. On reprend l'huile obtenue avec du n-pentane, filtre et sèche le précipité obtenu à 50°C sous pression réduite pendant 5 heures ; F = 124°C.

**EXEMPLE 64**

**5-Chloro-3-(2-chlorophényl)-1-(2,4-diméthoxybenzyl)-3-méthoxyméthylindolin-2-one.**

(I) :

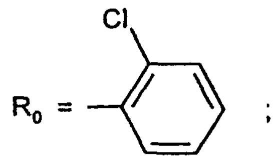

$R_1$ = $-CH_2OCH_3$ ; $R_2$ = H ; $R_3$ = 4 $-OCH_3$
$R_4$ = 2 $-OCH_3$ ; X = 5 -Cl ; Y = H.

A une solution de 70 mg du composé obtenu à l'EXEMPLE 47, dans 1 ml de dichlorométhane, on additionne à -20°C, 0,1 ml de trifluorométhanesulfonate de méthyle puis 0,07 ml de 2,6-di-(*tert*-butyl)pyridine, on maintient le mélange réactionnel pendant une semaine à + 5°C. On évapore le solvant sous pression réduite, ajoute 5 ml d'acide chlorhydrique 0,1N et extrait à l'acétate d'éthyle. On sèche le phase organique sur sulfate de sodium anhydre et évapore le solvant sous pression réduite. On purifie le résidu obtenu par chromatographie sur une colonne de gel de silice en éluant avec un mélange cyclohexane/acétate d'éthyle 95/5 (v/v). La résine obtenue est reprise par du *n*-pentane.

On filtre et sèche le solide obtenu 5 heures à 50°C ; F = 125°C (0,4$H_2O$)

**EXEMPLE 65**

**5-Chloro-3-(2-chloro-5-hydroxyméthylphényl)-1-(2,4-diméthoxybenzyl)-3-hydroxyindolin-2-one**

(I) :

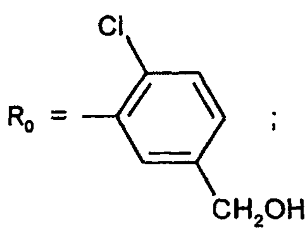

$R_1$ = -OH ; $R_2$ = H ; $R_3$ = 4-$OCH_3$ ;
$R_4$ = 2 $-OCH_3$ ; X = 5 -Cl ; Y=H

On chauffe à 50°C pendant 2 heures un mélange de 0,307 g du composé de l'EXEMPLE 19, 15 ml de méthanol et 1 ml d'acide chlorhydrique 10N. On évapore le solvant sous pression réduite et reprend le résidu obtenu avec du dichlorométhane et de l'eau. On lave la phase organique deux fois à l'eau puis la sèche sur sulfate de sodium anhydre. On évapore les solvants sous pression réduite. On isole le produit attendu après concrétion dans du cyclohexane, filtration et séchage à 30°C sous pression réduite pendant 6 heures ; F = 107°C.

**EXEMPLE 66**

**5-Chloro-3-[2-chloro-5-(diméthylamino)phényl]-1-(2,4-diméthoxybenzyl)-3-méthylindolin-2-one**

(I) :

(I) : $R_0$ = 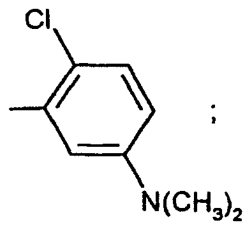 ;

; $R_1$ = $-CH_3$ ; $R_2$ = H ; $R_3$ = 4 $-OCH_3$ ; $R_4$ = 2-$OCH_3$
X = 5 -Cl ; Y= H

Préparé à partir du composé de l'EXEMPLE 43 et selon le mode opératoire décrit pour l'EXEMPLE 53. On isole le produit attendu après cristallisation dans de l'éther isopropylique ; F = 149°C (0,7 $H_2O$).

**EXEMPLE 67**

**4-Chloro-3-[5-chloro-1-(2,4-diméthoxybenzyl)-3-méthyl-2-oxoindolin-3-yl]phényl formamide**

Selon T.L. 1982, 23(33), 3315.

(I) :

$R_0$ = 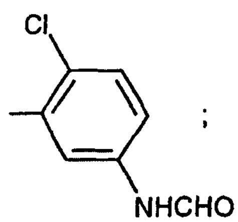 ;

$R_1$ = $-CH_3$ ; $R_2$ = H ; $R_3$ = 4 $-OCH_3$ ; $R_4$ = 2-$OCH_3$
X = 5-Cl; Y = H

A 0,44 ml d'anhydride acétique refroidi à 0°C, on ajoute 0,216 ml d'acide formique puis chauffe le mélange réactionnel pendant 1 heure 30 minutes à 58°C. Après refroidissement à 10°C, on ajoute 0,8 ml de tétrahydrofurane puis 0,80 g du composé de l'EXEMPLE 43 en solution dans 4 ml de tétrahydrofurane. Après 2 heures sous agitation à 20°C, on évapore les solvants sous pression réduite. On reprend le résidu obtenu par du n-pentane et filtre le produit ; F = 190°C.

**EXEMPLE 68**

**5-Chloro-3-[2-chloro-5-(méthylamino)phényl]-1-(2,4-diméthoxybenzyl)-3-méthylindolin-2-one**

(I) :

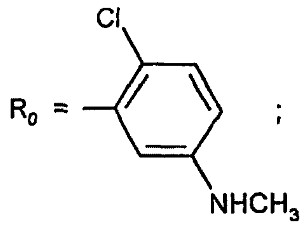

$R_1$ = $-CH_3$ ; $R_2$ = H ; $R_3$ = 4 $-OCH_3$ ; $R_4$ = 2-$OCH_3$
X = 5-Cl ; Y = H

Préparé selon T. L. 1982, 23(33), 3315.

A une solution de 0,4 g du composé de l'EXEMPLE 67 dans 1 ml de tétrahydrofurane refroidie à 0°C, on ajoute 0,56 ml d'une solution de borane diméthylsulfure 2M dans le tétrahydrofurane. Après 1 heure à 58°C, puis refroidissement à 0°C, on ajoute au mélange réactionnel 0,2 ml d'acide chlrorhydrique 10N et 1 ml de méthanol. On chauffe à 60°C pendant 1 heure, refroidit et évapore les solvants sous pression réduite. On traite le résidu solide avec 1 ml d'une solution saturée en carbonate de potassium et extrait plusieurs fois à l'acétate d'éthyle. On sèche les phases organiques sur sulfate de sodium anhydre et évapore les solvants sous pression réduite. On purifie par chromatographie sur une colonne de gel de silice en éluant avec un mélange cyclohexane/acétate d'éthyle 85/15 (v/v). On isole le produit attendu sous forme de chlorhydrate en traitant le résidu obtenu par de l'éther diéthylique contenant du chlorure d'hydrogène puis on filtre ; F = 121 °C (0,5 $H_2O$, 1HCl).

**EXEMPLE 69**

**5-Chloro-3-(2-chloro-5-[éthyl(méthyl)amino]phényl}-1-(2,4-diméthoxybenzyl)-3-méthylindolin-2-one**

(I) :

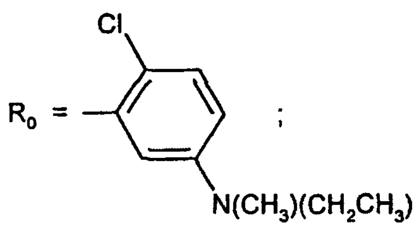

$R_1$ = $-CH_3$ ; $R_2$ = H ; $R_3$ = 4 $-OCH_3$ ; $R_4$ = 2-$OCH_3$
X = 5-Cl ; Y = H

Obtenu à partir du composé de l'EXEMPLE 68 et selon le même mode opératoire que pour l'EXEMPLE 60 ; F = 145°C.

**EXEMPLE 70**

***N*-{4-Chloro-3-[5-chloro-1-(2,4-diméthoxybenzyl)-3-méthyl-2-oxoindolin-3-yl]phényl} acétamide**

(I) :

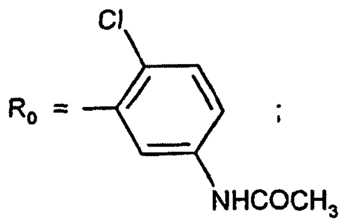

$R_1$ = -$CH_3$ ; $R_2$ = H ; $R_3$ = 4 -$OCH_3$ ; $R_4$ = 2-$OCH_3$
X = 5-Cl ; Y = H

Obtenu selon le même mode opératoire que le composé de l'EXEMPLE 62 à partir du composé de l'EXEMPLE 43 . F = 117°C.

De la même manière, on obtient les EXEMPLES 71 à 80 suivants :

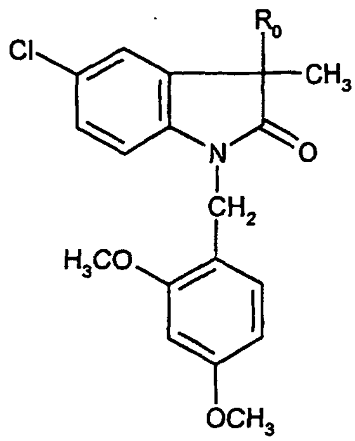

(I)

## TABLEAU 10

| EXEMPLES | $R_0$ | F ; °C |
| --- | --- | --- |
| 71 | 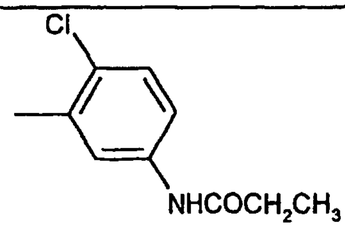 | 238<br>0,5 $H_2O$ |
| 72 | 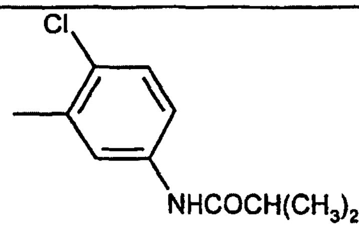 | 258<br>0,5 $H_2O$ |
| 73 | 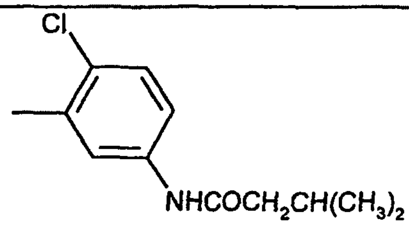 | 203<br>0,3 $H_2O$ |
| 74 | 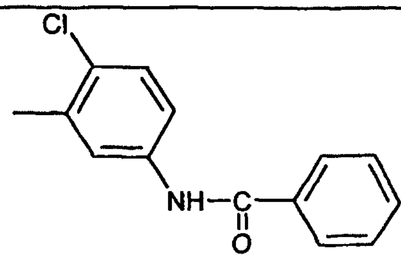 | 255<br>0,5 $H_2O$ |
| 75 | 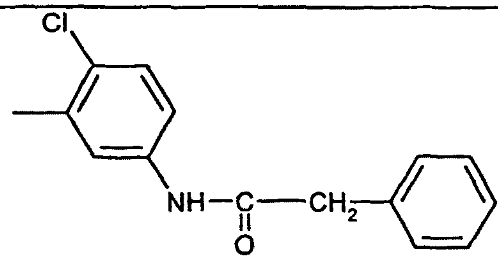 | 229<br>0,5 $H_2O$ |

**TABLEAU 10 (suite 1)**

| EXEMPLES | $R_0$ | F ; °C |
|---|---|---|
| 76 | 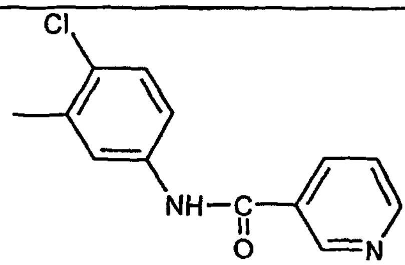 | 127<br>1 $H_2O$ |
| 77 | 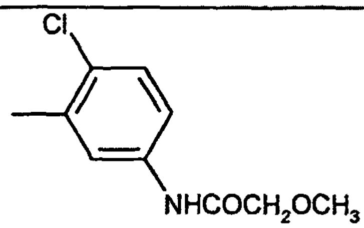 | 191 |
| 78 | 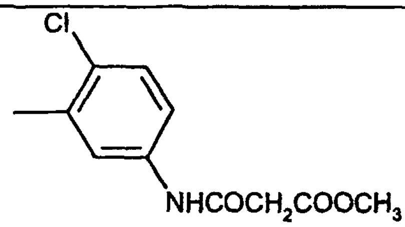 | 153<br>0,5 $H_2O$ |
| 79 | 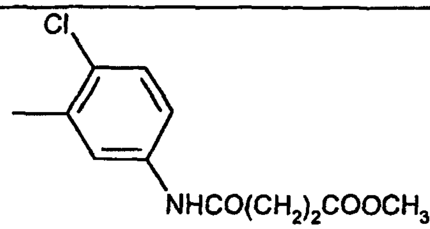 | 203<br>0,5 $H_2O$ |
| 80 | 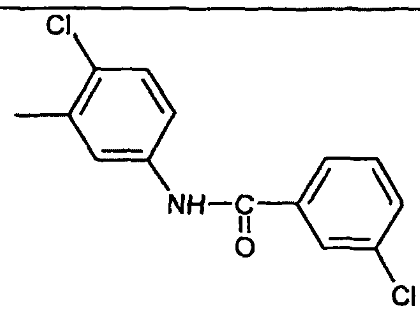 | 255<br>0,5 $H_2O$ |

**EXEMPLE 81**

***N*-{4-Chloro-3-[5-chloro-1-(2,4-diméthoxybenzyl)-3-méthyl-2-oxoindolin-3-yl]phényl}-3-méthoxypropanamide**

(I) :

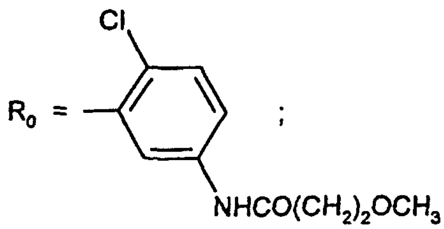

$R_1$ = $-CH_3$ ; $R_2$ = H ; $R_3$ = 4 $-OCH_3$ ; $R_4$ = 2-$OCH_3$
X = 5-Cl ; Y = H

A 0,3 g du composé de L'EXEMPLE 43 dans 10 ml de diméthylformamide, on ajoute 0,068 ml d'acide 3-méthoxypropionique, 320 mg d'hexafluorophosphate de benzotriazolyl-*N*-oxytrisdiméthylaminophosphonium. Après refroidissement à 0°C, on additionne 0,23 ml de triéthylamine. On agite le mélange réactionnel à température ambiante pendant 16 heures. On ajoute 40 ml d'eau et extrait avec 30 ml d'acétate d'éthyle. On traite la phase organique avec 20 ml d'une solution aqueuse de bicarbonate de sodium, sépare cette phase par décantation, la sèche sur sulfate de sodium anhydre et évapore les solvants sous pression réduite. On purifie le résidu par chromatographie sur une colonne de gel de silice en éluant avec du dichlorométhane. On obtient le produit attendu après cristallisation dans du *n*-pentane. ; F = 168°C (0,3 $H_2O$).

De la même manière on obtient les composés des EXEMPLES 82 à 86 suivants :

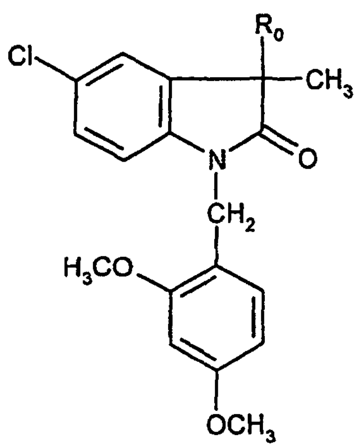

(I)

**TABLEAU 11**

| EXEMPLES | $R_0$ | F ; °C |
|---|---|---|
| 82 | 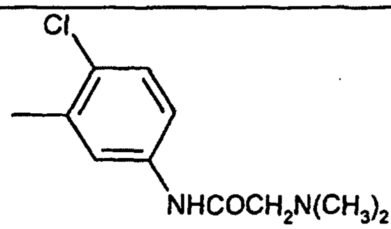 | F = 145<br>0,3 $H_2O$ |
| 83 | 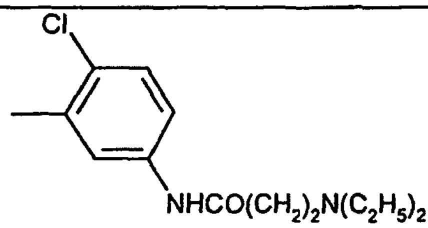 | 92 |
| 84 | 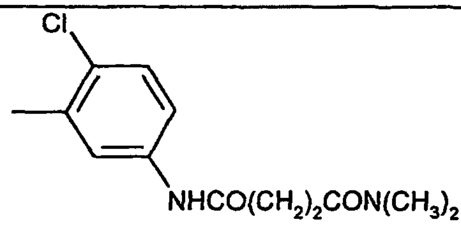 | 107<br>0,5 $H_2O$ |
| 85 | 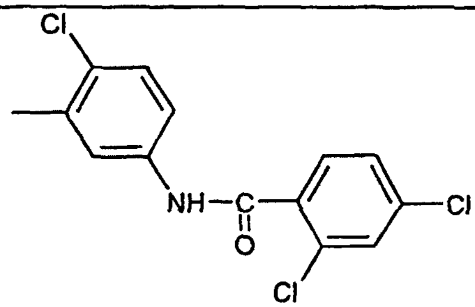 | 126<br>0,3 $H_2O$ |

**TABLEAU 11 (suite)**

| EXEMPLES | $R_0$ | F ; °C |
|---|---|---|
| 86 | 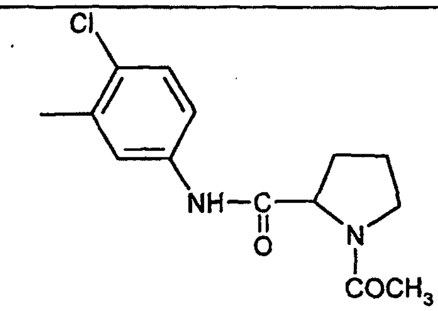 | 143<br>1 $H_2O$ |

**EXEMPLE 87**

***N*-{4-Chloro-3-[5-chloro-1-(2,4-diméthoxybenzyl)-3-méthyl-2-oxoindolin-3-yl] phényl}-*N*-méthylacétamide**

(I)

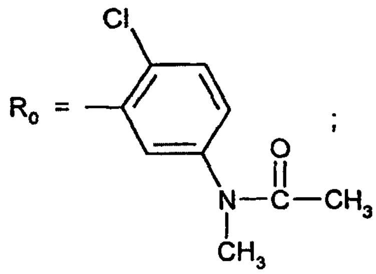

;

$R_1$ = -$CH_3$ ; $R_2$ = H ; $R_3$ = 4 -$OCH_3$ ; $R_4$ = 2-$OCH_3$
X = 5-Cl ; Y = H

Obtenu à partir du composé de l'EXEMPLE 68 selon le même mode opératoire que dans l'EXEMPLE 62 ; F = 84°C.

De la même manière, on obtient les composés des EXEMPLES 88 à 90 suivants :

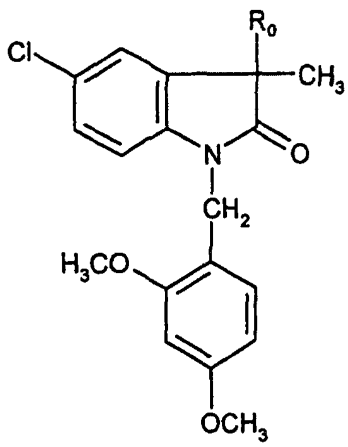

(*I*)

TABLEAU 12

| EXEMPLES | $R_0$ | F ; °C |
|---|---|---|
| 88 | 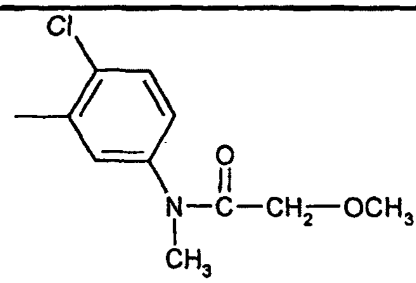 | 129 |
| 89 | 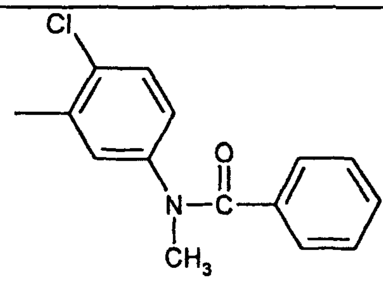 | 72 |
| 90 | 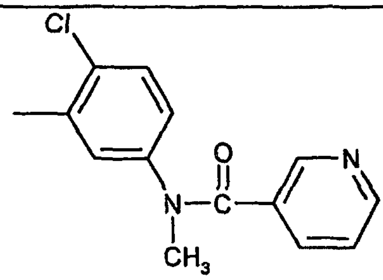 | 72 |

**EXEMPLE 91**

***N*-{4-Chloro-3-[5-chloro-1-(2,4-diméthoxybenzyl)-3-méthyl-2-oxoindolin-3-yl]phényl} -2-(diméthylamino)-*N*-méthylacétamide**

(I) :

$R_0$ = 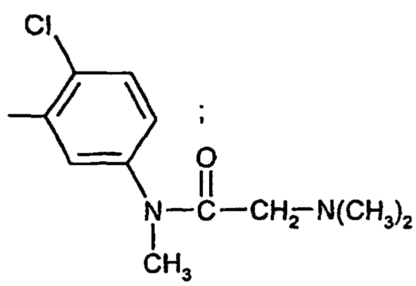 ;

$R_1 = -CH_3$ ; $R_2 = H$ ; $R_3 = 4\text{-}OCH_3$ ; $R_4 = 2\text{-}OCH_3$
X = 5-Cl ; Y = H

Selon le mode opératoire de Synthesis 1980, 547

A 0,32 g de l'EXEMPLE 68 dans 5 ml de dichlorométhane, on ajoute à 0°C 0,14 g de *N,N*-diméthylglycine, 0,40 ml de triéthylamine et 0,34 g de chlorure de *N*,*N*-bis[2-oxo-3-oxozalidinyl]phosphorodiamide. On agite le mélange

réactionnel pendant 24 heures à température ambiante, ajoute 20 ml d'une solution aqueuse de bicarbonate de sodium, extrait avec 30 ml d'acétate d'éthyle. La phase organique est à nouveau lavée par 20 ml d'une solution aqueuse de bicarbonate de sodium, puis séchée sur sulfate de sodium anhydre. Les solvants sont évaporés sous pression réduite. On purifie par chromatographie sur une colonne de gel de silice en éluant avec un mélange dichlorométhane/méthanol 97/3 (v/v). On isole le produit attendu par cristallisation dans du *n*-pentane ; F = 104°C.

**EXEMPLE 92**

**1-Acétyl-*N*-{4-Chloro-3-[5-chloro-1-(2,4-diméthoxybenzyl)-3-méthyl-2-oxoindolin-3-yl]phényl}-*N*-méthyl-2-pyrrolidinecarboxamide,**

(I) :

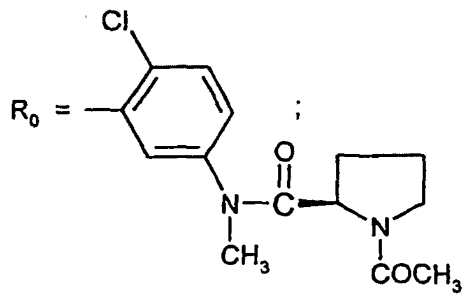

$R_1$ = -$CH_3$ ; $R_2$ = H ; $R_3$ = 4 -$OCH_3$ ; $R_4$ = 2-$OCH_3$
X = 5-Cl ; Y = H

Préparé selon le même mode opératoire qu'à l'EXEMPLE 91 . F = 74°C (2 $H_2O$)

**EXEMPLE 93**

***N*-{4-Chloro-3-[5-chloro-1-(2,4-diméthoxybenzyl)-3-méthyl-2-oxoindolin-3-yl]phényl}urée**

(I) :

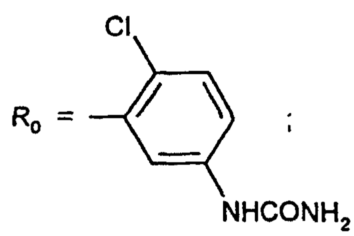

$R_1$ = -$CH_3$ ; $R_2$ = H ; $R_3$ = 4 -$OCH_3$ ; $R_4$ = 2-$OCH_3$
X = 5-Cl ; Y = H

a) formation du 4-chloro-3-[5-chloro-1-(2,4-diméthoxybenzyl)-3-méthyl-2-oxoindolin-3-yl] phénylcarbamate de phényle
A 0,4 g du composé de l'EXEMPLE 43 dans 20 ml de tétrahydrofurane, on ajoute 0,30 ml de soude à 30%. Après refroidissement à -5°C, on additionne au mélange réactionnel 0,33 ml de chlorocarbonate de phényle. Après 4 heures d'agitation à température ambiante, on ajoute 30 ml d'eau et extrait par 50 ml d'acétate d'éthyle. On sèche la phase organique sur sulfate de sodium anhydre, évapore les solvants sous pression réduite. L'huile obtenue est utilisée dans l'étape suivante.
b) obtention du composé de l'EXEMPLE 93

Le composé obtenu en a) est repris par 30 ml de dichlorométhane en présence de 1 ml d'ammoniac liquide. Après 48 heures d'agitation à température ambiante, on évapore partiellement le solvant et reprend le résidu obtenu à l'éther diéthylique. On filtre et lave à l'éther diéthylique; F = 254°C (1,5 $H_2O$).

**EXEMPLE 94**

***N*-{4-Chloro-3-[5-chloro-1-(2,4-diméthoxybenzyl)-3-méthyl-2-oxoindolin-3-yl]phényl}-*N*,*N*-diméthylurée**

(I) :

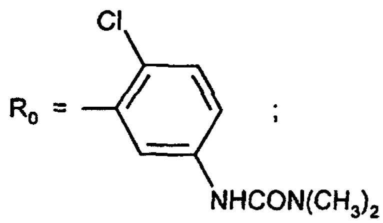

$R_1$ = -$CH_3$ ; $R_2$ = H ; $R_3$ = 4 -$OCH_3$ ; $R_4$ = 2-$OCH_3$
X = 5-Cl ; Y = H

Obtenu selon le même mode opératoire que pour l'EXEMPLE 93 ; F = 182°C (0,4 $H_2O$)

**EXEMPLE 95**

***N*-{4-Chloro-3-[5-chloro-1-(2,4-diméthoxybenzyl)-3-méthyl-2-oxoindolin-3-yl]phényl} méthanesulfonamide**

(I) :

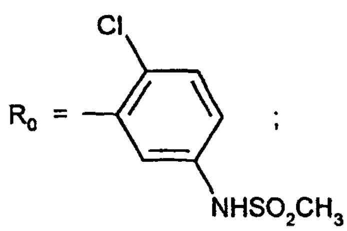

$R_1$ = -$CH_3$ ; $R_2$ = H ; $R_3$ = 4 -$OCH_3$ ; $R_4$ = 2-$OCH_3$
X = 5-Cl ; Y = H

A 0,3 g du composé de l'EXEMPLE 43 dans 10 ml de dichlorométhane, on ajoute 0,23 ml de triéthylamine puis, après refroidissement à -10°C, on additionne 56 µl de chlorure de méthanesulfonyle. Après 24 heures d'agitation à température ambiante, on ajoute 10 ml d'une solution aqueuse d'hydrogénocarbonate de sodium et 30 ml d'acétate d'éthyle. On isole la phase organique, la sèche sur sulfate de sodium anhydre et évapore les solvants sous pression réduite. On purifie le résidu obtenu par chromatographie sur colonne de gel de silice en éluant avec du dichlorométhane. On obtient le produit attendu après cristallisation dans du *n*-pentane ; F = 210°C (0,25 $H_2O$).

**EXEMPLE 96**

***N*-{4-Chloro-3-[5-chloro-1-(2,4-diméthoxybenzyl)-3-méthyl-2-oxoindolin-3-yl]phényl}-*N*-méthylsulfonyl)méthanesulfonamide**

(I) :

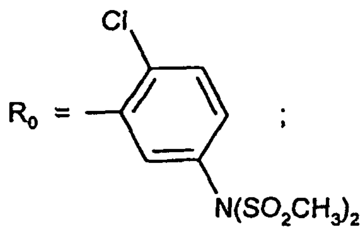

$R_1$ = $-CH_3$ ; $R_2$ = H ; $R_3$ = 4 $-OCH_3$ ; $R_4$ = 2-$OCH_3$
X = 5-Cl : Y = H

Obtenu selon le procédé de l'EXEMPLE 95 en utilisant deux équivalents de chlorure de méthanesulfonyle ; F = 159°C

**EXEMPLE 97**

***N*-{4-Chloro-3-[5-chloro-1-(2,4-diméthoxybenzyl)-3-méthyl-2-oxoindolin-3-yl]phényl}-*N*-méthylméthanesulfonamide**

(I) :

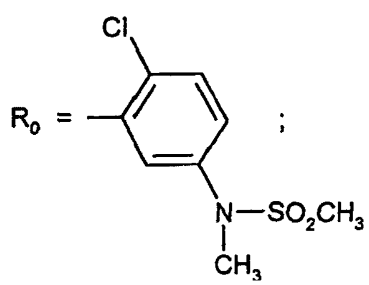

$R_1$ = $-CH_3$ ; $R_2$ = H ; $R_3$ = 4 $-OCH_3$ ; $R_4$ = 2-$OCH_3$
X = 5-Cl ; Y = H

Obtenu selon le mode opératoire de l'EXEMPLE 95 à partir de l'EXEMPLE 68 ; F = 76°C (0,8 $H_2O$).

**EXEMPLE 98**

***N*-{4-Chloro-3-[5-chloro-1-(2,4-diméthoxybenzyl)-3-méthyl-2-oxoindolin-3-yl]phényl}-*N*-méthylphénylsulfonamide**

(I) :

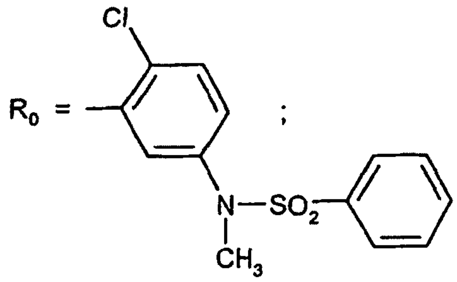

$R_1$ = $-CH_3$ ; $R_2$ = H ; $R_3$ = 4 $-OCH_3$ ; $R_4$ = 2-$OCH_3$
X = 5-Cl ; Y = H

Obtenu selon le même mode opératoire que pour l'EXEMPLE 97 ; F = 73°C (0,8 $H_2O$).

**EXEMPLE 99**

**5-Chloro-3-[2-chloro-5-(4-morpholinyl)phényl]-1-(2,4-diméthoxybenryl)-3-méthyl indolin-2-one**

(I) :

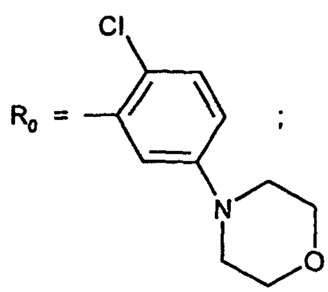

$R_1$ = $-CH_3$ ; $R_2$ = H ; $R_3$ = 4 $-OCH_3$ ; $R_4$ = 2-$OCH_3$
X = 5-Cl ; Y = H

Obtenu selon le même mode opératoire que pour l'EXEMPLE 52 à partir du composé de L'EXEMPLE 43 ; F = 168°C.

**EXEMPLE 100**

**3-Chloro-3-[2-chloro-5-(4-méthyl-1-pipérazinyl)phényl]1-(2,4-diméthoxybenzyl)-3-méthylindolin-2-one**

(I) :

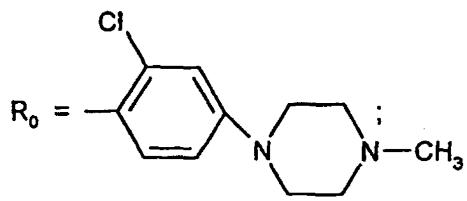

$R_1$ = -$CH_3$ ; $R_2$ = H ; $R_3$ = 4 -$OCH_3$ ; $R_4$ = 2-$OCH_3$
X = 5-Cl ; Y = H

Obtenu selon le même mode opératoire que pour l'EXEMPLE 1 à partir du composé II.9 ; F = 140°C (2HCl, 0,3$H_2O$)

**EXEMPLE 101**

**Acide 4-chloro-3-[5-chloro-1-(2,4-diméthoxybenzyl)-3-méthyl-2-oxoindolin-3-yl] benzoique**

(I) :

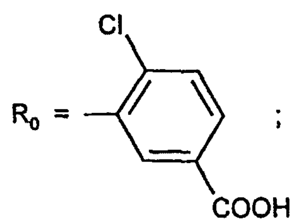

$R_1$ = -$CH_3$ ; $R_2$ = H ; $R_3$ = 4 -$OCH_3$ ; $R_4$ = 2-$OCH_3$
X = 5-Cl; Y = H

A 3,46 g du composé racémique de l'EXEMPLE 41 dans 300ml d'une solution contenant méthanol/dioxane (v/v), on ajoute 11 ml d'une solution aqueuse de soude 2N et laisse le mélange réactionnel sous agitation pendant 5 heures à 65°C. Après refroidissement à température ambiante, on évapore partiellement les solvants sous pression réduite et extrait avec de l'acétate d'éthyle. On acidifie la phase aqueuse à 10°C avec une solution d'acide chlorhydrique 1 N et extrait l'acide au dichlorométhane. On sèche cette dernière phase organique sur sulfate de sodium anhydre et on évapore les solvants sous pression réduite. On obtient le produit attendu par cristallisation de l'huile dans de l'éther isopropylique ; F = 186°C.

Par chromatographie chirale, dans les conditions de l'EXEMPLE 1, en éluant avec un mélange 2-méthyl-pentane/2-propanol 90/10 et 0,1 % d'acide trifluoroacétique, on isole l'énantiomère dextrogyre $[\alpha]^{20}_D$ = +101,8 (C=1, $CH_3OH$) F = 114°C et son antipode.

**EXEMPLE 102**

**4-Chloro-3-[5-chloro-1-(2,4-diméthoxybenzyl)-3-méthyl-2-oxoindolin-3-yl]-*N*,*N*-diéthylbenzamide**

(I) :

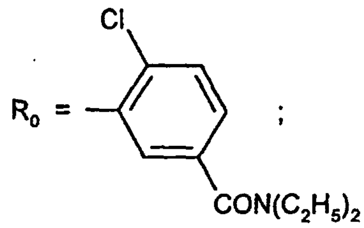

$R_1$ = $-CH_3$ ; $R_2$ = H ; $R_3$ = 4 $-OCH_3$ ; $R_4$ = 2-$OCH_3$
X = 5-Cl ; Y = H

A 0,48 g du composé de l'EXEMPLE 101 dans 10 ml de diméthylformamide, on ajoute à 0°C, 0,46 g d'hexafluorophosphate de benzotriazolyl-*N*-oxytrisdiméthylaminophospho nium, 0,20 ml de diéthylamine et 0,28 ml de triéthylamine. Après 15 heures d'agitation à 20°C, on hydrolyse le mélange réactionnel avec 70 ml d'acide chlorhydrique 0,1 N, et extrait avec de l'acétate d'étyle. On traite la phase organique avec 70 ml d'une solution aqueuse d'hydrogénocarbonate de sodium et sèche sur sulfate de sodium anhydre. On évapore les solvants sous pression réduite. On obtient le produit attendu par cristallisation dans du *n*-pentane ; F = 88°C.

Le composé de l'EXEMPLE 102 sous forme racémique est purifié par chromatographie sur une colonne ChiralPack® AD de Daicel en éluant avec un mélange 2-méthylpentane/propanol-2 90/10 (v/v). On isole ainsi l'énantiomère dextrogyre : F = 86°C ; $[\alpha]^{20}_D$ = + 100,3° (c = 1 , $CH_3CO_2C_2H_5$), et son antipode

De la même manière que pour le mélange racémique on obtient les amides du TABLEAU 13 :

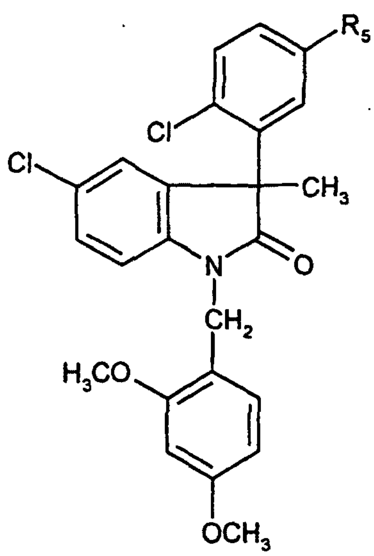

(I)

**TABLEAU 13**

| EXEMPLES | $R_5$ | F ; °C |
|---|---|---|
| 103 | 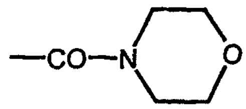 | 180<br>3 $H_2O$ |
| 104 | $-CONHCH_2COOCH_3$ | 98<br>5 $H_2O$ |
| 105 | $-CONH(CH_2)_3OCH_3$ | 125<br>5 $H_2O$ |
| 106 | 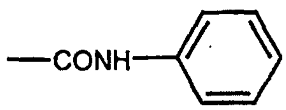 | 99<br>4 $H_2O$ |
| 107 | $-CONH(CH_2)_2N(CH_3)_2$ | 111<br>1,5 $H_2O$ |
| 108 | $-CONHCH_2C(CH_3)_2$ | 199<br>2 $H_2O$ |
| 109 | $-CON(CH_3)_2$ | 184<br>1 $H_2O$ |
| 110 | $-CONHCH_3$ | 181<br>3 $H_2O$ |
| 111 | $-CONH_2$ | 133<br>1 $H_2O$ |

## TABLEAU 13 (suite 1)

| EXEMPLES | $R_5$ | F ; °C |
|---|---|---|
| 112 | 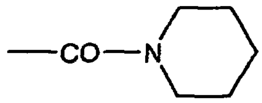 | 154<br>0,5 $H_2O$ |
| 113 | 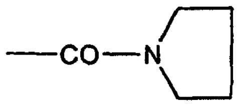 | 100<br>0,8 $H_2O$ |
| 114 | $-CONHCH_2CH_3$ | 248<br>0,5 $H_2O$ |
| 115 | 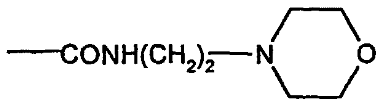 | 128<br>0,5 $H_2O$ |
| 116 | 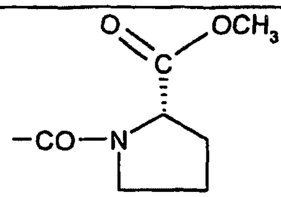 | 96<br>0,2 $H_2O$ |
| 117 | 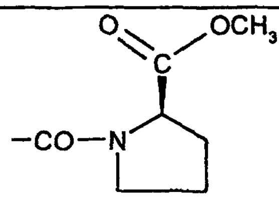 | 72<br>0,5 $H_2O$ |
| 118 | 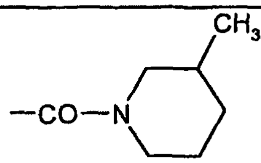 | 98 |
| 119 | 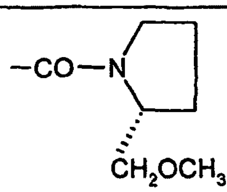 | 98 |
| 120 | 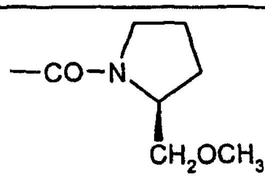 | 87 |
| 121 | 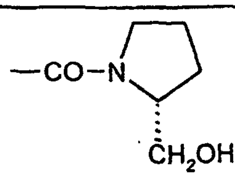 | 122 |

## TABLEAU 13 (suite 2)

| EXEMPLES | $R_5$ | F ; °C |
|---|---|---|
| 122 | 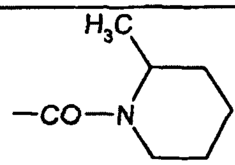 | 124<br>0,3 $H_2O$ |
| 123 | 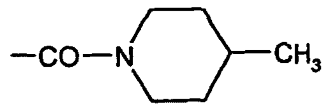 | 115 |
| 124 | 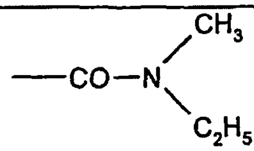 | 166 |
| 125 | 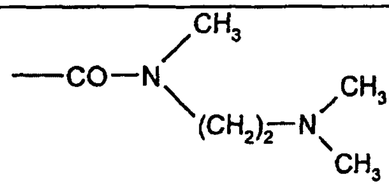 | 102<br>1 $H_2O$ |
| 126 | 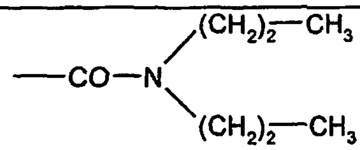 | cire |
| 127 | 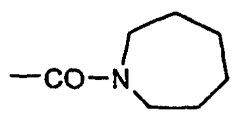 | 123 |
| 128 | 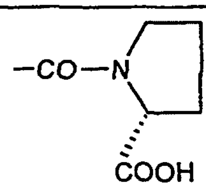 | 175<br>0,4 $H_2O$ |
| 129 | 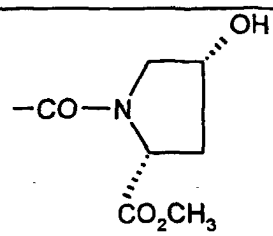 | 110 |
| 130 | 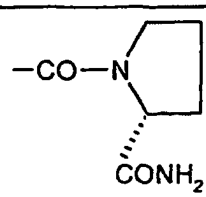 | 138<br>0,2 $H_2O$ |

**TABLEAU 13 (suite 3)**

| EXEMPLES | $R_5$ | F ; °C |
|---|---|---|
| 131 | 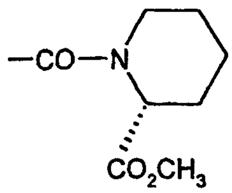 | 116 |
| 132 | 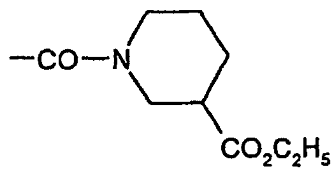 | 114 |
| 133 | 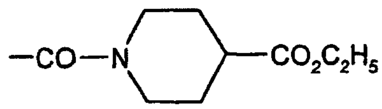 | 118 |
| 134 | 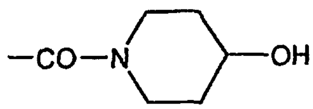 | 142<br>0,3 $H_2O$ |
| 135 | 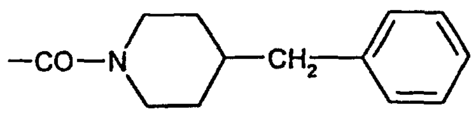 | 114<br>0,6 $H_2O$ |
| 136 | 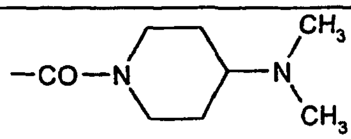 | 130<br>0,5 $H_2CO_3$ |
| 137 | 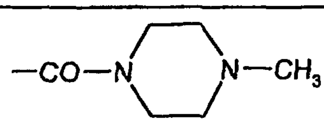 | 121<br>0,4 $H_2O$ |
| 138 | 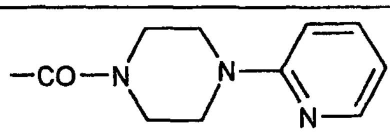 | 127<br>0,5 $H_2O$ |
| 139 | 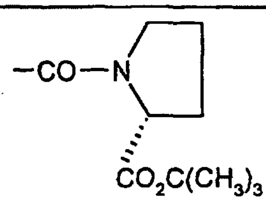 | 110 |

Le composé racémique de l'EXEMPLE 119 est chromatographié sur une colonne chirale dans les conditions de l'EXEMPLE 102. On obtient l'énantiomère dextrogyre et son antipode ; F = 86°C ; $[\alpha]^{20}_D$ = + 129° (c = 1, acétate d'éthyle).

- idem à partir du composé de l'EXEMPLE 134. On obtient l'énantiomère dextrogyre et son antipode ; F = 174°C

($H_2O$) ; $[\alpha]^{20}_D$ = + 107° (c = 1, acétate d'éthyle).

- idem à partir du composé de l'EXEMPLE 131. On obtient l'énantiomère dextrogyre et son antipode ; F = 91°C ; $[\alpha]^{20}_D$ = + 129° (c = 1, acétate d'éthyle).
- idem à partir de l'EXEMPLE 112. On obtient l'énantiomère dextrogyre et son antipode ; F = 273°C ; $[\alpha]^{20}_D$ = + 88,3° (c = 1, acétate d'éthyle).

EXEMPLE 140

**5-Chloro-3-[2-chloro-5-(hydroxyméthyl)phényl]-1-(2,4-diméthoxybenzyl)-3-méthylindolin-2-one**

(I) :

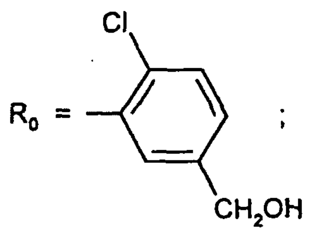

$R_1$ = $-CH_3$ ; $R_2$ = H ; $R_3$ = 4 $-OCH_3$ ; $R_4$ = 2-$OCH_3$
X = 5-Cl; Y = H

A 3,3 g du composé racémique de l'EXEMPLE 41 dans 130 ml de dichlorométhane à-68°C, on ajoute 16,5 ml d'une solution d'hydrure de disobutylaluminium (DIBAL). A-30°C, on traite le mélange réactionnel avec 10 ml de méthanol puis avec une solution aqueuse de chlorure d'ammonium et extrait avec du dichlorométhane. On filtre sur célite, lave la phase organique à l'eau, sèche sur sulfate de sodium anhydre et évapore les solvants sous pression réduite. On obtient le produit attendu après cristallisation dans un mélange cyclohexane/heptane. ; F = 97°C.

On peut également obtenir ce produit par déprotection en milieu acide du composé de l'EXEMPLE 40 dans les conditions de l'EXEMPLE 65.

**EXEMPLE 141**

**5-Chloro-3-[2-chloro-5-(méthoxyméthyl)phényl]-1-(2,4-diméthoxybenzyl)-3-méthylindolin-2-one**

(I) :

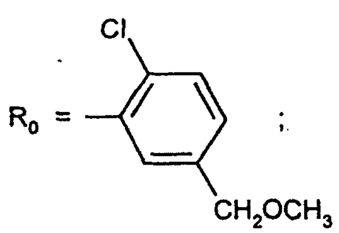

$R_1$ = $-CH_3$ ; $R_2$ = H ; $R_3$ = 4 $-OCH_3$ ; $R_4$ = 2-$OCH_3$
X = 5-Cl ; Y = H

A 0,2 g du composé de l'EXEMPLE 140 dans 2 ml de tétrahydrofurane, on ajoute 0,08 ml d'iodure de méthyle puis, à 0°C, 0,02 g d'hydrure de sodium en suspension à 60% dans de l'huile. Après 16 heures d'agitation à température ambiante, on hydrolyse le mélange réactionnel avec une solution aqueuse de chlorure d'ammonium à 5% et on extrait avec de l'acétate d'éthyle. La phase organique est lavée à l'eau et séchée sur sulfate de sodium anhydre. On évapore les solvants sous pression réduite. Le produit est obtenu après cristallisation dans du *n*-pentane ; F = 122°C.

De la même manière, on obtient les composés des EXEMPLES 142 à 144 suivants :

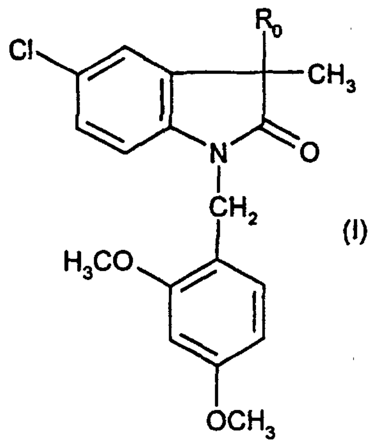

(I)

**TABLEAU 14**

| EXEMPLES | $R_0$ | F ; °C |
|---|---|---|
| 142 | 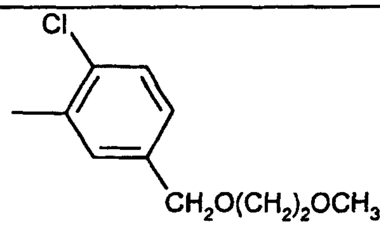 | 114 |
| 143 | 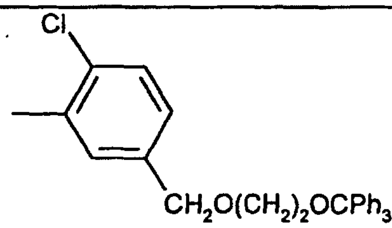 | 71 |
| 144 | 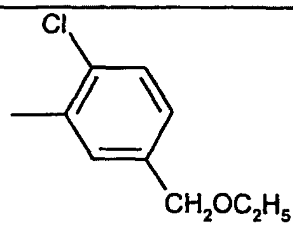 | 119 |

**EXEMPLE 145**

**5-Chloro-3-{2-chloro-5-[(diméthylamino)méthyl]phényl}-1-(2,4-diméthoxybenzyl)-3-méthylindolin-2-one**

(I) :

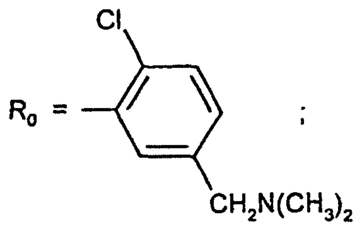

;

$R_1$ = $-CH_3$ ; $R_2$ = H ; $R_3$ = 4 $-OCH_3$ ; $R_4$ = 2-$OCH_3$
X = 5-Cl ; Y = H

a) Préparation du 4-chloro-3-[5-chloro-1-(2,4-diméthoxybenzyl)-3-méthyl-2-oxoindolin-3-yl]benzaldéhyde
A 0,69 g de pyridinium chlorochromate dans 10 ml de dichlorométhane, on ajoute 1 g du composé de l'EXEMPLE 140. Après 1 heure d'agitation à 10°C, on filtre sur célite, évapore les solvants sous pression réduite et purifie le résidu par chromatographie sur une colonne de gel de silice en éluant avec un mélange cyclohexane/acétate d'éthyle 90/10 (v/v). Le produit attendu cristallise dans du pentane ; F = 134°C.
b) Le composé de l'EXEMPLE 145 est obtenu par amination réductrice du composé obtenu en a) selon le mode opératoire de l'EXEMPLE 33 ; F = 125°C (0,6 $H_2O$).

De la même manière on obtient les composés des EXEMPLES 146 à 149 suivants :

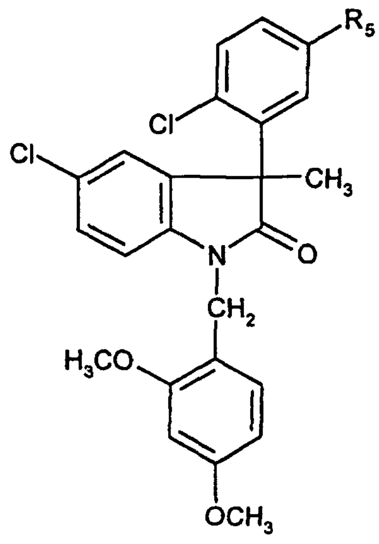

(I)

## TABLEAU 15

| EXEMPLES | $R_5$ | F ; °C<br>sel |
|---|---|---|
| 146 | $-CH_2NHCH_3$ | 76<br>$H_2CO_3$ |
| 147 | 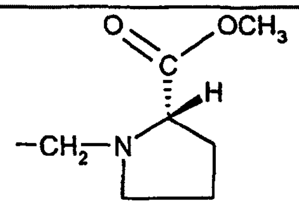 | 102 |
| 148 | 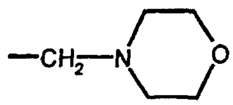 | 145 |
| 149 | 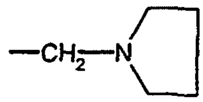 | 106<br>0,5 $H_2O$ |

Le composé racémique de l'EXEMPLE 148 est chromatographié sur une colonne chirale dans des conditions analogues à l'EXEMPLE 102. On obtient l'énantiomère dextrogyre salifié par acide chlorhydrique dans de l'éther éthylique et son antipode ; F = 139°C.

### EXEMPLE 150

***N*-{4-Chloro-3-[5-chloro-1-(2,4-diméthoxybenzyl)-3-méthyl-2-oxoindolin-3-yl]benzyl}-*N*-méthylacétamide**

(I) :

$R_0$ = 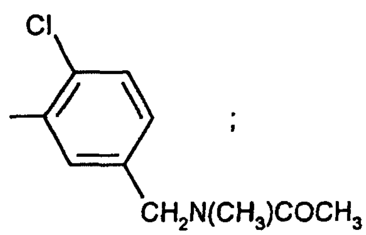 ;

$R_1$ = $-CH_3$ ; $R_2$ = H ; $R_3$ = 4 $-OCH_3$ ; $R_4$ = 2-$OCH_3$
X = 5-Cl ; Y = H

Obtenu selon le même mode opératoire que le composé de l'EXEMPLE 62 à partir de l'EXEMPLE 146 ; F = 81 °C (0,6 $H_2O$).

**EXEMPLE 151**

**5-Chloro-3-{2-chloro-5-[(2-hydroxyéthoxy)méthyl]phényl}-1-(2,4diméthoxybenzyl)-3-méthylindolin-2-one**

(I) :

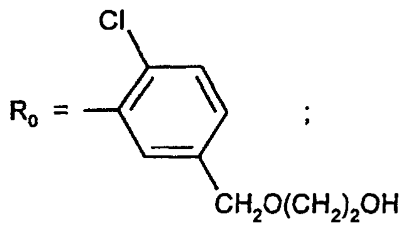

$R_1$ = -$CH_3$ ; $R_2$ = H ; $R_3$ = 4 -$OCH_3$ ; $R_4$ = 2-$OCH_3$
X = 5-Cl ; Y = H

a) Préparation du 5-chloro-3-[2-chloro-5-(1,3-dioxolan-2-yl)phényl]-1-(2,4-diméthoxybenzyl) -3-méthylindolin-2-one
A 2,044 g du composé préparé en a) de l'EXEMPLE 145 en solution dans 22 ml de toluène, on ajoute 1 ml d'éthylèneglycol et 16 mg d'acide *p*-toluènesulfonique. On chauffe le mélange réactionnel à reflux pendant 16 heures dans un réacteur muni d'un système Dean Stark afin d'éliminer l'eau provenant de la réaction. Après refroidissement à température ambiante, on ajoute 30 ml d'eau et extrait avec de l'acétate d'éthyle. On sèche la phase organique sur sulfate de sodium anhydre et évapore les solvants sous pression réduite pour obtenir le produit attendu directement utilisé dans l'étape suivante.
b) A 2,20 g du composé préparé en a), en solution dans 14 ml de dichlorométhane, à 6°C, on ajoute une solution de borohydrure de zinc à 0,29M dans de l'éther diéthylique (préparé selon la méthode décrite dans Chem. Pharm. Bull. 1984, *32*(4), 1411-1415), puis 1,2 ml de chlorure de triméthylsilane. Après 2 heures 30 minutes d'agitation à température ambiante, on hydrolyse le mélange réactionnel avec 30 ml d'acide chlorhydrique 1N et extrait avec de l'acétate d'éthyle. La phase organique est lavée à l'eau, séchée sur sulfate de sodium anhydre et les solvants sont évaporés sous pression réduite. On purifie le résidu obtenu par chromatographie sur une colonne de gel de silice en éluant avec un mélange dichlorométhanelméthanol 99/1 (v/v). Le produit final est obtenu après cristallisation dans du n-pentane ; F = 53°C.

Ce produit peut également être obtenu par déprotection en milieu acide du composé de l'EXEMPLE 143 selon T. L. 1977, 3473 ou toute autre méthode décrite dans Protective Groups in O. S. par T. W. Green *et al.* Chez WILEY-INTERSCIENCE (3rd Edition, 1999).

**EXEMPLE 152**

**5-Chloro-3-(2-chloro-5-{[2-(4-morpholinyl)éthoxy]méthyl}phényl)-1-(2,4-diméthoxy benzyl)-3-méthylindolin-2-one**

(I) :

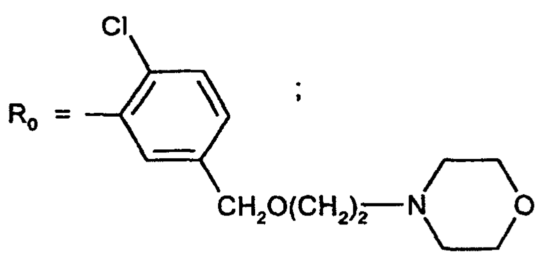

$R_1$ = -$CH_3$ ; $R_2$ = H ; $R_3$ = 4 -$OCH_3$ ; $R_4$ = 2-$OCH_3$
X = 5-Cl ; Y = H

a) Préparation du dérivé 5-chloro-3-(2-chloro-5-({2-[(4-méthylphényl)sulfonyl)éthoxy} méthyl)phényl]-1-(2,4-diméthoxybenzyl)-3-méthylindolin-2-one
A 0,48 g du composé de l'EXEMPLE 151 dans 1,5 ml de tétrahydrofurane à 0°C, on ajoute 0,39 ml de triéthylamine puis 0,27 g de chlorure de *p*-toluènesulfonyle. Après 16 heures d'agitation à température ambiante, on hydrolyse le mélange réactionnel avec 10 ml d'eau et extrait avec de l'acétate d'éthyle. On lave la phase organique avec une solution aqueuse d'hydrogénocarbonate de sodium, puis avec de l'eau. On sèche la phase organique sur sulfate de sodium anhydre et évapore les solvants sous pression réduite pour obtenir le produit attendu sous forme de pâte que l'on utilise dans l'étape suivante.
b) A 0,75 g du composé obtenu en a) en solution dans 2 ml d'acétonitrile, on ajoute 0,12 g de carbonate de sodium puis 0,20 ml de morpholine. Après 2 heures à 75°C, on refroidit le mélange réactionnel à température ambiante, hydrolyse avec 20 ml d'eau et extrait avec de l'acétate d'éthyle. On lave la phase organique encore une fois à l'eau, la sèche sur sulfate de sodium anhydre et évapore les solvants sous pression réduite. On purifie le résidu par chromatographie sur une colonne de gel de silice en éluant avec un mélange cyclohexane/acétate d'éthyle 20/80 (v/v). On obtient le produit attendu après chlorhydration avec un solution d'acide chlorhydrique dans de l'éther diéthylique, évaporation et cristallisation du résidu dans du *n*-pentane ; F = 81°C (0,7 $H_2O$, 1 HCl).

De la même manière que pour l'EXEMPLE 102, on obtient les énantiomères du composé de l'EXEMPLE 152 qui sont salifiés avec de l'acide fumarique dans de l'acétone. On isole les fumarates après évaporation de l'acétone et cristallisation dans de l'éther diéthylique :
l'énantiomère dextrogyre : F = + 112°C ; $[\alpha]^{20}_D$ = + 76,7 (c = 1 , $CH_3OH$) et son antipode . De la même manière on obtient les composés racémiques des EXEMPLES 153 à 157 suivants :

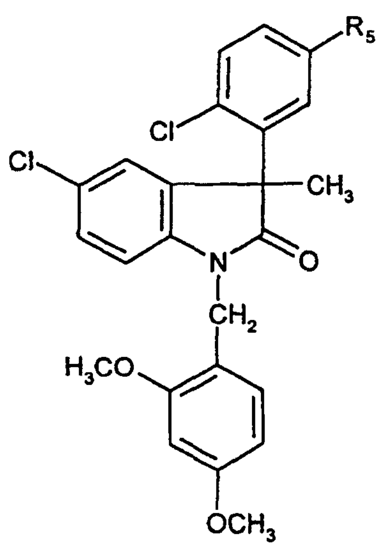

(I)

## TABLEAU 16

| EXEMPLES | $R_5$ | F ; °C sel |
|---|---|---|
| 153 | 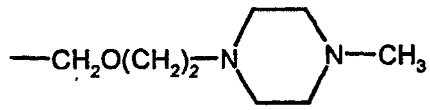 | 98<br>4 $H_2O$ ; 1 HCl |
| 154 | 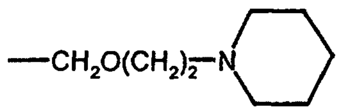 | 61<br>1 $H_2O$ ; 1 HCl |
| 155 | 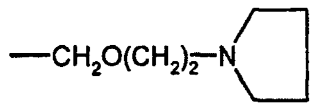 | 83<br>0,5 $H_2O$ ; 1 HCl |
| 156 | 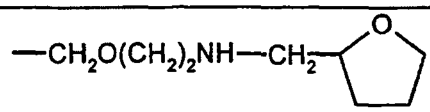 | 95<br>1 $H_2O$ ; 1 HCl |
| 157 | $-CH_2O(CH_2)_2N(CH_3)_2$ | 111<br>1 HCl , 1,5 $H_2O$ |

Selon le mode opératoire décrit pour l'EXEMPLE 18, on prépare les composés des EXEMPLES 158 à 162 ci après :

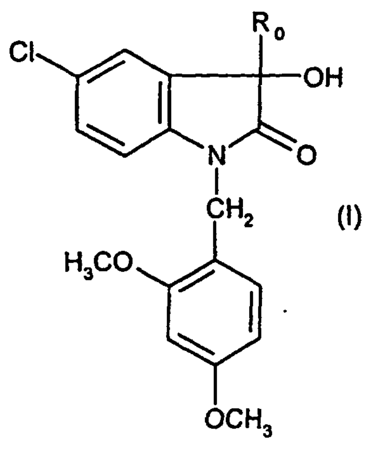

(I)

## TABLEAU 17

| EXEMPLES | $R_0$ | F ; °C sel |
|---|---|---|
| 158 | 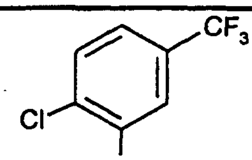 | 154 |
| 159 | 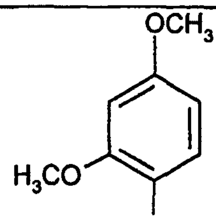 | 187 |
| 160 | 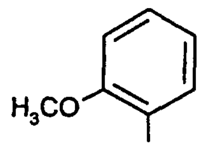 | 153 |
| 161 | 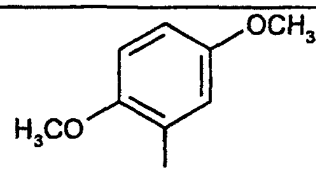 | 184 |
| 162 | 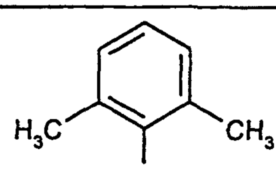 | 206 |
| 163 | 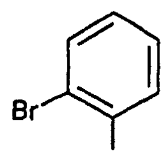 | 172 |

**EXEMPLE 164**

**5-Chloro-3-(2-chlorophényl)-1-(4-hydroxy-2-méthoxybenzyl)3-méthylindolin-2-one**

$R_0$ = 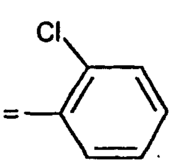 ;

$R_1$ = $-CH_3$ ; $R_2$ = H ; $R_3$ = 4-OH ; $R_4$ = 2-$OCH_3$ ; X = 5-Cl
Ce composé est déjà décrit dans a) de l'EXEMPLE 63.
F = 200°C.

**EXEMPLE 165**

**4-Chloro-3-[5-chloro-1-(2,4-diméthoxybenzyl)-3-hydroxy-2-oxoindolin-3-yl]-*N*,*N*-diéthylbenzamide**

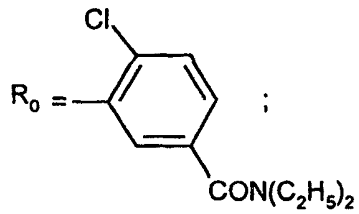

$R_1$ = -OH ; $R_2$ = H ; $R_3$ = 4-$OCH_3$ ; $R_4$ = 2-$OCH_3$ ; X = 5-Cl ; Y = H

a) **Acide 4-chloro-3-[5-chloro-1-(2,4-diméthoxybenzyl)-3-hydroxy-2-oxoindolin-3-yl] benzoïque.**
A partir du composé de l'EXEMPLE 20 et dans les conditions décrites dans l'EXEMPLE 101, on isole un solide que l'on engage dans l'étape suivante ; F = 200°C
b) En traitant l'acide précédant dans les conditions de l'EXEMPLE 102 on obtient le composé attendu ; F = 244°C.

**EXEMPLE 166**

**1-[4-Chloro-3-[5-chloro-1-(2,4-diméthoxybenzyl)-3-méthyl-2-oxoindolin-3-yl]benzoyl]-4-méthoxypipéridine**

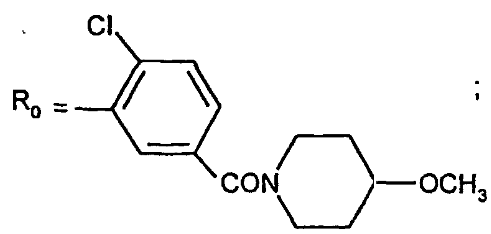

$R_1$ = -$CH_3$ ; $R_2$ = H ; $R_3$ = 4-$OCH_3$ ; $R_4$ = 2-$OCH_3$ ; X = 5-Cl ; Y = H

a) **4-méthoxypipéridine :**
A 11 g de 1-*tert*-butoxycarbonyl-4-hydroxypipéridine, préparée selon J. Med. Chem., 1998, 41, 25, 4983-4994, dilué dans 400 ml de dichlorométhane et 22,2 ml de 2,6-diterbutylpyridine, vers -20°C, on ajoute lentement 21,8 ml de trifluorométhane sulfonate de méthyle. Après 16 heures à 20°C on hydrolyse avec de l'acide chlorhydrique 0,5 N et extrait au dichlorométhane. On isole la phase organique sur sulfate de sodium, évapore le solvant sous pression réduite et purifie le résidu sur une colonne de silice en éluant avec un mélange dichlorométhanelcyclohexane 60/40. L'huile obtenue est engagée dans l'étape de déprotection suivante en présence de 50 ml d'une solution de chlorure d'hydrogène 2M dans de l'acétate d'éthyle. Après deux heures à 20°C, on évapore sous pression réduite, on triture le résidu avec de l'éther éthylique, filtre le solide blanc que l'on sèche sous pression réduite vers 50°C pendant trois heures. On obtient le chlorhydrate du composé attendu ; F = 135°C.
b) En traitant l'énantiomère dextrogyre du composé de l'EXEMPLE 101 avec l'amine décrite en a) dans des conditions analogues à l'EXEMPLE 102, on isole le produit attendu cristallisé dans de l'éther isopropylique ; F = 92°C ($1H_2O$) ;
$[\alpha]^{20}_D$ = + 93, 3° (c = 1, acétate d'éthyle).

**EXEMPLE 167**

**1-[4-Chloro-3-[5-chloro-1-(2,4-diméthoxybenzyl)-3-méthyl-2-oxoindolin-3-yl]benzoyl] 4-éthoxypipéridine**

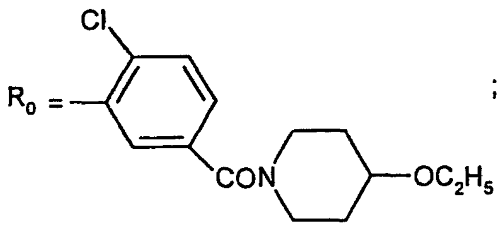

;

$R_1$ = -$CH_3$ ; $R_2$ = H ; $R_3$ = 4-$OCH_3$ ; $R_4$ = 2-$OCH_3$ ; X = 5-Cl ; Y = H

a) **4-éthoxypipéridine** :
Dans les conditions de l'EXEMPLE 166 a), en utilisant du trifluorométhane sulfonate d'éthyle, on isole le chlorhydrate de l'amine attendue ; F = 148°C
b) L'EXEMPLE 167 est obtenu comme pour l'EXEMPLE 166 b), en utilisant l'amine ci-dessus ; F = 108°C (1 $H_2O$) $[\alpha]^{20}_D$ = + 100,1 ° (c = 1, acétate d'éthyle).

**EXEMPLE 168**

**1-[4-Chloro-3-[5-chloro-1-(2,4-diméthoxybenzyl)-3-méthyl-2-oxoindolin-3-yl]benzoyl]-(R)-2-pyrrolidinocarbonylpipéridine**

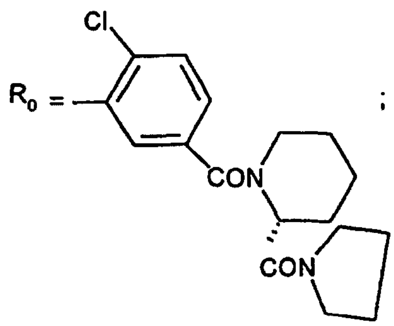

;

$R_1$ = -$CH_3$ ; $R_2$ = H ; $R_3$ = 4-$OCH_3$ ; $R_4$ = 2-$OCH_3$ ; X = 5-Cl ; Y = H

a) **(R)-2-(pyrrolidinocarbonyl)-1-(*ter*butoxycarbonyl)-pipéridine**
A partir d'acide 1-*ter*butoxycarbonyl-(R)-2-pipéridine carboxylique et de pyrrolidine, dans des conditions analogues à l'EXEMPLE 102, on obtient le produit attendu après purification sur une colonne de silice en éluant avec un mélange dichlorométhane/méthanol 98/2 ; F = 105°C.
b) **(R)-2-(pyrrolidinocarbonyl)-pipéridine, chlorhydrate**
Déprotection du composé précédent dans les conditions décrites dans l'EXEMPLE 166 a) ; F = 258°C.
c) L'EXEMPLE 168 est obtenu avec l'amine précédente et comme pour l'EXEMPLE 166 b), on obtient le produit attendu cristallisé dans de l'éther isopropylique; F = 114°C (0,5$H_2O$).

**EXEMPLE 169**

**1-[4-Chloro-3-[5-chloro-1-(2,4-diméthoxybenryl)-3-méthyl-2-oxoindolin-3-yl]benzoyl]-(R)-2-*N*,*N*-diméthylaminocarbonylpipéridine**

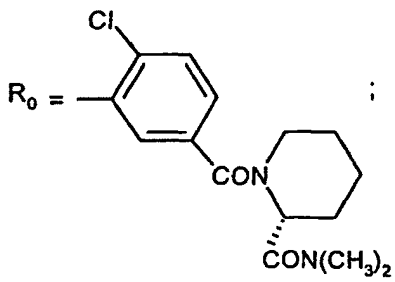

$R_1$ = -$CH_3$ ; $R_2$ = H ; $R_3$ = 4-$OCH_3$ ; $R_4$ = 2-$OCH_3$ ; X = 5-Cl ; Y = H

a) ***N*,*N*-diméthyl-1-*ter*butoxycarbonyl-(R)-2-pipéridine carboxamide :**
Obtenu comme pour l'EXEMPLE 168 a) ; F = 76°C
b) ***N*,*N*-diméthyl-(R)-2-pipéridine carboxamide/chrorhydrate :**
Obtenu comme pour l'EXEMPLE 168 b) ; F = 194.4°C.
c) L'EXEMPLE 169 est obtenu avec l'amine précédente et comme pour l'EXEMPLE 166 b) , on obtient le produit attendu qui cristallise dans l'éther isopropylique ; F = 123°C (1$H_2O$).

**EXEMPLE 170**

**1-[4-Chloro-3-[5-chloro-1-(2,4-diméthoxybenzyl)-3-méthyl-2-oxoindolin-3-yl]benzoyl]-(R)-2-(*N*-méthyl-*N*-2,2,2-trifluoroéthylaminocarbonyl)pipéridine**

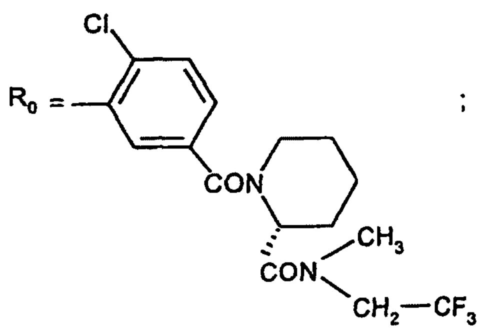

$R_1$ = -$CH_3$ ; $R_2$ = H ; $R_3$ = 4-$OCH_3$ ; $R_4$ = 2-$OCH_3$ ; X = 5-Cl ; Y = H

**a) *N*-méthyl-N-2,2,2-trifluoroéthyl-1-*ter*butoxycarbonyl-(R)-2-pipéridinecarboxamide :**
Obtenu comme pour l'EXEMPLE 169 a) avec le chlorhydrate de la *N*-méthyl-2,2,2-trifluoroéthylamine (F = 185°C) préparée selon J.O.C., 1959, 24, 1256 ; F = 93°C.
**b)** L'EXEMPLE 170 est obtenu en déprotègeant l'amine comme pour l'EXEMPLE 169 b), le chlorhydrate hygroscopique obtenu est engagé avec l'énantiomère dextrogyre du composé de l'EXEMPLE 101 dans des conditions analogues à l'EXEMPLE 102. On isole le produit attendu cristallisé dans du pentane ; F = 99°C
$[\alpha]^{20}_D$ = + 103,6° (c = 1, acétate d'éthyle).

**EXEMPLE 171**

**4-Chloro-3-[5-chloro-1-(2,4-diméthoxybenzyl)-3-méthyl-2-oxoindolin-3-yl]-*N*-méthyl-*N*-(2,2,2-trifluoroéthyl) benzamide**

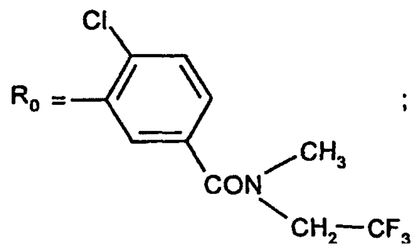

;

$R_1$ = $-CH_3$ ; $R_2$ = H ; $R_3$ = 4-$OCH_3$ ; $R_4$ = 2-$OCH_3$ ; X = 5-Cl ; Y = H

Obtenu selon l'EXEMPLE 166 b) avec le chlorhydrate de la N-méthyl-2,2,2-trifluoroéthylamine cité en a) de l'EXEMPLE 170 ; F = 89°C
$[\alpha]^{20}_D$ = + 83,4° (c = 1, acétate d'éthyle).

**EXEMPLE 172**

**1-[4-Chloro-3-[5-chloro-1-(2,4-diméthoxybenzyl)-3-méthyl-2-oxoindolin-3-yl]benzoyl]-4-difluorométhylidène-pipéridine**

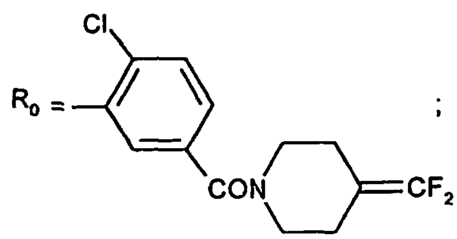

;

; $R_1$ = $-CH_3$ ; $R_2$ = H ; $R_3$ = 4-$OCH_3$ ; $R_4$ = 2-$OCH_3$ ; X = 5-Cl ; Y = H

a) **4-difluorométhylidène-pipéridine, chlorhydrate :**
Obtenu par déméthylation du composé *N*-méthyl correspondant, décrit dans Tetrahedron, 1980, 36, 3241, par action d'$\alpha$-chloroéthylchloroformate selon J.O.C. 1984, 49, 2081-2082 ; F = 211,5°C.
b) L'EXEMPLE 172 est préparé selon l'EXEMPLE 166 b) avec l'amine préparée en a).
On isole le produit attendu par cristallisation dans le pentane. F = 119°C.

**EXEMPLE 973**

**1-[4-Chloro-3-[5-chloro-1-(2,4-diméthoxybenzyl)-3-méthyl-2-oxoindolin-3-yl]benzoyl]-(R)-2-éthoxycarbonyl-(R)-4-méthylpipéridine**

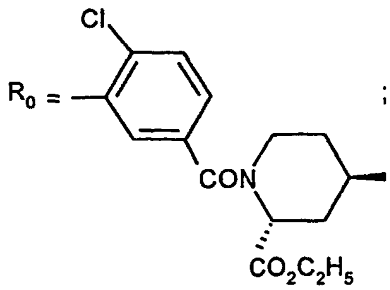

$R_1$ = $-CH_3$ ; $R_2$ = H ; $R_3$ = 4-$OCH_3$ ; $R_4$ = 2-$OCH_3$ ; X = 5-Cl ; Y = H

Obtenu selon b) de l'EXEMPLE 166 avec la (R)-4-méthyl-(R)-2-pipéridine carboxylate d'éthyle décrite dans J. Med. Chem. 37, 1994, 23, 3889-3901. On isole le produit attendu cristallisé dans du pentane ; F = 106°C.

**EXEMPLE 174**

**1-[4-Chloro-3-[5-chloro-1-(2,4-diméthoxybenzyl)-3-méthyl-2-oxoindolin-3-yl]benzoyl]-(S)-2-méthylpipéridine**

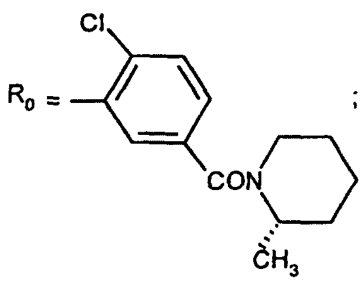

$R_1$ = $-CH_3$ ; $R_2$ = H ; $R_3$ = 4-$OCH_3$ ; $R_4$ = 2-$OCH_3$ ; X = 5-Cl ; Y = H

Obtenu selon b) de l'EXEMPLE 166 avec la (S)-2-méthylpipéridine décrite dans Tetrahedron Asymetry 8, 1997, 8, 1275-1278. F = 102°C (0,5$H_2O$).

**EXEMPLE 175**

**1-[4-Chloro-3-[5-chloro-1-(2,4-diméthoxybenzyl)-3-méthyl-2-oxoindolin-3-yl]benzoyl]-(R)-2-éthoxycarbonylpipéridine**

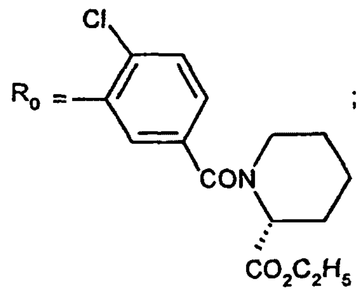

$R_1$ = $-CH_3$ ; $R_2$ = H ; $R_3$ = 4-$OCH_3$ ; $R_4$ = 2-$OCH_3$ ; X = 5-Cl ; Y = H

Obtenu selon b) de l'EXEMPLE 166 avec la (R)-2-pipéridine-carboxylate d'éthyle décrite dans J. Med. Chem. 42, 1999, 22, 4584-4603. F = 113°C.

**EXEMPLE 176**

**1-[4-Chloro-3-[5-chloro-1-(2,4-diméthoxybenzyl)-3-méthyl-2-oxoindolin-3-yl]benzoyl]-(R)-2-*ter*butyloxycarbonylpipéridine**

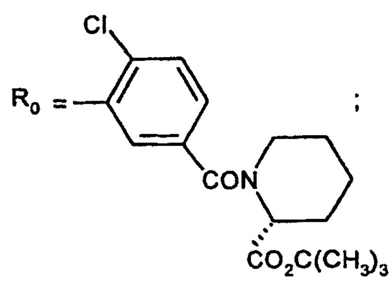

$R_1$ = $-CH_3$ ; $R_2$ = H ; $R_3$ = 4-$OCH_3$ ; $R_4$ = 2-$OCH_3$ ; X = 5-Cl ; Y = H

a) **(R)-2-pipéridine-carboxylate de *tert*-butyle :**
Dans un autoclave, on place le mélange de 0,5 g de (R)-homoproline, 22 ml de dioxanne, 2,2 ml d'acide sulfurique concentré puis environ 20 ml d'isobutylène condensé à basse température. Après vingt-quatre heures d'agitation à température ambiante on verse le milieu refroidit vers -10°C sur 150 ml d'une solution aqueuse de carbonate de potassium puis on extrait avec de l'acétate d'éthyle. Les phases organiques réunies sont lavées à l'eau, séchées sur $NO_2SO_4$, évaporées à sec. Le résidu est distillé sous pression réduite. Eb = 46°C sous 30 Pa.
b) L'EXEMPLE 176 est obtenu selon b) de l'EXEMPLE 166 avec l'amine préparée en a). Le produit attendu est cristallisé dans du pentane. F = 202,2°C.

**EXEMPLE 177**

**4-Chloro-3-[5-chloro-1-(2,4-diméthoxybenzyl)-3-méthyl-2-oxoindolin-3-yl]*N*-éthyl-*N*-(2-diméthylaminoéthyl) benzamide, chlorhydrate**

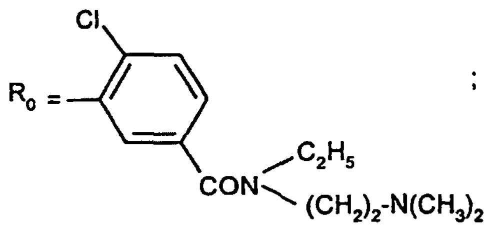

;

$R_1$ = $-CH_3$ ; $R_2$ = H ; $R_3$ = 4-$OCH_3$ ; $R_4$ = 2-$OCH_3$ ; X = 5-Cl ; Y = H

Obtenu selon b) de l'EXEMPLE 166 avec la *N*-éthyl-2-diméthylaminoéthylamine décrite dans J.A.C.S., 1963, 2256-2266. On isole le chlorhydrate du composé attendu dans de l'éther éthylique. F = 171,5°C (1 $H_2O$)
$[\alpha]^{20}_D$ = + 99° (C = 1, méthanol).

**EXEMPLE 178**

**4-Chloro-3-[5-chloro-1-(2,4-diméthoxybenzyl)-3-méthyl-2-oxoindolin-3-yl]-*N*-éthyl-*N*-(2-morpholinoéthyl)benzamide**

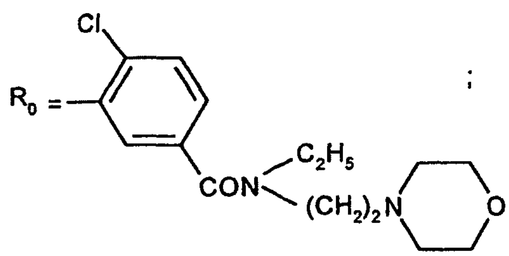

;

$R_1$ = $-CH_3$ ; $R_2$ = H ; $R_3$ = 4-$OCH_3$ ; $R_4$ = 2-$OCH_3$ ; X = 5-Cl ; Y = H

Obtenu selon b) de l'EXEMPLE 166 avec la *N*-éthyl-2-morpholino-éthylamine décrite dans Chem. Pharm. Bull. 45, 1997, *6*, 996-1007. F = 143°C (O,5$H_2O$).

**EXEMPLE 179**

**4-Chloro-3-[5-chloro-1-(2,4-diméthoxybenzyl)-3-méthyl-2-oxoindolin-3-yl]-*N*-éthyl-*N*-[2-(pyridin-4-yl)éthyl] benzamide**

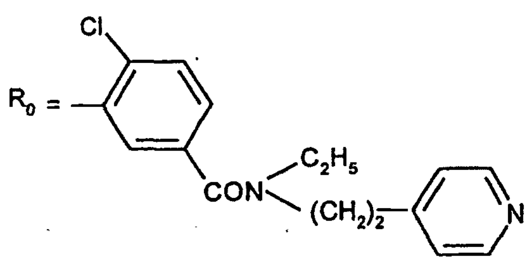

;

$R_1$ = -$CH_3$ ; $R_2$ = H ; $R_3$ = 4-$OCH_3$ ; $R_4$ = 2-$OCH_3$ ; X = 5-Cl ; Y = H

Obtenu selon b) de l'EXEMPLE 166 avec la *N*-éthyl-2-(pyridin-4-yl)-éthylamine décrite dans J.A.C.S., 1956, *78*, 4441. On isole le chlorhydrate du produit attendu dans de l'éther éthylique. F = 185°C (1,5 $H_2O$)

## EXEMPLE 180

**4-Chloro-3-[5-chloro-1-(2,4-diméthoxybenzyl)-3-méthyl-2-oxoindolin-3-yl]-*N*-éthyl-*N*-(2,2,2-trifluoroéthyl)benzamide**

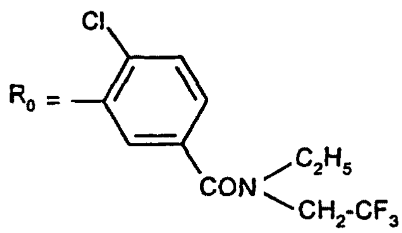
;

$R_1$ = -$CH_3$ ; $R_2$ = H ; $R_3$ = 4-$OCH_3$ ; $R_4$ = 2-$OCH_3$ ; X = 5-Cl ; Y = H

Obtenu selon b) de l'EXEMPLE 166 avec la *N*-éthyl-2,2,2-trifluoroéthylamine décrite dans J.A.C.S. 113, 1991, *4*, 1288-1294. F = 80°C (0,5 $H_2O$)

## EXEMPLE 181

**4-Chloro-3-[5-chloro-1-(2,4-diméthoxybenzyl)-3-méthyl-2-oxoindolin-3-yl]-*N*-éthyl-*N*-[2-(pyridin-2-yl)éthyl] benzamide, chlorohydrate**

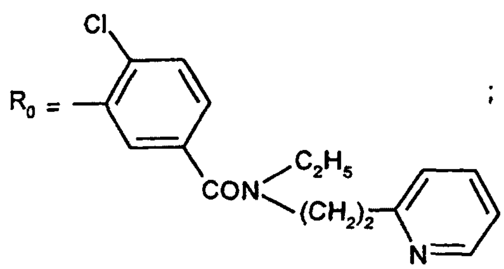
;

$R_1$ = -$CH_3$ ; $R_2$ = H ; $R_3$ = 4-$OCH_3$ ; $R_4$ = 2-$OCH_3$ ; X = 5-Cl ; Y = H

Obtenu selon b) de l'EXEMPLE 166 avec la *N*-éthyl-2-(pyridin-2-yl)éthylamine décrite dans J.A.C.S., 1955, 5434.

On isole le chlorhydrate du produit attendu dans de l'éther éthylique. F = 202°C (1 $H_2O$)

**EXEMPLE 182**

**4-Chloro-3-[5-chloro-1-(2,4-diméthoxybenzyl)-3-méthyl-2-oxoindolin-3-yl]-*N*-éthyl-*N*-(2-pyrrolidinoéthyl)benzamide, chlorhydrate**

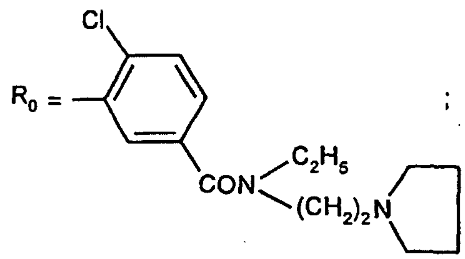

$R_1$ = $-CH_3$ ; $R_2$ = H ; $R_3$ = 4-$OCH_3$ ; $R_4$ = 2-$OCH_3$ ; X = 5-Cl ; Y = H

Obtenu selon b) de l'EXEMPLE 166 avec la *N*-éthyl-2-pyrrolidinoéthylamine décrite dans J. Med. Chem., 35, 1992, 1, 38-47. On isole le chlorhydrate du produit obtenu dans de l'éther éthylique. F = 109°C (1,5 $H_2O$)

**EXEMPLE 183**

**4-Chloro-3-[5-chloro-1-(2,4-diméthoxybenzyl)-3-méthyl-2-oxoindolin-3-yl]-*N*-éthyl-*N*-(2-pipéridinoéthyl)benzamide, chlorhydrate**

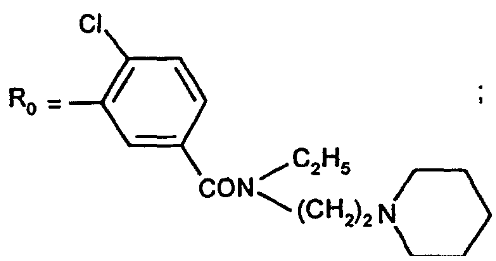

$R_1$ = $-CH_3$ ; $R_2$ = H ; $R_3$ = 4-$OCH_3$ ; $R_4$ = 2-$OCH_3$ ; X = 5-Cl ; Y = H

Obtenu selon b) de l'EXEMPLE 166 avec la *N*-éthyl-2-pipéridinoéthylamine décrite dans Chem. Pharm. Bull., 1997, *45*, 6, 996-1007.

**EXEMPLES 184 à 198**

Dans les conditions b) de l'EXEMPLE 166 et avec des amines commerciales on obtient les EXEMPLES 184 à 198 suivants :

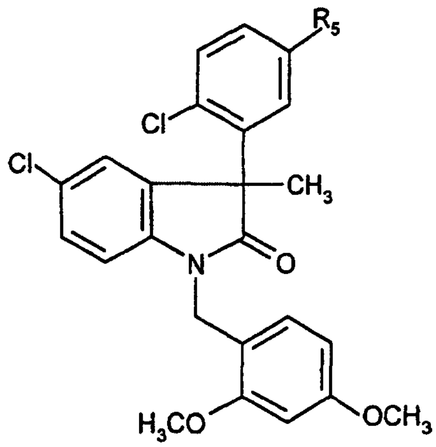

## TABLEAU 18

| EXEMPLES | $R_5$ | F ; °C | $[\alpha]^{20}_D$ |
|---|---|---|---|
| 184 | 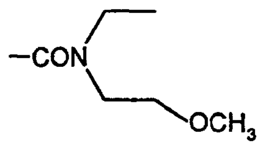 | 86,3 | |
| 185 | 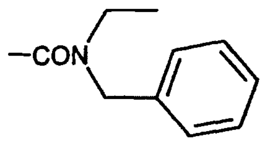 | 111,7 | |
| 186 | 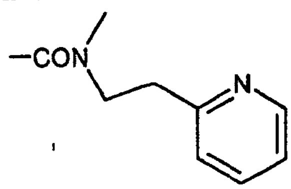 | 80,5<br>0,5 $H_2O$ | |
| 187 | 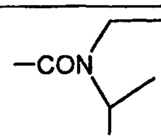 | 102,8 | |
| 188 | 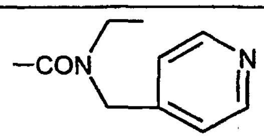 | 193,7<br>0,5 $H_2O$<br>1 HCl | + 96,8<br>(c = 1, méthanol) |
| 189 | 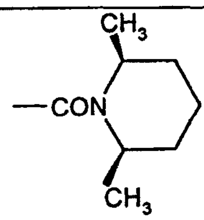 | 119,5<br>$H_2O$ | |
| 190 | 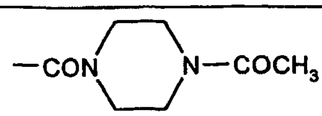 | 112<br>0,5 $H_2O$ | |

## TABLEAU 18 (suite 1)

| EXEMPLES | $R_5$ | F ; °C | $[\alpha]^{20}_D$ |
|---|---|---|---|
| 191 | 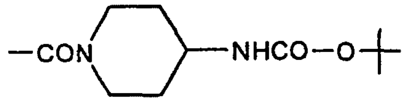 | 130<br>1,5 $H_2O$ | |
| 192 | 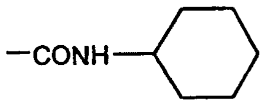 | 129,8 | + 114°<br>(C = 1, acétate d'éthyle) |
| 193 | 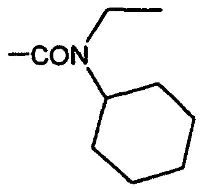 | 131 | |
| 194 | 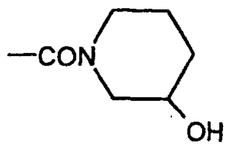 | 124<br>$2H_2O$ | |
| 195 | 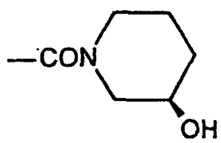 | 199,4<br>1,5 $H_2O$ | |
| 196 | 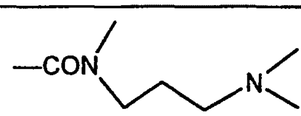 | 109<br>0,5 $H_2O$ | |
| 197 | 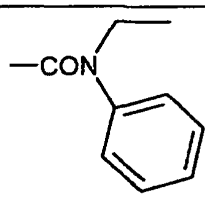 | 104<br>0,5 $H_2O$ | |
| 198 | 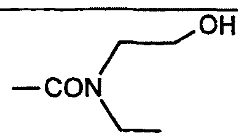 | 101<br>0,5 $H_2O$ | |
| 199 | 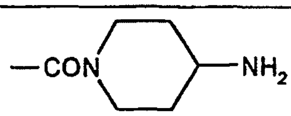 | 188<br>1 HCl | |

Le composé de l'EXEMPLE 199 est obtenu en traitant le composé de l'EXEMPLE 191 par une solution d'acide chlorhydrique dans de l'acétate d'éthyle, on isole le chlorhydrate après évaporation du solvant et reprise du résidu par du pentane.

**EXEMPLE 200**

**4-Chloro-3-[5-chloro-1-(2,4-diméthoxybenzyl)-3-méthyl-2-oxoindolin-3-yl]-*N*-éthyl-*N*-(2-pyridylméthyl)benzamide**

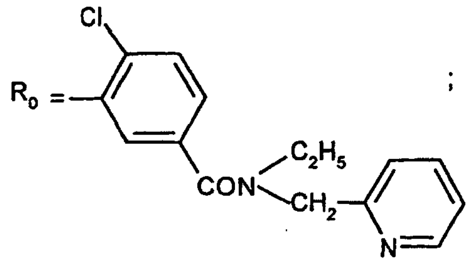

$R_1$ = $-CH_3$ ; $R_2$ = H ; $R_3$ = 4-$OCH_3$ ; $R_4$ = 2-$OCH_3$ ; X = 5-Cl ; Y = H

a) ***N*-éthyl-2-pyridylméthylamine :**
On ajoute 5 g de 2-pyridylcarboxaldéhyde au mélange de 3,8 g de chlorhydrate d'éthylamine, 60 ml de toluène, 110 ml d'éthanol et 13,2 ml de tri-éthylamine. Après 30 secondes d'agitation à 20°C on ajoute 25 g de tamis moléculaire 4 Å et maintient sous agitation à 20°C. On filtre l'insoluble, lave abondamment au dichlorométhane, évapore à sec et on reprend le résidu avec 50 ml de méthanol. A cette solution, vers 0°C, on ajoute 1,8 g de borohydrure de sodium. Après seize heures vers 20°C, on évapore le solvant sous pression réduite, on reprend le résidu avec du dichlorométhane. On lave la phase organique à la soude 1 N puis avec une solution aqueuse de chlorure de sodium, on la sèche sur sulfate de sodium, on évapore le solvant sous pression réduite puis distille le résidu. Eb. = 64°C sous 180 Pa.
b) L'EXEMPLE 200 est obtenu selon b) de l'EXEMPLE 166 avec l'amine préparée en a). F = 89°C (0,5 $H_2O$)

**EXEMPLE 201**

**4-Chloro-3-[5-chloro-1-(2,4-diméthoxybenzyl)-3-méthyl-2-oxoindolin-3-yl]-*N*-éthyl-*N*-(3-pyridylméthyl)benzamide**

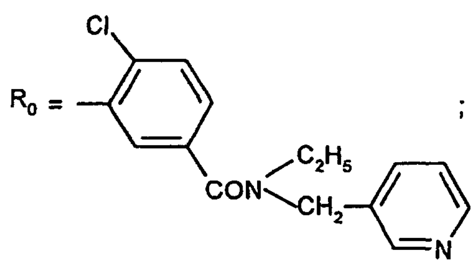

$R_1$ = $-CH_3$ ; $R_2$ = H ; $R_3$ = 4-$OCH_3$ ; $R_4$ = 2-$OCH_3$ ; X = 5-Cl ; Y = H

a) ***N*-éthyl-3-pyridylméthylamine :**
Obtenu de la même manière que dans l'EXEMPLE 200 a) à partir de la 3-pyridine carboxaldéhyde. Eb. = 77°C sous 530 Pa
b) L'EXEMPLE 201 est obtenu selon b) de l'EXEMPLE 166 avec l'amine préparée en a). F = 95,5°C (0,5 $H_2O$).

**EXEMPLE 202**

**4-Chloro-3-[5-chloro-1-(2,4-diméthoxybenzyl)-3-méthyl-2-oxoindolin-3-yl]-*N*-(2-diméthylaminoéthyl) -*N*-(2,2,2-trifluoroéthyl)benzamide, chlorhydrate**

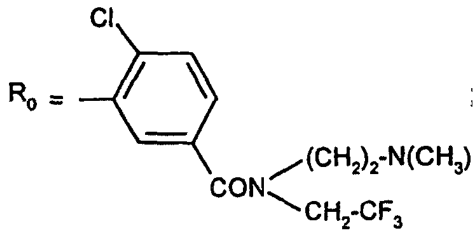

;

$R_1$ = -$CH_3$ ; $R_2$ = H ; $R_3$ = 4-$OCH_3$ ; $R_4$ = 2-$OCH_3$ ; X = 5-Cl ; Y = H

a) ***N*-(2-dimethylaminoethyl)trifluoroacetamide** :
A la solution de 6 g de 2-diméthylaminoéthylamine dans 150 ml de dichlorométhane et 23,9 ml de triéthylamine, on ajoute vers 0°C, 11,5 ml d'anhydride trifluoroacétique. A 20°C, on ajoute 50 ml d'une solution diluée de bicarbonate de sodium, on décante, on sèche la phase organique sur sulfate de sodium, on évapore le solvant et distille le résidu sous pression réduite. Eb. = 94°C sous 1975 Pa.
b) ***N*-2,2,2-trifluoroéthyl-2-diméthylaminoéthylamine :**
A 2,78 g d'hydrure de lithium aluminium dans 50 ml d'éther éthylique vers 0°C on ajoute la solution de 5 g d'amide préparé en a) dans 250 ml d'éther. Après une nuit d'agitation à 22°C on ajoute 20 ml d'une solution aqueuse saturée de sulfate de sodium, on filtre sur célite, on lave la célite avec 3 fois 100 ml d'éther, on évapore partiellement les filtrats réunis puis traite par une solution d'acide chlorhydrique dans de l'acétate d'éthyle. On filtre le chlorhydrate du produit attendu. F = 232,6°C.
c) L'EXEMPLE 202 est obtenu selon b) de l'EXEMPLE 166 avec l'amine préparée en b) puis salification par une solution d'acide chlorhydrique dans de l'éther éthylique. F = 201,5°C ($2H_2O$).

**EXEMPLE 203**

**4-Chloro-3-[5-chloro-1-(2,4-diméthoxybenzyl)-3-méthyl-2-oxoindolin-3-yl]-*N*-(3-diméthylaminopropyl)-*N*-éthylbenzamide, chlorhydrate**

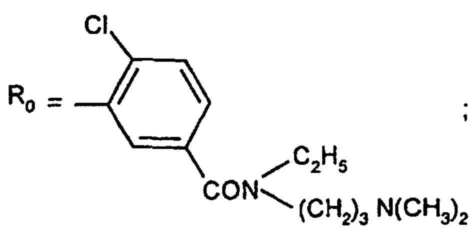

;

$R_1$ = -$CH_3$ ; $R_2$ = H ; $R_3$ = 4-$OCH_3$ ; $R_4$ = 2-$OCH_3$ ; X = 5-Cl ; Y = H

a) ***N*-(3-diméthylaminopropyl)-acétamide :**
Obtenu de la même manière que dans a) de l'EXEMPLE 202 avec de l'anhydride acétique et la 3-diméthylaminopropylamine. Eb. = 91 °C sous 84 Pa.
b) ***N*-éthyl-3-diméthylaminopropylamine :**
Obtenu dans des conditions analogues à b) de l'EXEMPLE 202, dans du tétrahydrofurane au reflux. Eb. = 75°C sous 45 Pa.
c) L'EXEMPLE 203 est obtenu selon b) de l'EXEMPLE 166 avec l'amine préparée en b). On isole le chlorhydrate par traitement avec de l'acide chlorhydrique dans de l'éther éthylique. F = 222°C.

**EXEMPLE 204**

**4-Chloro-3-[5-chloro-1-(2,4-diméthoxybenzyl)-3-méthyl-2-oxoindolin-3-yl)-N-éthyl-*N*-[3-(pyridin-4-yl)propyl] benzamide, chlorhydrate**

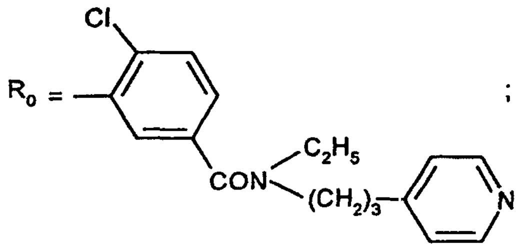

$R_1$ = $-CH_3$ ; $R_2$ = H ; $R_3$ = 4-$OCH_3$ ; $R_4$ = 2-$OCH_3$ ; X = 5-Cl ; Y = H

a) ***N*-éthyl-3-(pyridin-4-yl)propylamine** :
A partir de 3-(pyridin-4-yl)propionaldéhyde décrit dans J. Organometallic Chem. ; 599 ; 2 ; 2000 ; 298-303 et de chlorhydrate d'éthylamine, on obtient de façon analogue à a) de l'EXEMPLE 200, et après purification sur une colonne de silice en éluant avec un mélange dichlorométhane/méthanol 90/10 une huile que l'on engage dans l'étape suivante.
b) L'EXEMPLE 204 est obtenu selon b) de l'EXEMPLE 166 avec l'amine préparée en a). On isole le produit attendu après purification sur une colonne de silice en éluant par un mélange dichlorométhane/méthanol, 97/3, et chlorhydratation par une solution d'acide chlorhydrique dans de l'éther. F = 207°C.

**EXEMPLE 205**

**5-chloro-3-[2-chloro-5-(2-oxopipéridinométhyl)phényl]-1-(2,4-diméthoxybenzyl)-3-méthylindolin-2-one**

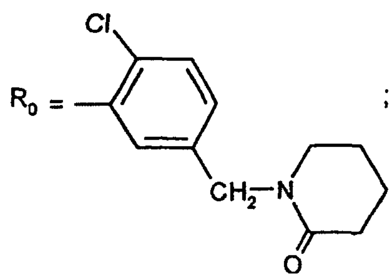

$R_1$ = $-CH_3$ ; $R_2$ = H ; $R_3$ = 4-$OCH_3$ ; $R_4$ = 2-$OCH_3$ ; X = 5-Cl ; Y = H

A la solution de 90 mg de chlorhydrate de 5-amino pentanoate de méthyle dans 3 ml de toluène, 2 ml d'éthanol et 150 ml de triéthylamine, vers 0°C, on ajoute 250 mg de l'aldéhyde préparé en a) de l'EXEMPLE 145. Quinze minutes après, on ajoute 1,9 g de tamis moléculaire 4 Å. Après trois heures vers 20°C, on filtre l'insoluble qui est lavé abondamment au dichlorométhane, on évapore les solvants du filtrat sous pression réduite. L'huile obtenue est reprise par 4,1 ml de méthanol, à 0°C on ajoute 20,1 mg de borohydrure de sodium . Après seize heures d'agitation vers 20°C, on évapore le solvant sous pression réduite, on reprend le résidu au dichlorométhane, on lave avec une solution aqueuse de soude 0,5*N* puis avec une solution aqueuse diluée de chlorure de sodium. On sèche la phase organique sur sulfate de sodium, évapore le solvant sous pression réduite, chromatographie le résidu sur une colonne de silice en éluant avec un mélange dichlorométhane/méthanot, 98/2. Le produit attendu est cristallisé dans du pentane. F = 72°C.

**EXEMPLE 206**

**1-[4-Chloro-3-[5-chloro-1-(4-chloro-2-méthoxybenzyl)-3-méthyl-2-oxoindolin-3-yl]benzoyl]pipéridine**

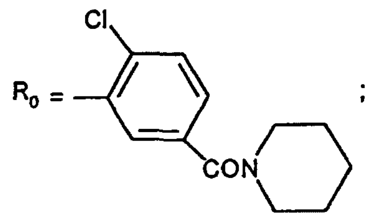

$R_1$ = -$CH_3$ ; $R_2$ = H ; $R_3$ = 4-Cl ; $R_4$ = 2-$OCH_3$ ; X = 5-Cl ; Y = H

a) **4-Chloro-3-[5-chloro-1-(4-chforo-2-méthoxybenzyl)-3-hydroxy-2-oxo-indolin-3-yl]benzoate de méthyle:**
A partir du *composé IV.2* et selon le mode opératoire décrit dans l'EXEMPLE 20, on obtient le produit attendu après chromatographie sur une colonne de silice en éluant avec un mélange cyclohexane/dichlorométhane, 50/50. F = 205°C

b) **4-Chloro-3-[3,5-dichloro-1-(4-chloro-2-méthoxybenzyl)-2-oxo-indolin-3-yl]benzoate de méthyle:**
Obtenu selon a) de la PREPARATION 13 à partir du composé décrit en a)

**c) 4-Chloro-3-[5-chloro-1-(4-chloro-1-(4-chloro-2-méthoxybenzyl)-3-H-2-oxo-indolin-3-yl]benzoate de méthyle ;** *composé III.9* :
Obtenu selon b) de la PREPARATION 13 à partir du composé décrit en b) ; F = 126°C.

d) **4-Chloro-3-[5-chloro-1-(4-chloro-2-méthoxybenzyl)-3-méthyl-2-oxo-indolin-3-yl]benzoate de méthyle :**
Obtenu selon le mode opératoire décrit dans l'EXEMPLE 37 à partir du *composé III.9* ; F = 158°C.

e) **Acide 4-chloro-3-[5-chloro-1-(4-chloro-2-méthoxybenzyl)-3-méthyl-2-oxoindolin-3-yl]benzoïque :**
Obtenu selon le mélange opérationnel décrit dans l'EXEMPLE 101 à partir du composé obtenu en d) : F = 199°C

f) L'EXEMPLE 206 est obtenu de la même manière que pour l'EXEMPLE 112 à partir de l'acide obtenu en e) ; F = 86°C.

**EXEMPLE 207**

**1-[4-Chloro-3-[5-chloro-1-(4-chloro-2-méthoxybenzyl)-3-méthyl-2-oxoindolin-3-yl]benzoyl]-4-hydroxypipéridine**

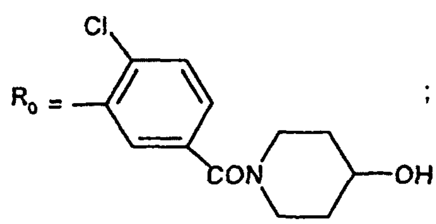

$R_1$ = -$CH_3$ ; $R_2$ = H ; $R_3$ = 4-Cl ; $R_4$ = 2-$OCH_3$ ; X = 5-Cl ; Y = H

Obtenu de la même manière que pour l'EXEMPLE 134 à partir de l'acide préparé en e) de l'EXEMPLE 206. F = 129°C (1$H_2O$).

**EXEMPLE 208**

**1-[4-Chloro-3-[5-chloro-1-(4-chloro-2-méthoxybenzyl)-3-méthyl-2-oxoindolin-3-yl]benzoyl]-(R)-2-méthoxycarbonylpipéridine**

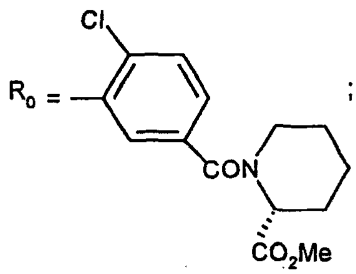

$R_1$ = $-CH_3$ ; $R_2$ = H ; $R_3$ = 4-Cl ; $R_4$ = 2-$OCH_3$ ; X = 5-Cl ; Y = H

Obtenu de la même manière que pour l'EXEMPLE 131 à partir de l'acide préparé en e) de l'EXEMPLE 206. F = 101°C.

**EXEMPLE 209**

**4-Chloro-3[5,7-dichloro-1-(2,4-diméthoxybenzyl)-3-méthyl-2-oxoindolin-3-yl]benzoate de méthyle**

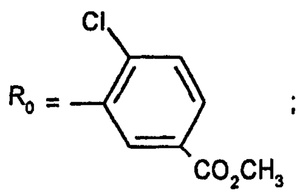

$R_1$ = $-CH_3$ ; $R_2$ = H ; $R_3$ = 4-$OCH_3$ ; $R_4$ = 2-$OCH_3$ ; X = 5-Cl ; Y = 7-Cl

a) **4-Chloro-3-[5,7-dichloro-1-(2,4-diméthoxybenzyl)3-hydroxy-2-oxoindolin-3-yl]benzoate de méthyle:**
A partir du *composé IV.3* et selon le mode opératoire décrit dans l'EXEMPLE 20, on isole de produit obtenu ; F = 225°C.
b) **4-Chloro-3-[1-(2,4-diméthoxybenzyl)-3,5,7-trichloro-2-oxoindolin-3-yl]benzoate de méthyle:**
Obtenu selon a) de la PREPARATION 13 à partir du produit décrit en a)
c) **4-Chloro-3-[5,7-dichloro-1-(2,4-diméthoxybenzyl)-3-H-2-oxoindolin-3-yl]benzoate de méthyle ;** *composé III.10* :
Obtenu selon b) de la PREPARATION 13 à partir du produit décrit en b). F = 173°C.
d) L'EXEMPLE 209 est obtenu selon le mode opératoire décrit dans l'EXEMPLE 37 à partir du produit *composé III.10* ; F = 166°C.

**EXEMPLE 210**

**3-(5-Amino-2-chlorophényl)-5,7-dichloro-1-(2,4-diméthoxybenzyl)-3-méthylindolin-2-one**

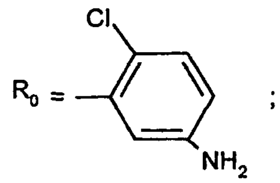

$R_1$ = -$CH_3$ ; $R_2$ = H ; $R_3$ = 4-$OCH_3$ ; $R_4$ = 2-$OCH_3$ ; X = 5-Cl ; Y = 7-Cl

a) **3-(2-Chloro-5-amino-phényl)5,7-dichloro-1-(2,4-diméthoxybenzyl)-3-hydroxy-indolin-2-one :** Obtenu à partir du *composé IV.3* et selon le mode opératoire décrit dans l'EXEMPLE 21. F = 124°C.
b) **3-(2-Chloro-5-aminophényl)-1-(2,4-diméthoxybenzyl)-3,5,7-trichloroindolin-2-one :** Obtenu selon a) de la PREPARATION 13 à partir du produit décrit en a).
c) **3-(2-Chloro-5-aminophényl)-1-(2,4-diméthoxybenzyl)-2,7-dichloro-3-H-indolin-2-one** ; *composé III.11 :* Obtenu selon b) de la PREPARATION 13 à partir du produit décrit en b). F = 118°C.
d) L'EXEMPLE 210 est obtenu selon le mode opératoire décrit dans l'EXEMPLE 37 à partir du produit ; *composé III.11 :* F = 112°C.

**EXEMPLE 211**

**1-[4-Chloro-3-[5-chloro-1-(2,4-diméthoxybenzyl)-3-méthyl-2-oxoindolin-3-yl]-benzoyl]-4,4-difluoropipéridine**

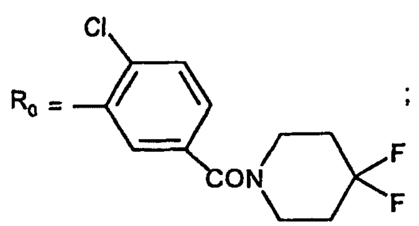

$R_1$ = -$CH_3$ ; $R_2$ = H ; $R_3$ = 4-$OCH_3$ ; $R_4$ = 2-$OCH_3$ ; X = 5-Cl ; Y = H

Obtenu selon b) de l'EXEMPLE 166 avec la 4,4-difluoropipéridine décrite dans Chem. Pharm. Bull., 1993, 41, *11*, 1971-1986.
F = 98,5°C (0,5 $H_2O$)

**EXEMPLE 212**

**5-Chloro-3-(2-chlorophényl)-1-(4-(2-butylamino)-2-méthoxybenzyl]-3-méthylindolin-2-one**

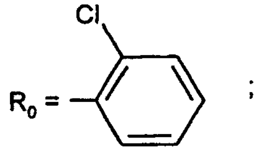

$R_1 = CH_3$ ; $R_2 = H$ ; $R_3 = 4\text{-}NH[CH(CH_3)(C_2H_5)]$ ; $R_4 = 2\text{-}OCH_3$ ; X = 5-Cl ; Y = H

Obtenu dans des conditions analogues à l'EXEMPLE 56. F = 158°C

## EXEMPLE 213

**5-Chloro-3-(2-chlorophényl)-1-(4-isobutylamino-2-méthoxybenzyl)-3-méthylindolin-2-one**

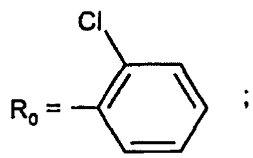

$R_1 = CH_3$ ; $R_2 = H$ ; $R_3 = 4\text{-}NHCH_2CH(CH_3)_2$ ; $R_4 = 2\text{-}OCH_3$ ; X = 5-Cl : Y = H

Obtenu dans des conditions analogues à l'EXEMPLE 55. F = 136°C

## EXEMPLE 214

**4-Chloro-3-[5-fluoro-1-(2,4-diméthoxybenzyl)-3-méthyl-2-oxoindolin-3-yl]-*N*,*N*-diéthylbenzamide**

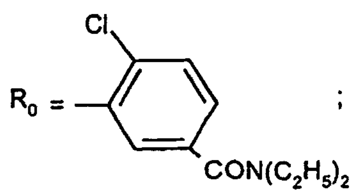

$R_1 = -CH_3$ ; $R_2 = H$ ; $R_3 = 4\text{-}OCH_3$ ; $R_4 = 2\text{-}OCH_3$ ; X = 5-F ; Y = H

a) **4-Chloro-3-[1-(2,4-diméthoxybenzyl)-5-fluoro-3-hydroxy-2-oxoindolin-3-yl]benzoate de méthyle :**
A partir du *composé IV.4* et selon le mode opératoire décrit dans l'EXEMPLE 20, on isole le produit attendu ; F = 188°C.

b) **4-Chloro-3-[3-chloro-1-(2,4-diméthoxybenzyl)-5-fluoro-2-oxoindolin-3-yl]benzoate de méthyle:**
Obtenu selon a) de la préparation 13 à partir du produit décrit en a)

c) **4-Chloro-3-[1-(2,4-diméthoxybenzyl)-5-fluoro-3-*H*-2-oxoindolin-3-yl]benzoate de méthyle** ; *composé III.12* :
Obtenu selon b) de la préparation 13 à partir du *composé III.12* ; F = 138°C :

d) **Acide 4-chloro-3-[1-(2,4-diméthoxybenzyl)-3-méthyl-5-fluoro-2-oxoindolin-3-yl]benzoïque :**
A partir du produit décrit en c) et selon le mode opératoire de l'EXEMPLE 37, on obtient l'ester méthylique du composé attendu que l'on engage directement en réaction de saponification dans les conditions de l'EXEMPLE 101 ; F = 89°C :

e) Le composé racémique de l'EXEMPLE 214 est obtenu dans les conditions de l'EXEMPLE 102 ; F = 79°C :

**EXEMPLE 215**

**4-Chloro-3-[1-(2,4-diméthoxybenzyl)-3-méthyl-2-oxoindolin-3-yl]-*N*,*N*-diéthylbenzamide**

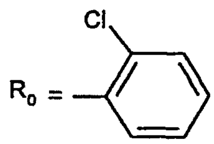

;

$R_1$ = -$CH_3$ ; $R_2$ = H ; $R_3$ = 4-$OCH_3$ ; $R_4$ = 2-$OCH_3$ ; X = H ; Y = H

a) **4-Chloro-3-[1-(2,4-diméthoxybenzyl)-3-hydroxy-2-oxoindolin-3-yl]benzoate de méthyle :**
A partir du *composé IV.5* et selon le mode opératoire décrit dans l'EXEMPLE 20, on isole le produit attendu ; F = 172°C.

b) **4-Chloro-3-[3-chloro-1-(2,4-diméthoxybenzyl)-2-oxoindolin-3-yl]benzoate de méthyle:**
Obtenu selon a) de la préparation 13 à partir du produit décrit en a)

c) **4-Chloro-3-[1-(2,4-diméthoxybenzyl)-3-*H*-2-oxoindolin-3-yl]benzoate de méthyle;** *composé III.13* :
Obtenu selon b) de la préparation 13 à partir du *composé III.13* ; F = 122°C :

d) **Acide 4-chloro-3-[1-(2,4-diméthoxybenzyl)-3-méthyl-2-oxoindolin-3-yl]benzoïque :**
A partir du produit décrit en c) et selon le mode opératoire de l'EXEMPLE 37, on obtient l'ester méthylique du composé attendu que l'on engage directement en réaction de saponification dans les conditions de l'EXEMPLE 101 ; F = 103°C.

e) Le composé racémique de l'EXEMPLE 215 est obtenu dans les conditions de l'EXEMPLE 102 ; F = 85°C.

## Revendications

**1.** Composé sous forme d'énantiomère pur ou de mélange d'énantiomères de formule :

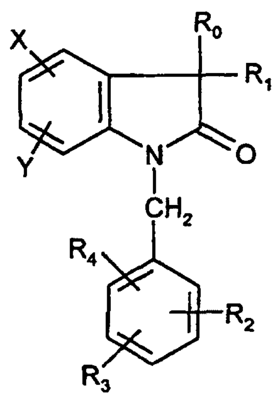

(I)

dans laquelle :

- $R_0$ représente un groupe choisi parmi :
(i) :

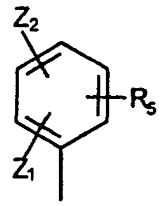

dans lequel :

- $Z_1$ représente un atome de chlore, brome, iode ou fluor, un groupe $(C_1-C_4)$alkyle, $(C_1-C_4)$alcoxy ou trifluorométhyle ;
- $Z_2$ représente un atome d'hydrogène, de chlore, brome, iode ou fluor, un groupe $(C_1-C_4)$alkyle, un groupe $(C_3-C_5)$cycloalkyle, $(C_1-C_4)$alcoxy, un groupe $(C_3-C_5)$cycloalcoxy, ou $(C_1-C_4)$polyfluoroalkyle ;
- $R_5$ représente $T_1W$ dans lequel Ti représente $-(CH_2)_m$, m pouvant être égal à 0 ou 1, W réprésente un atome d'hydrogène, un groupe hydroxycarbonyle (ou carboxyle), $(C_{1-}C_4)$alcoxycarbonyle, 1,3-dioxolan-2-yle, 1,3-dioxan-2-yle,

  ou bien W représente un groupe $-NR_6R_7$ dans lequel $R_6$ et $R_7$ représentent indépendamment l'un de l'autre un atome d'hydrogène, un groupe $(C_1-C_4)$alkyle, un groupe $(C_1-C_4)$alkylsulfonyle ou phénylsulfonyle dans lequel le groupe phényle peut être mono, di ou trisubstitué par $Z_5$ ; ou bien $R_6$ et $R_7$ forment avec l'atome d'azote auquel ils sont liés un groupe morpholinyle éventuellement substitué par un groupe $(C_1-C_4)$alkyle ou un oxo, ou bien $R_6$ et $R_7$ forment avec l'atome d'azote auquel ils sont liés un groupe pipérazinyle éventuellement substitué en position 4 par un substituant $Z_3$ ; ou bien $R_6$ et $R_7$ forment avec l'atome d'azote auquel ils sont liés un groupe pyrrolidinyle ou pipéridinyle, lesdits groupes pyrrolidinyle et pipéridinyle étant éventuellement substitués par $Z_4$ ;

  ou bien W représente un groupe $-NR_8COR_9$ dans lequel $R_8$ représente un atome d'hydrogène ou un groupe $(C_1-C_4)$alkyle et $R_9$ représente un atome d'hydrogène, un groupe $(C_1-C_4)$alkyle, benzyle, pyridyle, phényle, ledit groupe phényle pouvant être mono, di ou trisubstitué par $Z_5$ ; ou bien $R_9$ représente un groupe $-NR_{10}R_{11}$ dans lequel $R_{10}$ et $R_{11}$ représentent indépendamment l'un de l'autre un atome d'hydrogène ou un $(C_{1-}C_4)$alkyle, ou bien $R_{10}$ et $R_{11}$ forment avec l'atome d'azote auquel ils sont liés un groupe pyrrolidinyle, pipéridinyle, ou morpholinyle éventuellement substitué par un groupe $(C_1-C_4)$alkyle, ou bien $R_9$ représente un groupe pyrrolidin-2-yle ou 3-yle, pipéridin-2-yle, 3-yle ou 4-yle, lesdits groupes pyrrolidinyle et pipéridinyle étant éventuellement substitués par $Z_7$ ; ou bien $R_9$ représente un groupe $-T_2-R_{12}$ ou - $T_2-COR_{12}$ dans lequel $T_2$ représente $-(CH_2)_n-$, n pouvant être égal à 1, 2, 3 et 4, et $R_{12}$ représente un groupe $(C_{1-}C_4)$alcoxy ou $-NR_{10}R_{11}$, $R_{10}$ et $R_{11}$ étant tels que définis ci-dessus ;

ou bien W représente un groupe $-CONR_{13}R_{14}$ dans lequel $R_{13}$ représente un atome d'hydrogène, un groupe $(C_{1-}C_4)$alkyle, $(C_3-C_7)$cycloalkyle, un mono- ou polyfluoro$(C_1-C_4)$alkyle, et $R_{14}$ représente un atome d'hydrogène, un groupe $(C_{1-}C_4)$alkyle, phényle éventuellement substitué par $Z_5$, un groupe $-T_4-R_{15}$ dans lequel $T_4$ représente $-(CH_2)_q$ avec q égal à 1, 2, 3 ou 4 et $R_{15}$ représente un groupe hydroxyle, un groupe $(C_{1-}C_4)$alcoxy, $(C_{1-}C_4)$alcoxycarbonyle, $(C_1-C_4)$alcoxycarbonylamino, phényle éventuellement mono-, disubstitué par $Z_5$, un pyrid-2-yle, 3-yle ou 4-yle, un groupe $-NR_{16}R_{17}$ dans lequel $R_{16}$ et $R_{17}$ représentent indépendamment l'un de l'autre un atome d'hydrogène, ou un $(C_{1-}C_4)$alkyle, ou bien $R_{16}$ et $R_{17}$ forment avec l'atome d'azote auquel ils sont liés un groupe morpholinyle éventuellement mono ou disubstitué par un groupe $(C_1-C_4)$alkyle, ou bien $R_{16}$ et $R_{17}$ forment avec l'atome d'azote auquel ils sont liés un groupe pipérazinyle éventuellement substitué en position 4 par un substituant $Z_3$, ou bien $R_{16}$ et $R_{17}$ forment avec l'atome d'azote auquel ils sont liés un groupe pyrrolidinyle ou pipéridinyle, lesdits groupes pyrrolidinyle et pipéridinyle étant éventuellement substitués par $Z_5$ étant entendu que lorsque q = 1, $R_{15}$ est différent de hydroxyle, $(C_1-C_4)$alcoxy, $(C_1-C_4)$alcoxycarbonylamino, $-NR_{16}R_{17}$ ; ou bien $R_{13}$ et $R_{14}$ forment avec l'atome d'azote auquel ils sont liés un groupe morpholinyle éventuellement mono ou disubstitué par un groupe $(C_1-C_4)$alkyle, pipérazinyle éventuellement substitué en position 4 par un substituant $Z_3$ ; ou bien $R_{13}$ et $R_{14}$ forment avec l'atome d'azote auquel ils sont liés un groupe azétidinyle, pyrrolidinyle ou pipéridinyle, hexahydroazépinyle, lesdits groupes pyrrolidinyle, pipéridinyle et hexahydroazépinyle étant éventuellement mono ou disubstitués par $Z_8$ ;

ou bien W représente un groupe $OR_{18}$ dans lequel $R_{18}$ représente un atome d'hydrogène, un groupe $(C_1-C_4)$ alkyle, $(C_1-C_4)$alcoxy$(C_1-C_4)$alkyle ou $-T_3-R_{19}$ dans lequel $T_3$ représente $-(CH_2)_p-$, p pouvant être égal à 2, 3 et $R_{19}$ est choisi parmi les groupes hydroxyle, triphénytméthoxy, $-NR_{20}R_{21}$ dans lequel $R_{20}$ représente un atome d'hydrogène ou un groupe $(C_{1-}C_4)$alkyle et $R_{21}$ représente un atome d'hydrogène, un groupe $(C_{1-}C_4)$alkyle, tétrahydrofuranylméthyle ou tétrahydropyranylméthyle, ou bien $R_{20}$ et $R_{21}$ forment avec l'atome d'azote auquel ils sont liés un groupe morpholinyle éventuellement mono ou disubstitué par un groupe $(C_1-C_4)$alkyle, pipérazinyle éven-

tuellement substitué en position 4 par un substituant $Z_3$ ou bien $R_{20}$ et $R_{21}$ forment avec l'atome d'azote auquel ils sont liés un groupe pyrrolidinyle ou pipéridinyle, lesdits groupes pyrrolidinyle et pipéridinyle étant éventuellement substitués par $Z_5$ ;

- $Z_3$ représente un groupe $(C_1\text{-}C_4)$alkyle, pyridyle ou phényle, un groupe $(C_1\text{-}C_4)$alkylcarbonyle, $(C_1\text{-}C_4)$ alcoxycarbonyle ;
- $Z_4$ représente un oxo, un atome de fluor, un hydroxyle, un $(C_1\text{-}C_4)$alkyle, un benzyle, un amino, un $(C_1\text{-}C_4)$ alkylamino, un di$(C_1\text{-}C_4)$alkylamino, un $(C_1\text{-}C_4)$ alcoxy, un $(C_1\text{-}C_4)$alcoxycarbonyle, un $(C_1\text{-}C_4)$ alcoxycarbonylamino ;
- $Z_5$ représente un atome de chlore, brome, iode ou fluor, un groupe hydroxyle, un groupe $(C_1\text{-}C_4)$alkyle, ou $(C_1\text{-}C_4)$alcoxy ;
- $Z_7$ représente un atome de fluor, un groupe hydroxyle, un groupe hydroxy$(C_1\text{-}C_4)$alkyle, un $(C_1\text{-}C_4)$alkyle, un $(C_1\text{-}C_4)$alcoxy, un $(C_1\text{-}C_4)$alkylcarbonyle ;
- $Z_8$ représente un atome de fluor, un groupe hydroxyle, $(C_1\text{-}C_4)$alkyle, $(C_3\text{-}C_6)$cycloalkyle, benzyle, amino, $(C_1\text{-}C_4)$alkylamino, di$(C_1\text{-}C_4)$alkylamino, $(C_1\text{-}C_4)$alcoxycarbonyle, $(C_1\text{-}C_4)$alcoxycarbonylamino, $(C_3\text{-}C_6)$cycloalcoxy, hydroxycarbonyle, hydroxy$(C_1\text{-}C_4)$alkyle ou $(C_1\text{-}C_4)$alcoxy$(C_1\text{-}C_4)$alkyle, $(C_1\text{-}C_4)$alcoxy, -$CONR_{23}R_{24}$ dans lequel $R_{23}$ et $R_{24}$ représentent indépendamment l'un de l'autre, un atome d'hydrogène, un $(C_1\text{-}C_4)$alkyle, un mono ou polyfluoro$(C_1\text{-}C_4)$alkyle ou bien $R_{23}$ et $R_{24}$ forment avec l'atome d'azote auquel ils sont liés un groupe pyrrolidinyle ou pipéridinyle, lesdits groupes pyrrolidinyle ou pipéridinyle étant éventuellement substitués par $Z_3$, un difluorométhylidène ;

  (ii) :

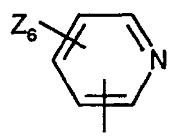

- $Z_6$ représente un atome de chlore ou un groupe $(C_1\text{-}C_4)$alkyle ou $(C_1\text{-}C_4)$alcoxy ;
- $R_1$ représente un groupe $(C_1\text{-}C_4)$alkyle comportant éventuellement une double ou une triple liaison ; $(C_1\text{-}C_4)$ alcoxycarbonyle ; phényloxycarbonyle ou un groupe $T_1\text{-}R_{22}$ dans lequel $T_1$ est tel que défini ci-dessus et $R_{22}$ représente un groupe hydroxyle ou $(C_1\text{-}C_4)$alcoxy ;
- $R_2$ et $R_4$ représentent indépendamment l'un de l'autre, un atome d'hydrogène, de chlore ou de fluor, un groupe $(C_1\text{-}C_4)$alkyle ou $(C_1\text{-}C_4)$alcoxy ;
- $R_3$ représente un atome de chlore ou de fluor, un groupe $(C_1\text{-}C_4)$alkyle ; $(C_1\text{-}C_4)$alcoxy ; hydroxyle; un groupe $(C_1\text{-}C_4)$carbamoyle ; un $(C_1\text{-}C_4)$alkylcarbonylamino ; nitro ; cyano; trifluorométhyle ; amino ; $(C_3\text{-}C_6)$ cycloalkylamino ; $(C_1\text{-}C_4)$alkylamino ; di$(C_1\text{-}C_4)$alkylamino ; tri$(C_1\text{-}C_4)$alkylammonium, $A^-$, $A^-$ étant un anion ; pyrrolidin-1-yle ; pipéridin-1-yle; pipérazin-1-yle ; morpholin-4-yle ou hexahydroazépin-1-yle ;
- X et Y représentent indépendamment l'un de l'autre un atome d'hydrogène, de chlore, brome, iode ou fluor ou un groupe $(C_1\text{-}C_4)$alcoxy ou trifluorométhoxy ;

ainsi que leurs sels pharmaceutiquement acceptables, solvats et hydrates.

**2.** Composés selon la revendication 1 sous forme d'énantiomère pur ou de mélange d'énantiomères de formule :

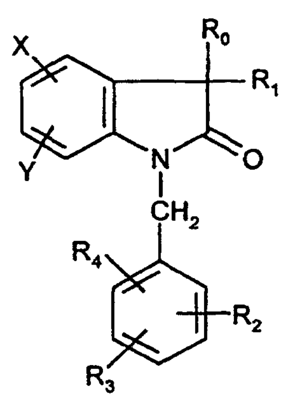

(I)

dans laqueile :

$R_0$ représente
(i) :

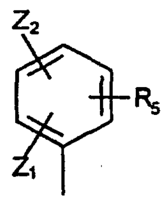

$Z_1$, $Z_2$, $R_1$, $R_2$, $R_3$, $R_4$, $R_5$, Y, X sont tels que définis pour (I) et leurs sels pharmaceutiquement acceptables, solvats et hydrates.

**3.** Composés selon la revendication 2 de formule:

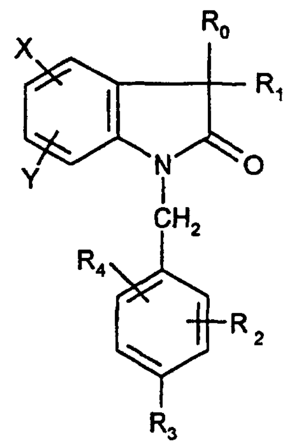

(Ia)

dans laquelle $R_1$ représente un groupe méthyle ou hydroxyle et $R_0$, $R_2$, $R_3$, $R_4$, X et Y sont tels que définis pour (I) ; sous forme d'énantiomère pur ou de mélange d'énantiomères, ainsi que leurs sels pharmaceutiquement acceptables solvats et hydrates.

**4.** Composés selon la revendication 3 de formule:

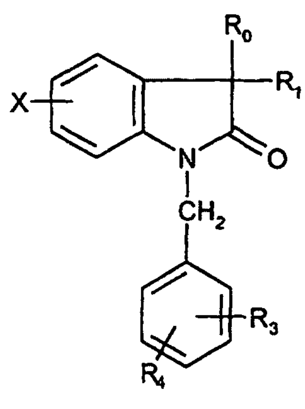

(Ib)

dans laquelle $R_1$ représente un groupe méthyle ou hydroxyle et $R_0$, $R_3$, $R_4$ et X sont tels que définis pour (I) ; sous forme d'énantiomère pur ou de mélange d'énantiomères, ainsi que leurs sels pharmaceutiquement acceptables solvats et hydrates.

**5.** Composés selon la revendication 4 de formule :

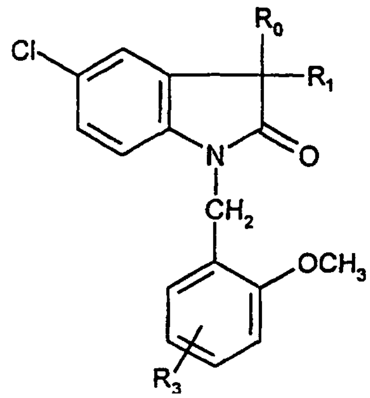

(Ic)

dans laquelle $R_1$ représente un groupe méthyle ou hydroxyle et $R_0$ et $R_3$ sont tels que définis pour (I) ; sous forme d'énantiomère pur ou de mélange d'énantiomères, ainsi que leurs sels pharmaceutiquement acceptables solvats et hydrates.

**6.** Composés selon la revendication 5 de formule:

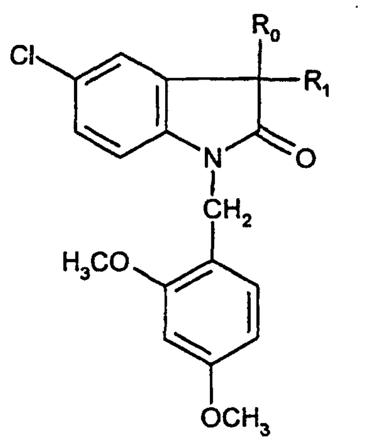

(Id)

dans laquelle $R_1$ représente un groupe méthyle ou hydroxyle et $R_0$ est tel que défini pour (I) ; sous forme d'énantiomère pur ou de mélange d'énantiomères, ainsi que leurs sels pharmaceutiquement acceptables solvats et hydrates.

**7.** Composés selon l'une quelconque des revendications 1 à 6 dans lesquelles $R_0$ représente le groupe :

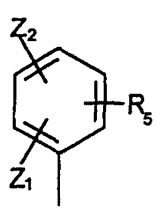

$Z_1$, $Z_2$ et $R_5$ étant tels que définis pour (I).

**8.** Composé selon la revendication 7 dans lesquels $R_0$ représente le groupe :

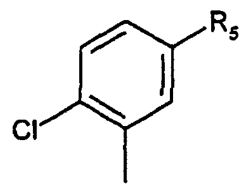

$R_5$ étant tel que défini pour (I).

**9.** Composés selon l'une quelconque des revendications 1 à 8 dans lesquels $R_1$ représente un groupe méthyle.

**10.** Composés selon la revendication 1 choisis parmi :

5-Chloro-3-(2-chlorophényl)-1-(2,4-diméthoxybenzyl)-3-méthylindolin-2-one ;
5-Chloro-3-(2-chlorophényl)-1-[4-(isopropylamino)-2-méthoxybenzyl]-3-méthylindolin-2-one ;
*N*-{4-Chloro-3-[5-chloro-1-(2,4-diméthoxybenzyl)-3-méthyl-2-oxoindolin-3-yl]phényl} acétamide ;
*N*-{4-Chloro-3-[5-chloro-1-(2,4-diméthoxybenzyl)-3-méthyl-2-oxoindolin-3-yl]phényl}-3-méthylbutanamide ;
*N*-{4-Chloro-3-[5-chloro-1-(2,4-diméthoxybenzyl)-3-méthyl-2-oxoindolin-3-yl]phényl} benzamide ;
*N*-{4-Chloro-3-[5-chloro-1-(2,4-diméthoxybenzyl)-3-méthyl-2-oxoindolin-3-yl]phényl} nicotinamide ;
*N*-{4-Chloro-3-[5-chloro-1-(2,4-diméthoxybenzyl)-3-méthyl-2-oxoindolin-3-yl]phényl}-2-méthoxyacétamide ;
3-{4-Chloro-3-[5-chloro-1-(2,4-diméthoxybenzyl)-3-méthyl-2-oxoindolin-3-yl]anilino}-3-oxopropanoate de méthyle ;
*N*-{4-Chloro-3-[5-chloro-1-(2,4-diméthoxybenzyl)-3-méthyl-2-oxoindolin-3-yl]phényl}-3-méthoxypropanamide ;
*N*-{4-Chloro-3-[5-chloro-1-(2,4-diméthoxybenzyl)-3-méthyl-2-oxoindolin-3-yl]phényl}-*N*-méthylacétamide ;
*N*-{4-Chloro-3-[5-chloro-1-(2,4-diméthoxybenzyl)-3-méthyl-2-oxoindolin-3-yl]phényl}-*N*-méthylméthanesulfonamide ;
4-Chloro-3-[5-chloro-1-(2,4-diméthoxybenzyl)-3-méthyl-2-oxoindolin-3-yl]-*N*,*N*-diéthyl benzamide ;
4-Chloro-3-[5-chloro-1-(2,4-diméthoxybenzyl)-3-méthyl-2-oxoindolin-3-yl]-*N*,*N*-diméthyl benzamide ;
5-Chloro-3-[2-chloro-5-(1-pipéridinylcarbonyl)phényl]-1-(2,4-diméthoxybenzyl)-3-méthyl indolin-2-one ;
4-Chloro-3-[5-chloro-1-(2,4-diméthoxybenzyl)-3-méthyl-2-oxoindolin-3-yl]-*N*-éthyl benzamide ;
5-Chloro-3-(2-chloro-5-{[2-(méthoxyméthyl)-1-pyrrolidinyl]carbonyl}phényl)-1-(2,4-diméthoxybenzyl)-3-méthylindolin-2-one ;
5-Chloro-3-{2-chloro-5-[(2-méthyl-1-pipéridinyl)carbonyl]phényl}-1-(2,4-diméthoxybenzyl)-3-méthylindolin-2-one ;
4-Chloro-3-[5-chloro-1-(2,4-diméthoxybenzyl)-3-méthyl-2-oxoindolin-3-yl]-*N*-éthyl-*N*-méthylbenzamide ;
1-{4-Chloro-3-[5-chloro-1-(2,4-diméthoxybenzyl)-3-méthyl-2-oxoindolin-3-yl]benzoyl}-2-pipéridinecarboxylate de méthyle ;
5-Chloro-3-{2-chloro-5-[(4-hydroxy-1-pipéridinyl)carbonyl]phényl}-1-(2,4-diméthoxybenzyl) -3-méthylindolin-2-one ;
5-Chloro-3-{2-chloro-5-[(2-méthoxyéthoxy)méthyl]phényl}-1-(2,4-diméthoxybenzyl)-3-méthylindolin-2-one ;
5-Chloro-3-[2-chloro-5-(4-morpholinylméthyl)phényl]-1-(2,4-diméthoxybenzyl)-3-méthyl indolin-2-one ;
5-Chloro-3-(2-chloro-5-{[2-(4-morpholinyl)éthoxy]méthyl}phényl)-1-(2,4-diméthoxybenzyl)-3-méthylindolin-2-one ;
1-[4-Chloro-3-[5-chloro-1-(2,4-diméthoxybenzyl)-3-méthyl-2-oxoindolin-3-yl]benzoyl}-3-hydroxypipéridine ;
1-[4-Chloro-3-[5-chloro-1-(2,4-diméthoxybenzyl)-3-méthyl-2-oxoindolin-3-yl]benzoyl]-(R)-3-hydroxypipéridine ;
1-[4-Chloro-3-[5-chloro-1-(2,4-diméthoxybenzyl)-3-méthyl-2-oxoindolin-3-yl]benzoyl]-4-méthoxypipéridine ;
1-[4-Chloro-3-[5-chloro-1-(2,4-diméthoxybenzyl)-3-méthyl-2-oxoindolin-3-yl]benzoyl]-4-éthoxypipéridine ;
1-[4-Chloro-3-[5-chloro-1-(2,4-diméthoxybenzyl)-3-méthyl-2-oxoindolin-3-yl]benzoyl]-(R,S)-2,6-diméthylpipéridine ;
1-[4-Chloro-3-[5-chloro-1-(2,4-diméthoxybenzyl)-3-méthyl-2-oxoindolin-3-yl]benzoyl]-(R)-2-éthoxycarbonylpipéridine ;
1-[4-Chloro-3-[5-chloro-1-(2,4-diméthoxybenzyl)-3-méthyl-2-oxoindolin-3-yl]benzoyl]-(R)-2-*N*,*N*-diméthylaminocarbonylpipéridine ;
1-[4-Chloro-3-[5-chloro-1-(2,4-diméthoxybenzyl)-3-méthyl-2-oxoindolin-3-yl]benzoyl]-(R)-2-(*N*-méthyl-*N*-2,2,2-trifluoroéthylaminocarbonyl)pipéridine ;

1-[4-Chloro-3-[5-chloro-1-(2,4-diméthoxybenzyl)-3-méthyl-2-oxoindolin-3-yl]benzoyl]-(R)-2-pyrrolidinocarbonylpipéridine ;
1-[4-Chloro-3-[5-chloro-1-(2,4-diméthoxybenzyl)-3-méthyl-2-oxoindolin-3-yl]benzoyl]-(S)-2-méthylpipéridine ;
4-Chloro-3-[5-chloro-1-(2,4-diméthoxybenzyl)-3-méthyl-2-oxoindolin-3-yl]-*N*-éthyl-*N*-(2-phényléthyl) benzamide ;
4-Chloro-3-[5-chloro-1-(2,4-diméthoxybenzyl)-3-méthyl-2-oxoindolin-3-yl]-*N*-éthyl-*N*-(4-pyridylméthyl)benzamide, chlorhydrate ;
4-Chloro-3-[5-chloro-1-(2,4-diméthoxybenzyl)-3-méthyl-2-oxoindolin-3-yl]-*N*-éthyl-*N*-(3-pyridylméthyl) benzamide ;
4-Chloro-3-[5-chloro-1-(2,4-diméthoxybenzyl)-3-méthyl-2-oxoindolin-3-yl]-*N*-éthyl-*N*-(2-pyridylméthyl) benzamide ;
4-Chloro-3-[5-chloro-1-(2,4-diméthoxybenzyl)-3-méthyl-2-oxoindolin-3-yl]-*N*-éthyl-*N*-(2-méthoxyéthyl) benzamide ;
4-Chloro-3-[5-chloro-1-(2,4-diméthoxybenzyl)-3-méthyl-2-oxoindolin-3-yl]-*N*-éthyl-*N*-(2-diméthylaminoéthyl) benzamide, chlorhydrate ;
4-Chloro-3-[5-chloro-1-(2,4-diméthoxybenzyl)-3-méthyl-2-oxoindolin-3-yl]-*N*-éthyl-*N*-(2-morpholinoéthyl) benzamide ;
4-Chloro-3-[5-chloro-1-(2,4-diméthoxybenzyl)-3-méthyl-2-oxoindolin-3-yl]-*N*-éthyl-*N*-(2-pyrrolidinoéthyl)benzamide, chlorhydrate ;
4-Chloro-3-[5-chloro-1-(2,4-diméthoxybenzyl)-3-méthyl-2-oxoindolin-3-yl]-*N*-éthyl-*N*-(2-pipéridinoéthyl)benzamide, chlorhydrate ;
4-Chloro-3-[5-chloro-1-(2,4-diméthoxybenzyl)-3-méthyl-2-oxoindolin-3-yl]-*N*-éthyl-*N*-(2-hydroxyéthyl) benzamide ;
4-Chloro-3-[5-chloro-1-(2,4-diméthoxybenzyl)-3-méthyl-2-oxoindolin-3-yl]-*N*-éthyl-*N*-[2-(pyridin-4-yl)éthyl] benzamide, chlorhydrate ;
4-Chloro-3-[5-chloro-1-(2,4-diméthoxybenzyl)-3-méthyl-2-oxoindolin-3-yl]-*N*-éthyl-*N*-(2,2,2-trifluoroéthyl) benzamide ;
4-Chloro-3-[5-chloro-1-(2,4-diméthoxybenzyl)-3-méthyl-2-oxoindolin-3-yl]-*N*-méthyl-*N*-(2,2,2-trifluoroéthyl) benzamide ;
4-Chloro-3-[5-chloro-1-(2,4-diméthoxybenzyl)-3-méthyl-2-oxoindolin-3-yl]-*N*-éthyl-*N*-isopropylbenzamide ;
4-Chloro-3-[5-chloro-1-(2,4-diméthoxybenzyl)-3-méthyl-2-oxoindolin-3-yl]-*N*-(2-diméthylaminoéthyl) -*N*-(2,2,2-trifluoroéthyl)benzamide, chlorhydrate ;
4-Chloro-3-[5-chloro-1-(2,4-diméthoxybenzyl)-3-méthyl-2-oxoindolin-3-yl]-*N*-cyclohexylbenzamide ;
4-Chloro-3-[5-chloro-1-(2,4-diméthoxybenzyl)-3-méthyl-2-oxoindolin-3-yl]-*N*-éthyl-*N*-[3-(pyridin-4-yl)propyl] benzamide ;

sous forme d'énantiomère pur ou de mélange d'énantiomères, ainsi que leurs sels pharmaceutiquement acceptables, solvats et hydrates.

**11.** Procédé de préparation des composés de formule (I) selon la revendication 1 **caractérisé en ce que** :

a) on fait réagir en présence d'une base un composé de formule :

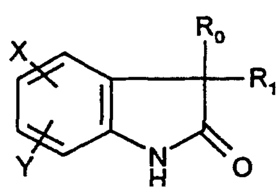

(II)

dans laquelle X, Y, $R_0$ et $R_1$ sont tels que définis pour (I), avec un halogènure de formule.

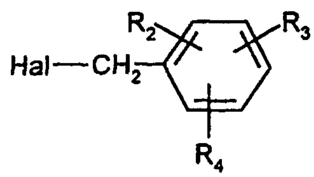

(1)

dans laquelle Hal représente un atome d'halogène et $R_2$, $R_3$, $R_4$ sont tels que définis pour (I) ;

b) ou bien, lorsque $R_1$ représente un groupe électrophile, on transforme le composé de formule :

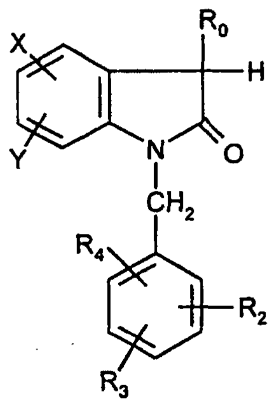

(III)

dans laquelle $R_0$, $R_2$, $R_3$, $R_4$, X et Y sont tels que définis pour (I), par l'action d'un dérivé $R_1$-Z, dans lequel Z représente un groupe partant, en présence d'une base ;

c) ou bien lorsque $R_1$ = OH, on met en réaction un dérivé de l'isatine de formule:

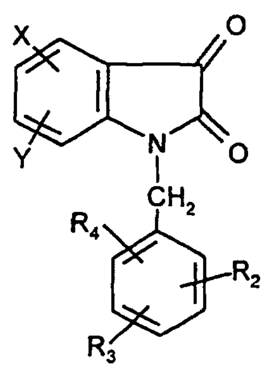

(IV)

dans laquelle $R_2$, $R_3$, $R_4$, X et Y sont tels que définis pour (I). avec un dérivé organométallique $R_0$-M ou $R_0$MgHal, $R_0$ étant tel que défini pour (I), M étant un atome de métal et Hal un atome de brome ou d'iode ;

d) ou bien on soumet le composé de formule :

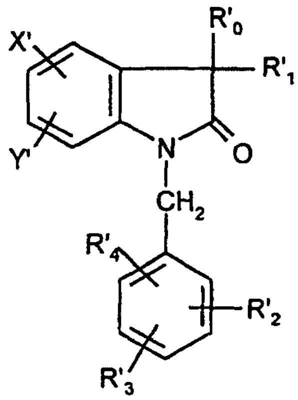

(Ip)

dans laquelle R'$_0$, R'$_1$, R'$_2$, R'$_3$, R'$_4$, X' et Y' représentent respectivement soit $R_0$, $R_1$, $R_2$, $R_3$, $R_4$, X et Y tels que définis pour (I), soit un groupe précurseur de $R_0$, $R_1$, $R_2$, $R_3$, $R_4$, X et Y, à un traitement ultérieur pour transformer l'un quelconque des groupes R'$_0$, R'$_1$, R'$_2$, R'$_3$, R'$_4$, X' ou Y' en respectivement, $R_0$, $R_1$, $R_2$, $R_3$, $R_4$, X ou Y tels que définis pour (I).

**12.** Composition pharmaceutique **caractérisée en ce qu'**elle contient à titre de principe actif un composé selon l'une quelconque des revendications 1 à 10.

**13.** Composition pharmaceutique selon la revendication 12 **caractérisée en ce qu'**elle contient un antagoniste des récepteurs de l'ocytocine selon l'une quelconque des revendications 1 à 10, en association avec un antagoniste des récepteurs $V_{1a}$ de la vasopressine.

**14.** Produit contenant un antagoniste des récepteurs de l'ocytocine selon l'une quelconque des revendications 1 à 10, et un antagoniste des récepteurs $V_{1a}$ de la vasopressine pour une utilisation simultanée, séparée ou étalée dans le temps dans le traitement des dysménorrhées, de l'endométriose, le contrôle du travail prématuré et pour contrôler le travail préparatoire en vue d'un accouchement par césarienne.

**15.** Utilisation d'un composé selon l'une quelconque des revendications 1 à 10 pour la préparation d'un médicament destiné à traiter les désordres ocytocine-dépendants.

**16.** Utilisation d'un composé selon l'une quelconque des revendications 1 à 10 pour la préparation d'un médicament tocolytique ou relaxant utérin.

**17.** Utilisation d'un composé selon l'une quelconque des revendications 1 à 10 pour la préparation de médicaments destinés à favoriser la cicatrisation, traiter l'analgésie, l'anxiolyse, la dépression , la schizophrénie, l'autisme, le syndrome obsessionnel et compulsif, améliorer le comportement maternel et social, faciliter la reconnaissance et l'acceptation de la mère par l'enfant, traiter les troubles de la mémoire, réguler la prise de nourriture et de boisson, la dépendance aux drogues, le sevrage et la motivation sexuelle, traiter les désordres de la sphère urogénitale dans les domaines obstétriques et gynécologiques, contrôler les contractions de l'utérus avant que la grossesse soit arrivée à terme, contrôler le travail prénatal, traiter les dysménorrhées, contrôler le travail préparatoire en vue d'un accouchement par césarienne, résoudre les problèmes de stérilité, fertilité, contrôler les naissances, contrôler l'oestrus, l'arrêt de l'allaitement, le sevrage, le transfert et l'implantation d'embryons, traiter l'endométriose, l'incontinence urinaire à l'effort ou d'urgence, l'hypertrophie bénigne de la prostate, les dysfonctions érectiles, l'hypertension, l'hyponatrémie, l'insuffisance cardiaque, l'arthérosclérose, l'angiogénèse, réguler le stockage des graisses par l'adipocyte et traiter les cancers mammaires ou prostatiques.

**18.** Médicament **caractérisé en ce qu'**il consiste en un composé selon l'une quelconque des revendications 1 à 10.

**Patentansprüche**

**1.** Verbindung in Form des reinen Enantiomeren oder einer Mischung der Enantiomeren der Formel:

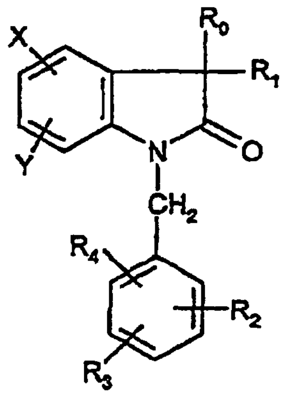

(I)

in der:

- $R_0$ eine Gruppe bedeutet ausgewählt aus:
  (i):

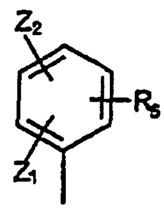

in der:
- $Z_1$ ein Chlor-, Brom-, Iod- oder Fluor-Atom, eine $(C_1-C_4)$-Alkyl-, $(C_1-C_4)$-Alkoxyoder Trifluormethyl-Gruppe bedeutet;
- $Z_2$ ein Wasserstoff-, Chlor-, Brom-, Iod- oder Fluor-Atom, eine $(C_1-C_4)$-Alkylgruppe, eine $(C_3-C_5)$-Cycloalkylgruppe, eine $(C_1-C_4)$-Alkoxygruppe, eine $(C_3-C_5)$-Cycloalkoxygruppe oder $(C_1-C_4)$-Polyfluoralkylgruppe darstellt;
- $R_5$ $T_1W$ bedeutet, worin $T_1$ $-(CH_2)_m$ darstellt, worin m den Wert 0 oder 1 besitzen kann und W ein Wasserstoffatom, eine Hydroxycarbonyl- (oder Carboxyl-), $(C_1-C_4)$-Alkoxycarbonyl-, 1,3-Dioxolan-2-yl- oder 1,3-Dioxan-2-yl-Gruppe bedeutet,

  oder W eine Gruppe $-NR_6R_7$ darstellt, in der $R_6$ und $R_7$ unabhängig voneinander ein Wasserstoffatom, eine $(C_1-C_4)$-Alkylgruppe, eine $(C_1-C_4)$-Alkylsulfonylgruppe oder Phenylsulfonylgruppe, in der die Phenylgruppe durch $Z_5$ einfach, zweifach oder dreifach substituiert sein kann, bedeuten; oder $R_6$ und $R_7$ zusammen mit dem Stickstoffatom, an das sie gebunden sind, eine Morpholinylgruppe bilden, die gegebenenfalls durch eine $(C_1-C_4)$-Alkylgruppe oder eine Oxogruppe substituiert ist, oder $R_6$ und $R_7$ zusammen mit dem Stickstoffatom, an das sie gebunden sind, eine Piperazinylgruppe bilden, die gegebenenfalls in der 4-Stellung durch einen Substituenten $Z_3$ substituiert ist; oder $R_6$ und $R_7$ zusammen mit dem Stickstoffatom, an das sie gebunden sind, eine Pyrrolidinyl- oder Piperidinyl-Gruppe bilden, wobei die Pyrrolidinyl- und Piperidinyl-Gruppen gegebenenfalls durch $Z_4$ substituiert sind;

  oder W eine Gruppe $-NR_8COR_9$ darstellt, in der $R_8$ ein Wasserstoffatom oder eine $(C_1-C_4)$-Alkylgruppe bedeutet und $R_9$ ein Wasserstoffatom, eine $(C_1-C_4)$-Alkyl-, Benzyl-, Pyridyl- oder Phenyl-Gruppe, wobei die Phenylgruppe durch $Z_5$ einfach, zweifach oder dreifach substituiert sein kann, bedeutet; oder $R_9$ eine Gruppe $-NR_{10}R_{11}$ darstellt, in der $R_{10}$ und $R_{11}$ unabhängig voneinander ein Wasserstoffatom oder eine $(C_1-C_4)$-Alkylgruppe bedeuten, oder $R_{10}$ und $R_{11}$ zusammen mit dem Stickstoffatom, an das sie gebunden sind, eine Pyrrolidinyl-, Piperidinyl- oder Morpholinyl-Gruppe bilden, die gegebenenfalls durch eine $(C_1-C_4)$-Alkylgruppe substituiert ist, oder $R_9$ eine Pyrrolidin-2-yl- oder -3-yl-, Piperidin-2-yl-, -3-yl- oder -4-yl-Gruppe bedeutet, wobei die Pyrrolidinyl- und Piperidinyl-Gruppen gegebenenfalls durch $Z_7$ substituiert sind; oder $R_9$ eine Gruppe $-T_2-R_{12}$ oder $-T_2-COR_{12}$ darstellt, in der $T_2$ $-(CH_2)_n-$ bedeutet, worin n 1, 2, 3 und 4 bedeuten kann und $R_{12}$ eine $(C_1-C_4)$-Alkoxygruppe oder $-NR_{10}R_{11}$ darstellt, worin $R_{10}$ und $R_{11}$ die oben angegebenen Bedeutungen besitzen;

oder W eine Gruppe $-CONR_{13}R_{14}$ darstellt, worin $R_{13}$ ein Wasserstoffatom, eine $(C_1-C_4)$-Alkyl-, $(C_3-C_7)$-Cycloalkyl-, Mono- oder Polyfluor-$(C_1-C_4)$-alkyl-Gruppe bedeutet und $R_{14}$ ein Wasserstoffatom, eine $(C_1-C_4)$-Alkylgruppe, eine Phenylgruppe, die gegebenenfalls durch $Z_5$ substituiert ist, eine Gruppe $-T_4-R_{15}$, in der $T_4$ $-(CH_2)_q$, worin q 1, 2, 3, oder 4 darstellt, und $R_{15}$ eine Hydroxygruppe, eine $(C_1-C_4)$-Alkoxy-, $(C_1-C_4)$-Alkoxycarbonyl-, $(C_1-C_4)$-Alkoxycarbonylamino-Gruppe, eine Phenylgruppe, die gegebenenfalls durch $Z_5$ einfach oder zweifach substituiert ist, eine Pyrid-2-yl-, -3-yl- oder -4-yl-Gruppe, eine Gruppe $-NR_{16}R_{17}$ bedeutet, worin $R_{16}$ und $R_{17}$ unabhängig voneinander ein Wasserstoffatom oder eine $(C_1-C_4)$-Alkylgruppe bedeuten, oder $R_{16}$ und $R_{17}$ zusammen mit dem Stickstoffatom, an das gebunden sind, eine Morpholinylgruppe bilden, die gegebenenfalls durch eine $(C_1-C_4)$-Alkylgruppe einfach oder zweifach substitutiert ist, oder $R_{16}$ und $R_{17}$ zusammen mit dem Stickstoffatom, an das sie gebunden sind, eine Piperazinylgruppe bilden, die gegebenenfalls in der 4-Stellung durch einen Substituenten $Z_3$ substituiert ist, oder $R_{16}$ und $R_{17}$ zusammen mit dem Stickstoffatom, an das sie gebunden sind, eine Pyrrolidinyl- oder Piperidinyl-Gruppe bilden, wobei die Pyrrolidinyl- und Piperidinyl-Gruppen gegebenenfalls durch $Z_5$ substituiert sind, mit der Maßgabe, daß wenn q = 1, $R_{15}$ von Hydroxy, $(C_1-C_4)$-Alkoxy, $(C_1-C_4)$-Alkoxycarbonylamino, $-NR_{16}R_{17}$ verschieden ist; oder $R_{13}$ und $R_{14}$ zusammen mit dem Stickstoffatom, an das sie gebunden sind, eine Morpholinylgruppe, die gegebenenfalls durch eine $(C_1-C_4)$-Alkylgruppe einfach oder zweifach substituiert ist, eine Piperazinylgruppe, die gegebenenfalls in der 4-Stellung durch einen Substituenten $Z_3$ substituiert ist, bilden; oder $R_{13}$ und $R_{14}$ zusammen mit dem Stickstoffatom, an das sie gebunden sind, eine Azetidinyl-, Pyrrolidinyl-, Piperidinyl- oder Hexahydroazepinyl-Gruppe bilden, wobei die Pyrrolidinyl-, Piperidinyl- und Hexahydroazepinyl-Gruppen gegebenfalls durch $Z_8$ einfach oder zweifach substituiert sind;

oder W eine Gruppe $OR_{18}$ bedeutet, worin $R_{18}$ ein Wasserstoffatom, eine $(C_1-C_4)$-Alkyl-, $(C_1-C_4)$-Alkoxy-$(C_1-C_4)$-alkyl-Gruppe oder eine Gruppe $-T_3-R_{19}$ darstellt, worin $T_3$ $-(CH_2)_p-$ bedeutet, worin p 2 oder 3 bedeutet und $R_{19}$ ausgewählt ist aus Hydroxygruppen, Triphenylmethoxygruppen und Gruppen-$NR_{20}R_{21}$, worin $R_{20}$ ein Wasserstoffatom oder eine $(C_1-C_4)$-Alkylgruppe darstellt und $R_{21}$ ein Wasserstoffatom, eine $(C_1-C_4)$-Alkyl-, Tetrahydrofuranylmethyl- oder Tetrahydropyranylmethyl-Gruppe bedeutet, oder $R_{20}$ und $R_{21}$ zusammen mit dem Stickstoffatom, an das sie gebunden sind, eine Morpholinylgruppe, die gegebenenfalls durch eine $(C_1-C_4)$-Alkylgruppe mono- oder disubstituiert ist, oder eine Piperazinylgruppe, die gegebenenfalls in der 4-Stellung durch einen Substituenten $Z_3$ substituiert ist, bilden oder $R_{20}$ und $R_{21}$ zusammen mit dem Stickstoffatom, an das sie gebunden sind, eine Pyrrolidinyl- oder Piperidinyl-Gruppe bilden, wobei die Pyrrolidinyl- und Piperidinyl-Gruppen gegebenenfalls durch $Z_5$ substituiert sind;

- $Z_3$ eine $(C_1-C_4)$-Alkyl-, Pyridyl-, Phenyl-, $(C_1-C_4)$-Alkylcarbonyl- oder $(C_1-C_4)$-Alkoxycarbonyl-Gruppe darstellt;
- $Z_4$ Oxo, ein Fluoratom, Hydroxy, $(C_1-C_4)$-Alkyl, Benzyl, Amino, $(C_1-C_4)$-Alkylamino, Di$(C_1-C_4)$-alkylamino, $(C_1-C_4)$-Alkoxy, $(C_1-C_4)$-Alkoxycarbonyl oder $(C_1-C_4)$-Alkoxycarbonylamino bedeutet;
- $Z_5$ ein Chlor-, Brom-, Iod- oder Fluoratom, eine Hydroxygruppe, eine $(C_1-C_4)$-Alkyl- oder $(C_1-C_4)$-Alkoxy-Gruppe bedeutet;
- $Z_7$ ein Fluoratom, eine Hydroxygruppe, eine Hydroxy-$(C_1-C_4)$-alkyl-, $(C_1-C_4)$-Alkyl-, $(C_1-C_4)$-Alkoxy-, $(C_1-C_4)$-Alkylcarbonyl-Gruppe bedeutet;
- $Z_8$ ein Fluoratom, eine Hydroxygruppe, eine $(C_1-C_4)$-Alkyl-, $(C_3-C_6)$-Cycloalkyl-, Benzyl-, Amino-, $(C_1-C_4)$-Alkylamino-, Di$(C_1-C_4)$-alkylamino-, $(C_1-C_4)$-Alkoxycarbonyl-, $(C_1-C_4)$-Alkoxycarbonylamino-, $(C_3-C_6)$-Cycloalkoxy-, Hydroxycarbonyl-, Hydroxy-$(C_1-C_4)$-alkyl- oder $(C_1-C_4)$-Alkoxy-$(C_1-C_4)$-alkyl-, oder $(C_1-C_4)$-Alkoxy-Gruppe oder $-CONR_{23}R_{24}$ bedeutet, worin $R_{23}$ und $R_{24}$ unabhängig voneinander ein Wasserstoffatom, $(C_1-C_4)$-Alkyl, Mono- oder Polyfluor-$(C_1-C_4)$-alkyl bedeuten oder $R_{23}$ und $R_{24}$ zusammen mit dem Stickstoffatom, an das sie gebunden sind, eine Pyrrolidinyl- oder Piperidinyl-Gruppe, wobei die Pyrrolidinyl- oder Piperidinyl-Gruppen gegebenenfalls durch $Z_3$ substituiert sind, oder eine Difluormethylidengruppe bilden;

(ii):

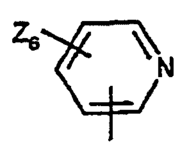

worin:

- $Z_6$ ein Chloratom oder eine $(C_1-C_4)$-Alkyl- oder $(C_1-C_4)$-Alkoxygruppe darstellt;
- $R_1$ eine $(C_1-C_4)$-Alkylgruppe, die gegebenenfalls eine Doppel- oder eine Dreifach-Bindung aufweist; $(C_1-C_4)$-Alkoxycarbonylgruppe; Phenyloxycarbonyl-Gruppe oder eine Gruppe $T_1-R_{22}$ bedeutet, worin $T_1$ die oben angegebenen Bedeutungen besitzt und $R_{22}$ eine Hydroxy- oder $(C_1-C_4)$-Alkoxy-Gruppe darstellt;

- $R_2$ und $R_4$ unabhängig voneinander ein Wasserstoff-, Chlor- oder Fluor-Atom oder eine ($C_1$-$C_4$)-Alkyl- oder ($C_1$-$C_4$)-Alkoxy-Gruppe bedeuten;
- $R_3$ ein Chlor- oder Fluor-Atom, eine ($C_1$-$C_4$)-Alkyl-, ($C_1$-$C_4$)-Alkoxy-, Hydroxy-, ($C_1$-$C_4$)-Carbamoyl-, ($C_1$-$C_4$) -Alkylcarbonylamino-, Nitro-, Cyano-, Trifluormethyl-, Amino-, ($C_3$-$C_6$)-Cycloalklyamino-, ($C_1$-$C_4$)-Alkylamino-, Di($C_1$-$C_4$)-alkylamino-, Tri($C_1$-$C_4$)-trialkylammonium-, $A^-$, worin $A^-$ ein Anion darstellt, Pyrrolidin-1-yl-, Piperidin-1-yl-, Piperazin-1-yl-, Morpholin-4-yl- oder Hexahydroazepin-1-yl-Gruppe bedeutet;
- X und Y unabhängig voneinander ein Wasserstoff-, Chlor-, Brom-, Iod- oder Fluor-Atom oder eine ($C_1$-$C_4$) -Alkoxy- oder Trifluormethoxy-Gruppe bedeuten;

sowie deren pharmazeutisch annehmbare Salze, Solvate und Hydrate.

**2.** Verbindung nach Anspruch 1 in Form des reinen Enantiomeren oder einer Mischung der Enantiomeren der Formel:

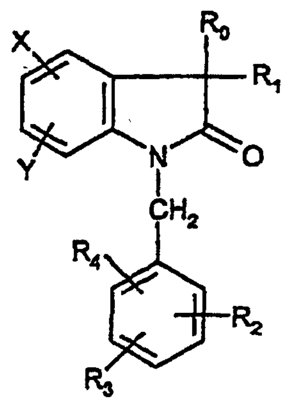

(I)

in der :

$R_0$

(i) :

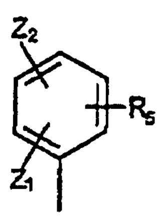

bedeutet und $Z_1$, $Z_2$, $R_1$, $R_2$, $R_3$, $R_4$, $R_5$, Y und X die für (I) angegebenen Bedeutungen besitzen, deren pharmazeutisch annehmbare Salze, Solvate und Hydrate.

**3.** Verbindungen nach Anspruch 2 der Formel:

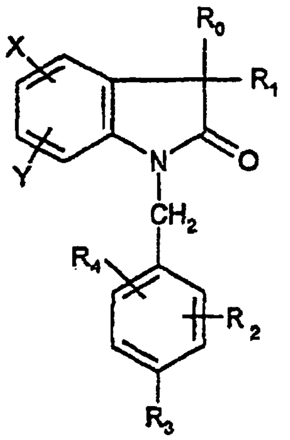

(Ia)

in der $R_1$ eine Methyl- oder Hydroxy-Gruppe bedeutet und $R_0$, $R_2$, $R_3$, $R_4$, X und Y die für (I) angegebenen Bedeutungen besitzen, in Form des reinen Enantiomeren oder einer Mischung der Enantiomeren sowie deren pharmazeutisch annehmbare Salze. Solvate und Hydrate.

**4.** Verbindungen nach Anspruch 3 der Formel:

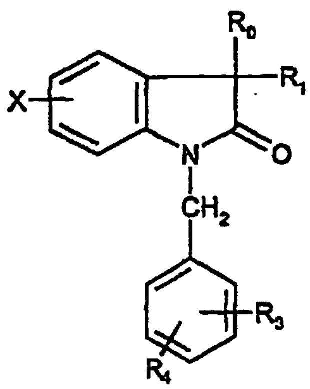

(Ib)

in der $R_1$ eine Methyl- oder Hydroxy-Gruppe bedeutet und $R_0$, $R_3$, $R_4$ und X die für (I) angegebenen Bedeutungen besitzen, in Form des reinen Enantiomeren oder der Mischung der Enantiomeren sowie deren pharmazeutisch annehmbare Salze, Solvate und Hydrate.

**5.** Verbindungen nach Anspruch 4 der Formel:

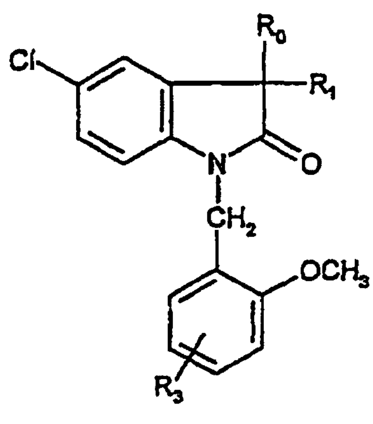

(Ic)

in der $R_1$ eine Methyl- oder Hydroxy-Gruppe bedeutet und $R_0$ und $R_3$ die für (I) angegebenen Bedeutungen besitzen, in Form des reinen Enantiomeren oder der Mischung der Enantiomeren sowie deren pharmazeutisch annehmbare Salze, Solvate und Hydrate.

**6.** Verbindungen nach Anspruch 5 der Formel:

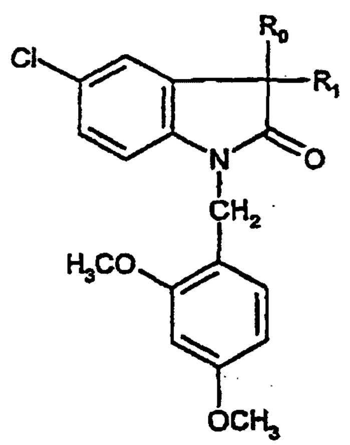

(Id)

in der $R_1$ eine Methyl- oder Hydroxy-Gruppe bedeutet und $R_0$ die für (I) angegeb nen Bedeutungen besitzt, in Form des reinen Enantiomeren oder der Mischung von Enantiomeren sowie deren pharmazeutisch annehmbare Salze, Solvate und Hydrate.

**7.** Verbindungen nach einem der Ansprüche 1 bis 6, worin $R_0$ die Gruppe:

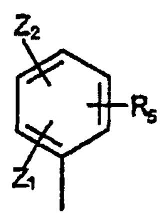

bedeutet, worin $Z_1$, $Z_2$ und $R_5$ die für (I) angegebenen Bedeutungen besitzen.

**8.** Verbindung nach Anspruch 7, worin $R_0$ die Gruppe

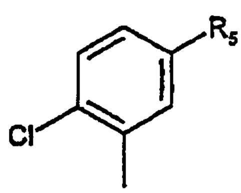

bedeutet, worin $R_5$ die für (I) angegebenen Bedeutungen besitzt.

**9.** Verbindungen nach einem der Ansprüche 1 bis 8, worin $R_1$ eine Methylgruppe bedeutet.

**10.** Verbindungen nach Anspruch 1 ausgewählt aus:

5-Chlor-3-(2-chlorphenyl)-1-(2,4-dimethoxybenzyl)-3-methylindolin-2-on;

5-Chlor-3-(2-chlorphenyl)-1-[4-(isopropylamino)-2-methoxybenzyl)-3-methylindolin-2-on;
*N*-{4-Chlor-3-[5-chlor-1-(2,4-dimethoxybenzyl)-3-methyl-2-oxoindolin-3-yl]-phenyl}-acetamid;
*N*-{4-Chlor-3-[5-chlor-1-(2,4-dimethoxybenzyl)-3-methyl-2-oxoindolin-3-yl]-phenyl}-3-methylbutanamid;
*N*-{4-Chlor-3-[5-chlor-1-(2,4-dimethoxybenzyl)-3-methyl-2-oxoindolin-3-yl]-phenyl}-benzamid;
*N*-{4-Chlor-3-[5-chlor-1-(2,4-dimethoxybenzyl)-3-methyl-2-oxoindolin-3-yl]-phenyl}-nicotinamid;
*N*-{4-Chlor-3-[5-chlor-1-(2,4-dimethoxybenzyl)-3-methyl-2-oxoindolin-3-yl]-phenyl}-2-methoxyacetamid;
3-{4-Chlor-3-[5-chlor-1-(2,4-dimethoxybenzyl)-3-methyl-2-oxoindolin-3-yl]-anilino}-3-oxopropansäuremethylester;
*N*-{4-Chlor-3-[5-chlor-1-(2,4-dimethoxybenzyl)-3-methyl-2-oxoindolin-3-yl]-phenyl}-3-methoxypropanamid;
*N*-{4-Chlor-3-[5-chlor-1-(2,4-dimethoxybenzyl)-3-methyl-2-oxoindolin-3-yl]-phenyl}-*N*-methylacetamid;
*N*-{4-Chlor-3-[5-chlor-1-(2,4-dimethoxybenzyl)-3-methyl-2-oxoindolin-3-yl]-phenyl}-*N*-methylmethansulfonamid;
4-Chlor-3-[5-chlor-1-(2,4-dimethoxybenzyl)-3-methyl-2-oxoindolin-3-yl]-*N*,*N*-diethylbenzamid;
4-Chlor-3-[5-chlor-1-(2,4-dimethoxybenzyl)-3-methyl-2-oxoindolin-3-yl]-*N*,*N*-dimethylbenzamid;
5-Chlor-3-[2-chlor-5-(1-piperidinylcarbonyl)-phenyl]-1-(2,4-dimethoxybenzyl)-3-methylindolin-2-on;
4-Chlor-3-[5-chlor-1-(2,4-dimethoxybenzyl)-3-methyl-2-oxoindolin-3-yl]-*N*-ethylbenzamid;
5-Chlor-3-(2-chlor-5{[2-(methoxymethyl)-1-pyrrolidinyl]-carbonyl}-phenyl)-1-(2,4-dimethoxybenzyl)-3-methylindolin-2-on;
5-Chlor-3-{2-chlor-5-[(2-methyl-1-piperidinyl)-carbonyl]-phenyl}-1-(2,4-dimethoxybenzyl)-3-methylindolin-2-on;
4-Chlor-3-[5-chlor-1-(2,4-dimethoxybenzyl)-3-methyl-2-oxoindolin-3-yl]-*N*-ethyl-*N*-methylbenzamid;
1-{4-Chlor-3-[5-chlor-1-(2,4-dimethoxybenzyl)-3-methyl-2-oxoindolin-3-yl]-benzoyl}-2-piperidincarbonsäuremethylester;
5-Chlor-3-(2-chlor-5-[(4-hydroxy-1-piperidinyl)-carbonyl]-phenyl)-1-(2,4-dimethoxybenzyl)-3-methylindolin-2-on;
5-Chlor-3-{2-chlor-5[(2-methoxyethoxy)-methyl]-phenyl}-1-(2,4-dimethoxybenzyl)-3-methylindolin-2-on;
5-Chlor-3-[2-chlor-5-(4-morpholinylmethyl)-phenyl]-1-(2,4-dimethoxybenzyl)-3-methylindolin-2-on;
5-Chlor-3-(2-chlor-5-{[2-(4-morpholinyl)-ethoxy]-methyl}-phenyl)-1-(2,4-dimethoxybenzyl)-3-methylindolin-2-on;
1-[4-Chlor-3-[5-chlor-1-(2,4-dimethoxybenzyl)-3-methyl-2-oxoindolin-3-yl]-benzoyl]-3-hydroxypiperidin;
1-[4-Chlor-3-[5-chlor-1-(2,4-dimethoxybenzyl)-3-methyl-2-oxoindolin-3-yl]-benzoyl]-(R)-3-hydroxypiperidin;
1-[4-Chlor-3-[5-chlor-1-(2,4-dimethoxybenzyl)-3-methyl-2-oxoindolin-3-yl]-benzoyl]-4-methoxypiperidin;
1-[4-Chlor-3-[5-chlor-1-(2,4-dimethoxybenzyl)-3-methyl-2-oxoindolin-3-yl]-benzoyl]-4-ethoxypiperidin;
1-[4-Chlor-3-[5-chlor-1-(2,4-dimethoxybenzyl)-3-methyl-2-oxoindolin-3-yl]-benzoyl]-(R,S)-2,6-dimethylpiperidin;
1-[4-Chlor-3-[5-chlor-1-(2,4-dimethoxybenzyl)-3-methyl-2-oxoindolin-3-yl]-benzoyl]-(R)-2-ethoxycarbonylpiperidin;
1-[4-Chlor-3-[5-chlor-1-(2,4-dimethoxybenzyl)-3-methyl-2-oxoindolin-3-yl]-benzoyl]-(R)-2-*N*,*N*-dimethylaminocarbonylpiperidin;
1-[4-Chlor-3-[5-chlor-1-(2,4-dimethoxybenzyl)-3-methyl-2-oxoindolin-3-yl)-benzoyl]-(R)-2-(*N*-methyl-*N*-2,2,2-trifluorethylaminocarbonyl]-piperidin;
1-[4-Chlor-3-[5-chlor-1-(2,4-dimethoxybenzyl)-3-methyl-2-oxoindolin-3-yl]-benzoyl]-(R)-2-pyrrolidinocarbonylpiperidin;
1-[4-Chlor-3-[5-chlor-1-(2,4-dimethoxybenzyl)-3-methyl-2-oxoindolin-3-yl]-benzoyl]-(S)-2-methylpiperidin;
4-Chlor-3-[5-chlor-1-(2,4-dimethoxybenzyl)-3-methyl-2-oxoindolin-3-yl]-*N*-ethyl-*N*-(2-phenylethyl)-benzamid;
4-Chlor-3-[5-chlor-1-(2,4-dimethoxybenzyl)-3-methyl-2-oxoindolin-3-yl]-*N*-ethyl-*N*-(4-pyridylmethyl)-benzamid-Hydrochlorid;
4-Chlor-3-[5-chlor-1-(2,4-dimethoxybenzyl)-3-methyl-2-oxoindolin-3-yl]-*N*-ethyl-*N*-(3-pyridylmethyl)-benzamid;
4-Chlor-3-[5-chlor-1-(2,4-dimethoxybenzyl)-3-methyl-2-oxoindolin-3-yl]-*N*-ethyl-*N*-(2-pyridylmethyl)-benzamid;
4-Chlor-3-[5-chlor-1-(2,4-dimethoxybenzyl)-3-methyl-2-oxoindolin-3-yl]-*N*-ethyl-*N*-(2-methoxyethyl)-benzamid;
4-Chlor-3-[5-chlor-1-(2,4-dimethoxybenzyl)-3-methyl-2-oxoindolin-3-yl]-*N*-ethyl-*N*-(2-dimethylaminoethyl)-benzamid-Hydrochlorid;
4-Chlor-3-[5-chlor-1-(2,4-dimethoxybenzyl)-3-methyl-2-oxoindolin-3-yl]-*N*-ethyl-*N*-(2-morpholinoethyl)-benzamid;
4-Chlor-3-[5-chlor-1-(2,4-dimethoxybenzyl)-3-methyl-2-oxoindolin-3-yl]-*N*-ethyl-*N*-(2-pyrrolidinoethyl)-benzamid-Hydrochlorid;
4-Chlor-3-[5-chlor-1-(2,4-dimethoxybenzyl)-3-methyl-2-oxoindolin-3-yl]-*N*-ethyl-*N*-(2-piperidinoethyl)-benzamid-Hydrochlorid;
4-Chlor-3-[5-chlor-1-(2,4-dimethoxybenzyl)-3-methyl-2-oxoindolin-3-yl]-*N*-ethyl-*N*-(2-hydroxyethyl)-benzamid;
4-Chlor-3-[5-chlor-1-(2,4-dimethoxybenzyl)-3-methyl-2-oxoindolin-3-yl]-*N*-ethyl-*N*-(2-pyridin-4-yl)-ethyl]-benzamid-Hydrochlorid;
4-Chlor-3-[5-chlor-1-(2,4-dimethoxybenzyl)-3-methyl-2-oxoindolin-3-yl]-*N*-ethyl-*N*-(2,2,2-trifluorethyl)-benzamid;
4-Chlor-3-[5-chlor-1-(2,4-dimethoxybenzyl)-3-methyl-2-oxoindolin-3-yl]-*N*-methyl-*N*-(2,2,2-trifluorethyl)-benzamid;
4-Chlor-3-[5-chlor-1-(2,4-dimethoxybenzyl)-3-methyl-2-oxoindolin-3-yl]-*N*-ethyl-*N*-isopropylbenzamid;
4-Chlor-3-[5-chlor-1-(2,4-dimethoxybenzyl)-3-methyl-2-oxoindolin-3-yl]-*N*-(2-dimethylaminoethyl) -*N*-(2,2,2-trifluorethyl)-benzamid-Hydrochlorid;
4-Chlor-3-[5-chlor-1-(2,4-dimethoxybenzyl)-3-methyl-2-oxoindolin-3-yl]-*N*-cyclohexylbenzamid;
4-Chlor-3-[5-chlor-1-(2,4-dimethoxybenzyl)-3-methyl-2-oxoindolin-3-yl]-*N*-ethyl-*N*-[3-(pyridin-4-yl)-propyl]-benzamid;

in Form des reinen Enantiomeren oder der Mischung von Enantiomeren, sowie deren pharmazeutisch annehmbare Salze, Solvate und Hydrate.

**11.** Verfahren zur Herstellung der Verbindung der Formel (I) nach Anspruch 1, **dadurch gekennzeichnet, daß** man:

a) eine Verbindung der Formel:

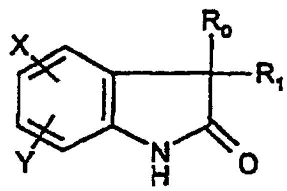

(II)

in der X, Y, $R_0$ und $R_1$ die für (I) angegebenen Bedeutungen besitzen, in Gegenwart einer Base mit einem Halogenid der Formel:

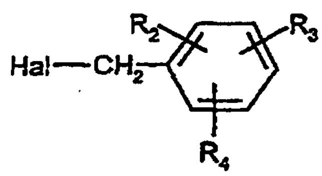

(1)

in der Hal ein Halogenatom darstellt und $R_2$, $R_3$ und $R_4$ die für (I) angegebenen Bedeutungen besitzen, umsetzt;
b) oder, wenn $R_1$ eine elektrophile Gruppe bedeutet, man die Verbindung der Formel:

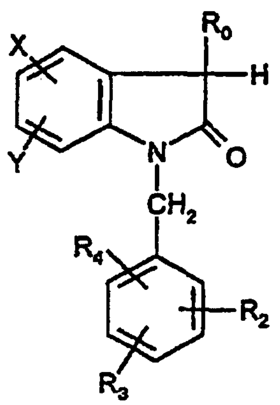

(III)

in der $R_0$, $R_2$, $R_3$, $R_4$, X und Y die für (I) angegebenen Bedeutungen besitzen, durch Einwirkung eines Derivats $R_1$-Z, worin Z eine austretende Gruppe darstellt, in Gegenwart einer Base umwandelt;

c) oder, wenn $R_1$ OH bedeutet, man ein Isatin-Derivat der Formel:

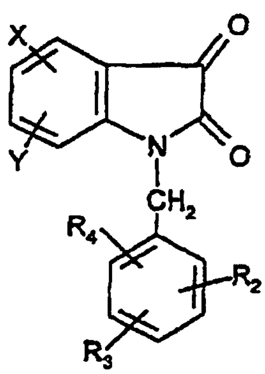

(IV)

in der $R_2$, $R_3$, $R_4$, X und Y die für (I) angegebenen Bedeutungen besitzen, mit einem metallorganischen Derivat $R_0$-M oder $R_0$MgHal, worin $R_0$ die für (I) angegebenen Bedeutungen besitzt, M ein Metallatom darstellt und Hal ein Brom- oder Iod-Atom bedeutet, umsetzt;

d) oder die Verbindung der Formel:

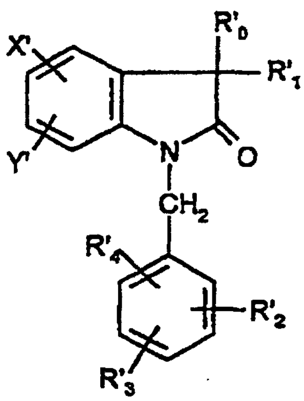

(Ip)

in der $R'_0$, $R'_1$, $R'_2$, $R'_3$, $R'_4$, X' und Y' entweder $R_0$, $R_1$, $R_2$, $R_3$, $R_4$, X beziehungsweise Y, wie sie für (I) definiert worden sind, oder eine Vorläufergruppe für $R_0$, $R_1$, $R_2$, $R_3$, $R_4$; X und Y bedeuten, einer Nachbehandlung unterzieht zur Umwandlung irgendeiner der Gruppen $R'_0$, $R'_1$, $R'_2$, $R'_3$, $R'_4$, X' oder Y' in eine Gruppe $R_0$, $R_1$, $R_2$, $R_3$, $R_4$, X beziehungsweise Y, wie sie für (I) definiert worden sind.

**12.** Pharmazeutische Zubereitung, **dadurch gekennzeichnet, daß** sie als Wirkstoff eine Verbindung nach einem der Ansprüche 1 bis 10 enthält.

**13.** Pharmazeutische Zubereitung nach Anspruch 12, **dadurch gekennzeichnet, daß** sie eine Antagonisten der Rezeptoren für Oxytocin nach einem der Ansprüche 1 bis 10 in Kombination mit einem Antagonisten der Rezeptoren $V_{1a}$ für Vasopressin enthält.

**14.** Produkt enthaltend einen Antagonisten der Rezeporten für Oxytocin nach einem der Ansprüche 1 bis 10 und einen Antagonisten der Rezeptoren $V_{1a}$ für Vasopressin für die gleichzeitige, getrennte, oder zeitlich verzögerte Anwendung bei der Behandlung von Dysmenorrhoe, Endometriose, die Steuerung vorzeitiger Wehen und zur Steuerung der vorbereitenden Wehen im Hinblick auf einen Kaiserschnitt.

**15.** Verwendung einer Verbindung nach einem der Ansprüche 1 bis 10 für die Herstellung eines Arzneimittels zur Behandlung von Oxytocin-abhängigen Störungen.

**16.** Verwendung einer Verbindung nach einem der Ansprüche 1 bis 10 für die Herstellung eines tocolytischen oder Uterus-relaxierenden Arzneimittels.

**17.** Verwendung einer Verbindung nach einem der Ansprüche 1 bis 10 für die Herstellung von Arzneimitteln, die bestimmt sind zur Unterstützung der Vernarbung, für die Behandlung der Analgesie, der Anxiolyse, der Depression, der Schizophrenie, des Autismus, des Besessenheits- und Krampf-Syndroms, zur Verbesserung des mütterlichen und sozialen Verhaltens, zur Erleichterung des Wiedererkennens und der Annahme der Mutter durch das Kind, zur Behandlung von Gedächtnisstörungen, zur Regulierung der Aufnahme von Nahrung und Getränk, der Drogenabhängigkeit, dem sexuellen Entzug und der sexuellen Motivation, zur Behandlung von Störungen im Urogenital-Bereich bei geburtshelferischen und gynäkologischen Handlungen, zur Steuerung von Uterus-Kontraktionen vor Beendigung der Schwangerschaft, zur Steuerung der pränatalen Wehen, zur Behandlung von Dysmenorrhoe, zur Steuerung der vorbereitenden Wehen im Hinblick auf eine Kaiserschnittgeburt, zur Lösung von Problemen der Sterilität oder Fertilität, zur Kontrolle der Geburt, zur Steuerung des Oestrus, zum Abstillen, für die Entwöhnung von der Muttermilch, für die Übertragung und die Implantation von Embryonen, zur Behandlung der Endometriose, der Harninkontinenz bei Anstrengungen oder Notfällen, der gutartigen Prostata-Hypertrophie, von Erektionsdysfunktionen, der Hypertension, der Hyponaträmie, von Herzinsuffizienz, der Artherosklerose, der Angiogenese, zur Steuerung der Fettspeicherung durch Fettzellen und zur Behandlung von Mamma- oder Prostata-Karzinomen.

**18.** Arzneimittel, **dadurch gekennzeichnet, daß** es aus einer Verbindung nach einem der Ansprüche 1 bis 10 besteht.

## Claims

**1.** Compound in the form of a pure enantiomer or of a mixture of enantiomers of formula:

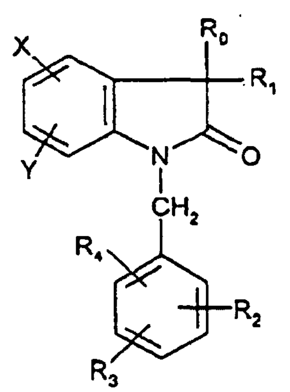

(I)

in which:

- $R_0$ represents a group chosen from:

(i) :

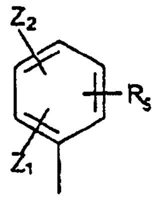

in which:

- $Z_1$ represents a chlorine, bromine, iodine or fluorine atom or a ($C_1$-$C_4$)alkyl, ($C_1$-$C_4$)alkoxy or trifluoromethyl group;
- $Z_2$ represents a hydrogen, chlorine, bromine, iodine or fluorine atom or a ($C_1$-$C_4$)alkyl, ($C_3$-$C_5$)cycloalkyl, ($C_1$-$C_4$)alkoxy, ($C_3$-$C_5$)cycloalkoxy or polyfluoro($C_1$-$C_4$)alkyl group;
- $R_5$ represents $T_1W$ in which $T_1$ represents $-(CH_2)_m-$, it being possible for m to be equal to 0 or 1, and W represents a hydrogen atom or a hydroxycarbonyl (or carboxyl), ($C_1$-$C_4$)alkoxycarbonyl, 1,3-dioxolan-2-yl or 1,3-dioxan-2-yl group,

or else W represents an $-NR_6R_7$ group in which $R_6$ and $R_7$ represent, independently of one another, a hydrogen atom, a ($C_1$-$C_4$)alkyl group, a ($C_1$-$C_4$)alkylsulphonyl group or a phenylsulphonyl group in which the phenyl group can be mono-, di- or trisubstituted by $Z_5$; or else $R_6$ and $R_7$ form, with the nitrogen atom to which they are bonded, a morpholinyl group optionally substituted by a ($C_1$-$C_4$)alkyl group or an oxo; or else $R_6$ and $R_7$ form, with the nitrogen atom to which they are bonded, a piperazinyl group optionally substituted in the 4-position by a $Z_3$ substituent; or else $R_6$ and $R_7$ form, with the nitrogen atom to which they are bonded, a pyrrolidinyl or piperidyl group, the said pyrrolidinyl and piperidyl groups optionally being substituted by $Z_4$;

or else W represents an $-NR_8COR_9$ group in which $R_8$ represents a hydrogen atom or a ($C_1$-$C_4$)alkyl group and $R_9$ represents a hydrogen atom or a ($C_1$-$C_4$)alkyl, benzyl, pyridyl or phenyl group, it being possible for the said phenyl group to be mono-, di- or trisubstituted by $Z_5$; or else $R_9$ represents an $-NR_{10}R_{11}$ group in which $R_{10}$ and $R_{11}$ represent, independently of one another, a hydrogen atom or a ($C_1$-$C_4$)alkyl or else $R_{10}$ and $R_{11}$ form, with the nitrogen atom to which they are bonded, a pyrrolidinyl, piperidyl or morpholinyl group optionally substituted by a ($C_1$-$C_4$)alkyl group; or else $R_9$ represents a pyrrolidin-2-yl or -3-yl or piperid-2-yl, -3-yl or -4-yl group, the said pyrrolidinyl and piperidyl groups optionally being substituted by $Z_7$; or else $R_9$ represents a $-T_2$-$R_{12}$ or - $T_2$-$COR_{12}$ group in which $T_2$ represents $-(CH_2)_n-$, it being possible for n to be equal to 1, 2, 3 and 4, and $R_{12}$ represents a ($C_1$-$C_4$)alkoxy or $-NR_{10}R_{11}$ group, $R_{10}$ and $R_{11}$ being as defined above;

or else W represents a $-CONR_{13}R_{14}$ group in which $R_{13}$ represents a hydrogen atom or a ($C_1$-$C_4$)alkyl, ($C_3$-$C_7$) cycloalkyl, monofluoro ($C_1$-$C_4$) alkyl or polyfluoro($C_1$-$C_4$)alkyl group and $R_{14}$ represents a hydrogen atom, a ($C_1$-$C_4$)alkyl group, a phenyl group optionally substituted by $Z_5$, a $-T_4$-$R_{15}$ group in which $T_4$ represerts $-(CH_2)_q-$, with q equal to 1, 2, 3 or 4, and $R_{15}$ represents a hydroxyl group, a ($C_1$-$C_4$)alkoxy group, a ($C_1$-$C_4$) alkoxycarbonyl group, a ($C_1$-$C_4$) alkoxycarbonylamino group, a phenyl group optionally mono- or disubstituted by $Z_5$, a pyrid-2-yl, -3-yl or -4-yl, or an $-NR_{16}R_{17}$ group in which $R_{16}$ and $R_{17}$ represent, independently of one another, a hydrogen atom or a ($C_1$-$C_4$)alkyl or else $R_{16}$ and $R_{17}$ form, with the nitrogen atom to which they are bonded, a morpholinyl group optionally mono- or disubstituted by a ($C_1$-$C_4$)alkyl group or else $R_{16}$ and $R_{17}$ form, with the nitrogen atom to which they are bonded, a piperazinyl group optionally substituted in the 4-position by a $Z_3$ substituent or else $R_{16}$ and $R_{17}$ form, with the nitrogen atom to which they are bonded, a pyrrolidinyl or piperidyl group, the said pyrrolidinyl and piperidyl groups optionally being substituted by $Z_5$, it being understood that, when q = 1, $R_{15}$ is other than hydroxyl, ($C_1$-$C_4$)alkoxy, ($C_1$-$C_4$)alkoxycarbonylamino or $-NR_{16}R_{17}$; or else $R_{13}$ and $R_{14}$ form, with the nitrogen atom to which they are bonded, a morpholinyl group optionally mono- or disubstituted by a ($C_1$-$C_4$)alkyl group or a piperazinyl group optionally substituted in the 4-position by a $Z_3$ substituent; or else $R_{13}$ and $R_{14}$ form, with the nitrogen atom to which they are bonded, an azetidinyl, pyrrolidinyl, piperidyl or hexahydroazepinyl group, the said pyrrolidinyl, piperidyl and hexahydroazepinyl groups optionally being mono- or disubstituted by $Z_8$;

or else W represents an $OR_{18}$ group in which $R_{16}$ represents a hydrogen atom or a ($C_1$-$C_4$)alkyl, ($C_1$-$C_4$) alkoxy($C_1$-$C_4$)alkyl or $-T_3$-$R_{19}$ group in which $T_3$ represents $-(CH_2)_p-$, it being possible for p to be equal to 2 or 3, and $R_{19}$ is chosen from the hydroxyl, triphenylmethoxy or $-NR_{20}R_{21}$ groups in which $R_{20}$ represents a hydrogen atom or a ($C_1$-$C_4$)alkyl group and $R_{21}$ represents a hydrogen atom or a ($C_1$-$C_4$)alkyl, tetrahydrofuranylmethyl or tetrahydropyranylmethyl group, or else $R_{20}$ and $R_{21}$ form, with the nitrogen atom to which they are bonded, a morpholinyl group optionally mono- or disubstituted by a ($C_1$-$C_4$)alkyl group or a piperazinyl group

optionally substituted in the 4-position by a $Z_3$ substituent, or else $R_{20}$ and $R_{21}$ form, with the nitrogen atom to which they are bonded, a pyrrolidinyl or piperidyl group, the said pyrrolidinyl and piperidyl groups optionally being substituted by $Z_5$;

- $Z_3$ represents a ($C_1$-$C_4$)alkyl, pyridyl, phenyl, ($C_1$-$C_4$)alkylcarbonyl or ($C_1$-$C_4$)alkoxycarbonyl group;
- $Z_4$ represents an oxo, a fluorine atom, a hydroxyl, a ($C_1$-$C_4$)alkyl, a benzyl, an amino, a ($C_1$-$C_4$)alkylamino, a di ($C_1$-$C_4$) alkyl amino, a ($C_1$-$C_4$)alkoxy, a ($C_1$-$C_4$)alkoxycarbonyl or a ($C_1$-$C_4$)alkoxycarbonylamino;
- $Z_5$ represents a chlorine, bromine, iodine or fluorine atom, a hydroxyl group, a ($C_1$-$C_4$)alkyl group or a ($C_1$-$C_4$) alkoxy group;
- $Z_7$ represents a fluorine atom, a hydroxyl group, a hydroxy ($C_1$-$C_4$) alkyl group, a ($C_1$-$C_4$) alkyl, a ($C_1$-$C_4$)alkoxy or a ($C_1$-$C_4$)alkylcarbonyl;
- $Z_8$ represents a fluorine atom or a hydroxyl, ($C_1$-$C_4$) alkyl, ($C_3$-$C_6$) cycloalkyl, benzyl, amino, ($C_1$-$C_4$) alkylamino, di ($C_1$-$C_4$)alkylamino, ($C_1$-$C_4$)alkoxycarbonyl, ($C_1$-$C_4$)alkoxycarbonylamino, ($C_3$-$C_6$)cycloalkoxy, hydroxycarbonyl, hydroxy($C_1$-$C_4$)alkyl, ($C_1$-$C_4$)alkoxy($C_1$-$C_4$)alkyl, ($C_1$-$C_4$)alkoxy or -$CONR_{23}R_{24}$ group in which $R_{23}$ and $R_{24}$ represent, independently of one another, a hydrogen atom, a ($C_1$-$C_4$)alkyl, a monofluoro($C_1$-$C_4$)alkyl or a polyfluoro ($C_1$-$C_4$)alkyl, or else $R_{23}$ and $R_{24}$ form, with the nitrogen atom to which they are bonded, a pyrrolidinyl or piperidyl group, the said pyrrolidinyl or piperidyl groups optionally being substituted by $Z_3$ or a difluoromethylidene;

(ii) :

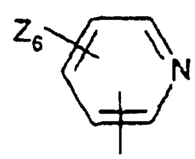

- $Z_6$ represents a chlorine atom or a ($C_1$-$C_4$)alkyl or ($C_1$-$C_4$)alkoxy group;
- $R_1$ represents a ($C_1$-$C_4$)alkyl group optionally comprising a double or a triple bond, a ($C_1$-$C_4$)alkoxycarbonyl group, a phenyloxycarbonyl group or a $T_1$-$R_{22}$ group in which $T_1$ is as defined above and $R_{22}$ represents a hydroxyl or ($C_1$-$C_4$)alkoxy group;
- $R_2$ and $R_4$ represent, independently of one another, a hydrogen, chlorine or fluorine atom or a ($C_1$-$C_4$)alkyl or ($C_1$-$C_4$)alkoxy group;
- $R_3$ represents a chlorine or fluorine atom or a ($C_1$-$C_4$)alkyl, ($C_1$-$C_4$)alkoxy, hydroxyl, ($C_1$-$C_4$)carbamoyl, ($C_1$-$C_4$) alkylcarbonylamino, nitro, cyano, trifluoromethyl, amino, ($C_3$-$C_6$)cycloalkylamino, ($C_1$-$C_4$)alkylamino, di ($C_1$-$C_4$)alkylamino, tri($C_1$-$C_4$)alkylammonium $A^-$, $A^-$ being an anion, pyrrolidin-1-yl, piperid-1-yl, piperazin-1-yl, morpholin-4-yl or hexahydroazepin-1-yl group;
- X and Y represent, independently of one another, a hydrogen, chlorine, bromine, iodine or fluorine atom or a ($C_1$-$C_4$)alkoxy or trifluoromethoxy group;

and their pharmaceutically acceptable salts, their solvates and their hydrates.

**2.** Compounds according to Claim 1 in the form of a pure enantiomer or of a mixture of enantiomers of formula:

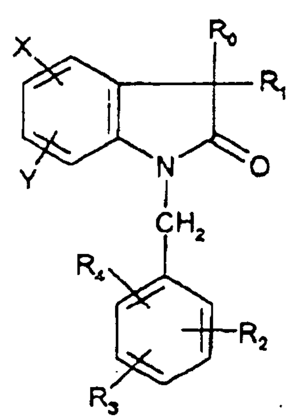

(I)

in which:

$R_0$ represents

(i) :

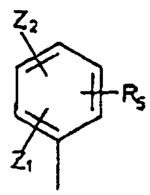

$Z_1$, $Z_2$, $R_1$, $R_2$, $R_3$, $R_4$, $R_5$, Y and X being as defined for (I), and their pharmaceutically acceptable salts, their solvates and their hydrates.

**3.** Compounds according to Claim 2 of formula:

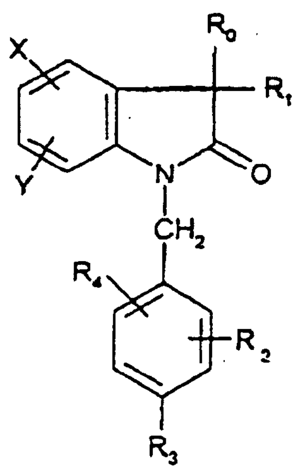

(Ia)

in which $R_1$ represents a methyl or hydroxyl group and $R_0$, $R_2$, $R_3$, $R_4$, X and Y are as defined for (I); in the form of a pure enantiomer or of a mixture of enantiomers, and their pharmaceutically acceptable salts, their solvates and their hydrates.

**4.** Compounds according to Claim 3 of formula:

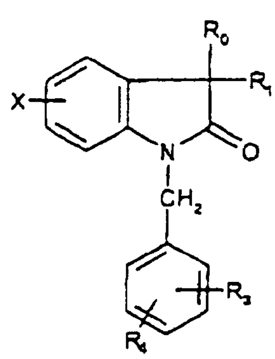

(Ib)

in which $R_1$ represents a methyl or hydroxyl group and $R_0$, $R_3$, $R_4$ and X are as defined for (I); in the form of a pure enantiomer or of a mixture of enantiomers, and their pharmaceutically acceptable salts, their solvates and their hydrates.

**5.** Compounds according to Claim 4 of formula:

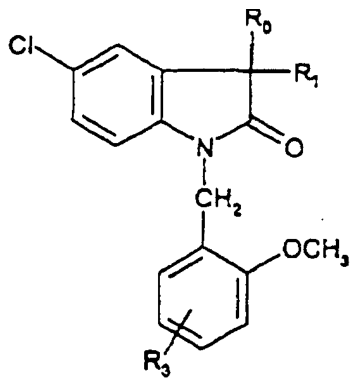

(Ic)

in which $R_1$ represents a methyl or hydroxyl group and $R_0$ and $R_3$ are as defined for (I); in the form of a pure enantiomer or of a mixture of enantiomers, and their pharmaceutically acceptable salts, their solvates and their hydrates.

**6.** Compounds according to Claim 5 of formula:

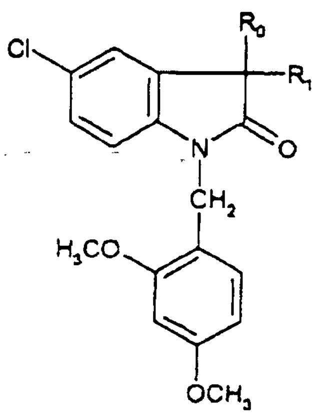

(Id)

in which $R_1$ represents a methyl or hydroxyl group and $R_0$ is as defined for (I); in the form of a pure enantiomer or of a mixture of enantiomers, and their pharmaceutically acceptable salts, their solvates and their hydrates.

**7.** Compounds according to any one of Claims 1 to 6 in which $R_0$ represents the group:

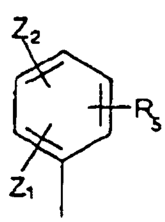

$Z_1$, $Z_2$ and $R_5$ being as defined for (I).

**8.** Compound according to Claim 7 in which $R_0$ represents the group:

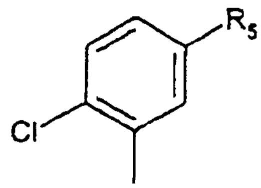

$R_5$ being as defined for (I).

**9.** Compounds according to any one of Claims 1 to 8 in which $R_1$ represents a methyl group.

**10.** Compounds according to Claim 1 chosen from:

5-Chloro-3-(2-chlorophenyl)-1-(2,4-dimethoxybenzyl)-3-methylindolin-2-one;
5-Chloro-3-(2-chlorophenyl)-1-[4-(isopropylamino)-2-methoxybenzyl]-3-methylindolin-2-one;
*N*-{4-Chloro-3-[5-chloro-1-(2,4-dimethoxybenzyl)-3-methyl-2-oxoindolin-3-yl)phenyl}acetamide;
*N*-{4-Chloro-3-[5-chloro-1-(2,4-dimethoxybenzyl)-3-methyl-2-oxoindolin-3-yl]phenyl}-3-methylbutanamide;
*N*-{4-Chloro-3-[5-chloro-1-(2,4-dimethoxybenzyl)-3-methyl-2-oxoindolin-3-yl]phenyl}benzamide;
*N*-{4-Chloro-3-[5-chloro-1-(2,4-dimethoxybenzyl)-3-methyl-2-oxoindolin-3-yl]phenyl}nicotinamide;
*N*-{4-Chloro-3-[5-chloro-1-(2,4-dimethoxybenzyl)-3-methyl-2-oxoindolin-3-yl]phenyl}-2-methoxyacetamide;
Methyl 3-{4-chloro-3-[5-chloro-1-(2,4-dimethoxybenzyl)-3-methyl-2-oxoindolin-3-yl)anilino}-3-oxopropanoate;
*N*-{4-Chloro-3-[5-chloro-1-(2,4-dimethoxybenzyl)-3-methyl-2-oxoindolin-3-yl]phenyl}-3-methoxypropanamide;
*N*-{4-Chloro-3-[5-chloro-1-(2,4-dimethoxybenzyl)-3-methyl-2-oxoindolin-3-yl]phenyl}-*N*-methylacetamide;
*N*-(4-Chloro-3-[5-chloro-1-(2,4-dimethoxybenzyl)-3-methyl-2-oxoindolin-3-yl]phenyl}-*N*-methylmethanesulphonamide;
4-Chloro-3-[5-chloro-1-(2,4-dimethoxybenzyl)-3-methyl-2-oxoindolin-3-yl]-*N*,*N*-diethylbenzamide;
4-Chloro-3-[5-chloro-1-(2,4-dimethoxybenzyl)-3-methyl-2-oxoindolin-3-yl]-*N*,*N*-dimethylbenzamide;
5-Chloro-3-[2-chloro-5-(1-piperidylcarbonyl)phenyl]-1-(2,4-dimethoxybenzyl)-3-methylindolin-2-one;
4-Chloro-3-[5-chloro-1-(2,4-dimethoxybenzyl)-3-methyl-2-oxoindolin-3-yl]-*N*-ethylbenzamide;
5-Chloro-3-(2-chloro-5-{[2-(methoxymethyl)-1-pyrrolidinyl]carbonyl}phenyl)-1-(2,4-dimethoxybenzyl)-3-methylindolin-2-one;
5-Chloro-3-{2-chloro-5-[(2-methyl-1-piperidyl)-carbonyl]phenyl}-1-(2,4-dimethoxybenzyl)-3-methylindolin-2-one;
4-Chloro-3-[5-chloro-1-(2,4-dimethoxybenzyl)-3-methyl-2-oxoindolin-3-yl]-*N*-ethyl-*N*-methylbenzamide;
Methyl 1-{4-chloro-3-[5-chloro-1-(2,4-dimethoxybenzyl)-3-methyl-2-oxoindolin-3-yl]benzoyl)-2-piperidinecarboxylate;
5-Chloro-3-{2-chloro-5-[(4-hydroxy-1-piperidyl)-carbonyl)phenyl}-1-(2,4-dimethoxybenzyl)-3-methylindolin-2-one;
5-Chloro-3-{2-chloro-5-[(2-methoxyethoxy)methyl]-phenyl}-1-(2,4-dimethoxybenzyl)-3-methylindolin-2-one;
5-Chloro-3-[2-chloro-5-(4-morpholinylmethyl)phenyl]-1-(2,4-dimethoxybenzyl)-3-methylindolin-2-one;
5-Chloro-3-(2-chloro-5-{[2-(4-morpholinyl)ethoxy]-methyl}phenyl)-1-(2,4-dimethoxybenzyl)-3-methylindolin-2-one;
1-[4-Chloro-3-[5-chloro-1-(2,4-dimethoxybenzyl)-3-methyl-2-oxoindolin-3-yl]benzoyl]-3-hydroxypiperidine;
1-[4-Chloro-3-[5-chloro-1-(2,4-dimethoxybenzyl)-3-methyl-2-oxoindolin-3-yl]benzoyl]-(R)-3-hydroxypiperidine;
1-[4-Chloro-3-[5-chloro-1-(2,4-dimethoxybenzyl)-3-methyl-2-oxoindolin-3-yl]benzoyl]-4-methoxypiperidine;
1-[4-Chloro-3-[5-chloro-1-(2,4-dimethoxybenzyl)-3-methyl-2-oxoindolin-3-yl]benzoyl]-4-ethoxypiperidine;
1-[4-Chloro-3-[5-chloro-1-(2,4-dimethoxybenzyl)-3-methyl-2-oxcindolin-3-yl]benzoyl]-(R,S)-2,6-dimethylpiperidine;
1-[4-Chloro-3-[5-chloro-1-(2,4-dimethoxybenzyl)-3-methyl-2-oxoindolin-3-yl]benzoyl]-(R)-2-ethoxycarbonylpiperidine;
1-[4-Chloro-3-[5-chloro-1-(2,4-dimethoxybenzyl)3-methyl-2-oxoindolin-3-yl]benzoyl]-(R)-2-*N*,*N*-dimethylaminocarbonylpiperidine;
1-[4-Chloro-3-[5-chloro-1-(2,4-dimethoxybenzyl)-3-methyl-2-oxoindolin-3-yl]benzoyl]-(R)-2-(N-methyl-*N*-2,2,2-trifluoroethylaminocarbonyl)piperidine;

1-[4-Chloro-3-[5-chloro-1-(2,4-dimethoxybenzyl)-3-methyl-2-oxoindolin-3-yl]benzoyl]-(R)-2-pyrrolidinocarbonylpiperidine;
1-[4-Chloro-3-[5-chloro-1-(2,4-dimethoxybenzyl)-3-methyl-2-oxoindolin-3-yl]benzoyl]-(S)-2-methylpiperidine;
4-chloro-3-[5-chloro-1-(2,4-dimethoxybenzyl)-3-methyl-2-oxoindolin-3-yl]-*N*-ethyl-*N*-(2-phenylethyl)benzamide;
4-Chloro-3-[5-chloro-1-(2,4-dimethoxybenzyl)-3-methyl-2-oxoindolin-3-yl]-*N*-ethyl-*N*-(4-pyridylmethyl)-benzamide hydrochloride;
4-Chloro-3-[5-chloro-1-(2,4-dimethoxybenzyl)-3-methyl-2-oxoindolin-3-yl]-*N*-ethyl-*N*-(3-pyridylmethyl)-benzamide;
4-Chloro-3-[5-chloro-1-(2,4-dimethoxybenzyl)-3-methyl-2-oxoindolin-3-yl]-*N*-ethyl-*N*-(2-pyridylmethyl)-benzamide;
4-Chloro-3-[5-chloro-1-(2,4-dimethoxybenzyl)-3-methyl-2-oxoindolin-3-yl]-*N*-ethyl-*N*-(2-methoxyethyl)benzamide;
4-Chloro-3-[5-chloro-1-(2,4-dimethoxybenzyl)-3-methyl-2-oxoindolin-3-yl]-*N*-ethyl-*N*-(2-dimethylaminoethyl)-benzamide hydrochloride;
4-Chloro-3-(5-chloro-1-(2,4-dimethoxybenzyl)-3-methyl-2-oxoindolin-3-yl]-*N*-ethyl-*N*-(2-morpholinoethyl)-benzamide;
4-Chloro-3-[5-chloro-1-(2,4-dimethoxybenzyl)-3-methyl-2-oxoindolin-3-yl]-*N*-ethyl-*N*-(2-pyrrolidinoethyl)-benzamide hydrochloride;
4-Chloro-3-[5-chloro-1-(2,4-dimethoxybenzyl)-3-methyl-2-oxoindolin-3-yl]-*N*-ethyl-*N*-(2-piperidinoethyl)-benzamide hydrochloride;
4-Chloro-3-[5-chloro-1-(2,4-dimethoxybenzyl)-3-methyl-2-oxoindolin-3-yl]-*N*-ethyl-*N*-(2-hydroxyethyl)benzamide;
4-Chloro-3-[5-chloro-1-(2,4-dimethoxybenzyl)-3-methyl-2-oxoindolin-3-yl]-*N*-ethyl-*N*-[2-(pyrid-4-yl)ethyl]-benzamide hydrochloride;
4-Chloro-3-[5-chloro-1-(2,4-dimethoxybenzyl)-3-methyl-2-oxoindolin-3-yl]-*N*-ethyl-*N*-(2,2,2-trifluoroethyl)-benzamide;
4-Chloro-3-[5-chloro-1-(2,4-dimethoxybenzyl)-3-methyl-2-oxoindolin-3-yl]-*N*-methyl-*N*-(2,2,2-trifluoroethyl)-benzamide;
4-Chloro-3-[5-chloro-1-(2,4-dimethoxybenzyl)-3-methyl-2-oxoindolin-3-yl]-*N*-ethyl-*N*-isopropylbenzamide;
4-Chloro-3-[5-chloro-1-(2,4-dimethoxybenzyl)-3-methyl-2-oxoindolin-3-yl]-*N*-(2-dimethylaminoethyl) -N-(2,2,2-trifluoroethyl)benzamide hydrochloride;
4-Chloro-3-[5-chloro-1-(2,4-dimethoxybenzyl)-3-methyl-2-oxoindolin-3-yl]-*N*-cyclohexylbenzamide;
4-Chloro-3-[5-chloro-1-(2,4-dimethoxybenzyl)-3-methyl-2-oxoindolin-3-yl]-*N*-ethyl-*N*-[3-(pyrid-4-yl)propyl]-benzamide;

in the form of a pure enantiomer or of a mixture of enantiomers, and their pharmaceutically acceptable salts, their solvates and their hydrates.

**11.** Process for the preparation of the compounds of formula (I) according to Claim 1, **characterized in that**:

a) a compound of formula:

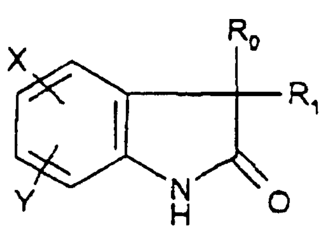

(II)

in which X, Y, $R_0$ and $R_1$ are as defined for (I), is reacted in the presence of a base with a halide of formula:

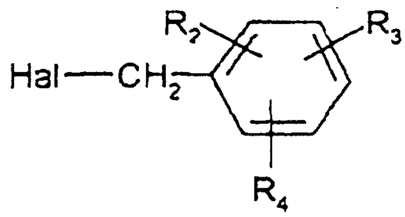

(1)

in which Hal represents a halogen atom and $R_2$, $R_3$ and $R_4$ are as defined for (I);

b) or else, when $R_1$ represents an electrophilic group, the compound of formula:

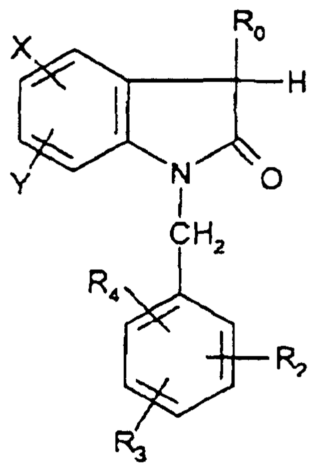

(III)

in which $R_0$, $R_2$, $R_3$, $R_4$, X and Y are as defined for (I), is converted by the action of a derivative $R_1$-Z, in which Z represents a leaving group, in the presence of a base;

c) or else, when $R_1$ = OH, an isatin derivative of formula:

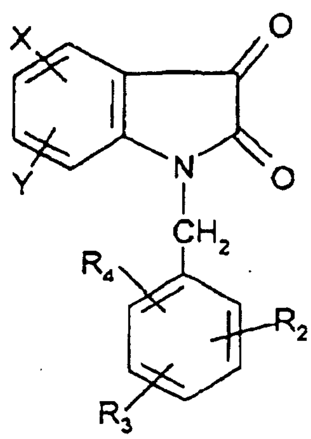

(IV)

in which $R_2$, $R_3$, $R_4$, X and Y are as defined for (I), is reacted with an organometallic derivative $R_0$-M or $R_0$MgHal, $R_0$ being as defined for (I), M being a metal atom and Hal being a bromine or iodine atom;

d) or else the compound of formula:

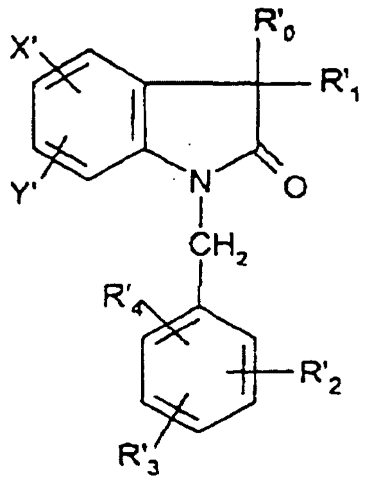

(Ip)

in which $R'_0$, $R'_1$, $R'_2$, $R'_3$, $R'_4$, X' and Y' respectively represent either $R_0$, $R_1$, $R_2$, $R_3$, $R_4$, X and Y as defined for (I) or a precursor group for $R_0$, $R_1$, $R_2$, $R_3$, $R_4$, X and Y, is subjected to a subsequent treatment to convert any one of the $R'_0$, $R'_1$, $R'_2$, $R'_3$, $R'_4$, X' and Y' groups to respectively $R_0$, $R_1$, $R_2$, $R_3$, $R_4$, X or Y as defined for (I).

**12.** Pharmaceutical composition, **characterized in that** it comprises, as active principle, a compound according to any one of Claims 1 to 10.

**13.** Pharmaceutical composition according to Claim 12, **characterized in that** it comprises an antagonist of oxytocin receptors according to any one of Claims 1 to 10 in combination with an antagonist of vasopressin $V_{1a}$ receptors.

**14.** Product comprising an antagonist of oxytocin receptors according to any one of Claims 1 to 10 and an antagonist of vasopressin $V_{1a}$ receptors for simultaneous or separate use or use spread out over time in the treatment of dysmenorrhoea or endometriosis or the control of premature labour and for controlling preparatory labour for the purpose of a caesarean delivery.

**15.** Use of a compound according to any one of Claims 1 to 10 in the preparation of a medicament intended for the treatment of oxytocin-dependent disorders.

**16.** Use of a compound according to any one of Claims 1 to 10 in the preparation of a uterine relaxant or tocolytic medicament.

**17.** Use of a compound according to any one of Claims 1 to 10 in the preparation of medicaments intended to promote cicatrization, to treat analgesia, anxiolysis, depression, schizophrenia, autism or obsessive compulsive syndrome, to improve maternal and social behaviour, to facilitate recognition and acceptance of the mother by the child, to treat memory disorders, to regulate food and drink intake, dependence on drugs, weaning and sexual motivation, to treat disorders of the urogenital sphere in the obstetric and gynaecological fields, to control contractions of the uterus before pregnancy has arrived at term, to control prenatal labour, to treat dysmenorrhoea, to control preparatory labour for the purpose of a caesarean delivery, to solve problems of sterility or fertility, to control births, to control oestrus, the halting of breast feeding, weaning, or the transfer and implantation of embryos, to treat endometriosis, urinary stress or urgency incontinence, benign prostate hypertrophy, erectile dysfunctions, hypertension, hyponatraemia, cardiac insufficiency, atherosclerosis or angiogenesis, to regulate the storage of fat by the adipocyte and to treat breast or prostate cancers.

**18.** Medicament, **characterized in that** it comprises a compound according to any one of Claims 1 to 10.